# NEW EUROPEAN PATENT SPECIFICATION

(11) **EP 0 577 752 B2**
(45) Date of publication and mention of the opposition decision: **11.07.2007**
(45) Mention of the grant of the patent: 05.07.2000
(21) Application number: 92910478.4
(22) Date of filing: 23.03.1992
(51) Int. Cl.: C12N 15/12, C12N 15/10, C07K 14/705, C12P 21/08, C12N 5/10

(54) **HUMAN PF4A RECEPTORS AND THEIR USE**
MENSCHLICHE PF4A REZEPTOREN UND IHRE VERWENDUNG
RECEPTOR HUMAIN PF4A ET LEUR UTILISATION

(30) Priority: 29.03.1991 US 677211; 19.12.1991 US 810782
(43) Date of publication of application: 12.01.1994
(73) Proprietor: GENENTECH, INC., South San Francisco California 94080 (US)
(72) Inventor: LEE, James, San Bruno, CA 94066 (US); HOLMES, William, E., Pacifica, CA 94044 (US); WOOD, William, I., San Mateo, CA 94402 (US)
(74) Representative: Armitage, Ian Michael
(86) International application number: PCT/US1992/002317
(87) International publication number: WO 1992/017497

(56) References cited:
- K.THOMAS ET AL: "Molecular cloning of the fMet-Leu-Phe receptor from neutrophils." THE JOURNAL OF BIOLOGICAL CHEMISTRY, vol. 265, no. 33, 25 November 1990 (1990-11-25), pages 20061-20064, XP002976714 BALTIMORE MD, USA cited in the application
- M. BECKMANN ET AL : "Molecular characterization of the interleukin-8 receptor" BIOCHEMICAL AND BIOPHYSICAL RESEARCH COMMUNICATIONS, vol. 179, no. 2, 16 September 1991 (1991-09-16), pages 784 -789, XP002976715 NEW YORK , USA

### SEQUENCE LISTING

(1) GENERAL INFORMATION:
   (i) APPLICANT: GENENTECH, INC.
   (ii) TITLE OF INVENTION: Human PF4A Receptors and Their Use
   (iii) NUMBER OF SEQUENCES: 3
   (iv) CORRESPONDENCE ADDRESS:
      (A) ADDRESSEE: Genentech, Inc.
      (B) STREET: 460 Point San Bruno Blvd
      (C) CITY: South San Francisco
      (D) STATE: California
      (E) COUNTRY: USA
      (F) ZIP: 94080
   (v) COMPUTER READABLE FORM:
      (A) MEDIUM TYPE: 5.25 inch, 360 Kb floppy disk
      (B) COMPUTER: IBM PC compatible
      (C) OPERATING SYSTEM: PC-DOS/MS-DOS
      (D) SOFTWARE: patin (Genentech)
   (vi) CURRENT APPLICATION DATA:
      (A) APPLICATION NUMBER:
      (B) FILING DATE: 23-Mar-1992
      (C) CLASSIFICATION:
   (vii) PRIOR APPLICATION DATA:
      (A) APPLICATION NUMBER: 07/677211
      (B) APPLICATION DATE: 29-Mar-1991
   (vii) PRIOR APPLICATION DATA:
      (A) APPLICATION NUMBER: 07/B10782
      (B) APPLICATION DATE: 19-Dec-1991
   (viii) ATTORNEY/AGENT INFORMATION:
      (A) NAME: Hensley, Max D.
      (B) REGISTRATION NUMBER: 27,043
      (C) REFERENCE/DOCKET NUMBER: 706P1
   (ix) TELECOMMUNICATION INFORMATION:
      (A) TELEPHONE: 415/266-1994
      (B) TELEFAX: 415/952-9881
      (C) TELEX: 910/371-7168
(2) INFORMATION FOR SEQ ID NO:1:
   (i) SEQUENCE CHARACTERISTICS:
      (A) LENGTH: 1933 bases
      (B) TYPE: nucleic acid
      (C) STRANDEDNESS: single
      (D) TOPOLOGY: linear
   (xi) SEQUENCE DESCRIPTION: SEQ ID NO:1:

      CCTGGCCGGT GCTTCAGTTA GATCAAACCA TTGCTGAAAC TGAAGAGGAC 50
(2) INFORMATION FOR SEQ ID NO:2:
   (i) SEQUENCE CHARACTERISTICS:
      (A) LENGTH: 1737 bases
      (B) TYPE: nucleic acid
      (C) STRANDEDNESS: single
      (D) TOPOLOGY: linear
   (xi) SEQUENCE DESCRIPTION: SEQ ID NO:2:
   GAATTCCAGT GTGCTGGCGG CGCGGCGCAA AGTGACGCCG AGGGCCTGAG 50
   TGCTCCAGTA GCCACCGCAT CTGGAGAACC AGCGGTTACC ATG GAG 96
                                               Met Glu
                                                 1
   GGG ATC AGT ATA TAC ACT TCA GAT AAC TAC ACC GAG GAA 135
   Gly Ile Ser Ile Tyr Thr Ser Asp Asn Tyr Thr Glu Glu
               5                    10                  15
   ATG GGC TCA GGG GAC TAT GAC TCC ATG AAG GAA CCC TGT 174
   Met Gly Ser Gly Asp Tyr Asp Ser Met Lys Glu Pro Cys
                   20                  25
   TTC CGT GAA GAA AAT GCT AAT TTC AAT AAA ATC TTC CTG 213
   Phe Arg Glu Glu Asn Ala Asn Phe Asn Lys Ile Phe Leu
       30                  35                  40
   CCC ACC ATC TAC TCC ATC ATC TTC TTA ACT GGC ATT GTG 252
   Pro Thr Ile Tyr Ser Ile Ile Phe Leu Thr Gly Ile Val
               45                  50
   GGC AAT GGA TTG GTC ATC CTG GTC ATG GGT TAC CAG AAG 291
   Gly Asn Gly Leu Val Ile Leu Val Met Gly Tyr Gln Lys
   55                  60                  65
   AAA CTG AGA AGC ATG ACG GAC AAG TAC AGG CTG CAC CTG 330
   Lys Leu Arg Ser Met Thr Asp Lys Tyr Arg Leu His Leu
               70                  75                  80
   TCA GTG GCC GAC CTC CTC TTT GTC ATC ACG CTT CCC TTC 369
   Ser Val Ala Asp Leu Leu Phe Val Ile Thr Leu Pro Phe
                   85                  90
   TGG GCA GTT GAT GCC GTG GCA AAC TGG TAC TTT GGG AAC 408
   Trp Ala Val Asp Ala Val Ala Asn Trp Tyr Phe Gly Asn
       95                  100                 105
   TTC CTA TGC AAG GCA GTC CAT GTC ATC TAC ACA GTC AAC 447
   Phe Leu Cys Lys Ala Val His Val Ile Tyr Thr Val Asn
               110                     115
   CTC TAC AGC AGT GTC CTC ATC CTG GCC TTC ATC AGT CTG 486
   Leu Tyr Ser Ser Val Leu Ile Leu Ala Phe Ile Ser Leu
   120                     125                     130
   GAC CGC TAC CTG GCC ATC GTC CAC GCC ACC AAC AGT CAG 525
   Asp Arg Tyr Leu Ala Ile Val His Ala Thr Asn Ser Gln
           135                     140                     145
   AGG CCA AGG AAG CTG TTG GCT GAA AAG GTG GTC TAT GTT 564
   Arg Pro Arg Lys Leu Leu Ala Glu Lys Val Val Tyr Val
                   150                     155
   GGC GTC TGG ATC CCT GCC CTC CTG CTG ACT ATT CCC GAC 603
   Gly Val Trp Ile Pro Ala Leu Leu Leu Thr Ile Pro Asp
       160                     165                     170
   TTC ATC TTT GCC AAC GTC AGT GAG GCA GAT GAC AGA TAT 642
   Phe Ile Phe Ala Asn Val Ser Glu Ala Asp Asp Arg Tyr
               175                     180
   ATC TGT GAC CGC TTC TAC CCC AAT GAC TTG TGG GTG GTT 681
   Ile Cys Asp Arg Phe Tyr Pro Asn Asp Leu Trp Val Val
   185                     190                     195
   GTG TTC CAG TTT CAG CAC ATC ATG GTT GGC CTT ATC CTG 720
   Val Phe Gln Phe Gln His Ile Met Val Gly Leu Ile Leu
           200                     205                     210
   CCT GGT ATT GTC ATC CTG TCC TGC TAT TGC ATT ATC ATC 759
   Pro Gly Ile Val Ile Leu Ser Cys Tyr Cys Ile Ile Ile
                   215                     220
   TCC AAG CTG TCA CAC TCC AAG GGC CAC CAG AAG CGC AAG 798
   Ser Lys Leu Ser His Ser Lys Gly His Gln Lys Arg Lys
       225                     230                     235
   GCC CTC AAG ACC ACA GTC ATC CTC ATC CTG GCT TTC TTC 837
   Ala Leu Lys Thr Thr Val Ile Leu Ile Leu Ala Phe Phe
               240                     245
   GCC TGT TGG CTG CCT TAC TAC ATT GGG ATC AGC ATC GAC 876
   Ala Cys Trp Leu Pro Tyr Tyr Ile Gly Ile Ser Ile Asp
   250                     255                     260
   TCC TTC ATC CTC CTG GAA ATC ATC AAG CAA GGG TGT GAG 915
   Ser Phe Ile Leu Leu Glu Ile Ile Lys Gln Gly Cys Glu
           265                     270                     275
   TTT GAG AAC ACT GTG CAC AAG TGG ATT TCC ATC ACC GAG 954
   Phe Glu Asn Thr Val His Lys Trp Ile Ser Ile Thr Glu
                   280                     285
   GCC CTA GCT TTC TTC CAC TGT TGT CTG AAC CCC ATC CTC 993
   Ala Leu Ala Phe Phe His Cys Cys Leu Asn Pro Ile Leu
       290                     295                     300
   TAT GCT TTC CTT GGA GCC AAA TTT AAA ACC TCT GCC CAG 1032
   Tyr Ala Phe Leu Gly Ala Lys Phe Lys Thr Ser Ala Gln
               305                     310
   CAC GCA CTC ACC TCT GTG AGC AGA GGG TCC AGC CTC AAG 1071
   His Ala Leu Thr Ser Val Ser Arg Gly Ser Ser Leu Lys
   315                     320                     325
   ATC CTC TCC AAA GGA AAG CGA GGT GGA CAT TCA TCT GTT 1110
   Ile Leu Ser Lys Gly Lys Arg Gly Gly His Ser Ser Val
             330                 335                 340
   TCC ACT GAG TCT GAG TCT TCA AGT TTT CAC TCC AGC TAAC 1150
   Ser Thr Glu Ser Glu Ser Ser Ser Phe His Ser Ser
               345                   350     352
   ACAGATGTAA AAGACTTTTT TTTATACGAT AAATAACTTT TTTTTAAGTT 1200
   ACACATTTTT CAGATATAAA AGACTGACCA ATATTGTACA GTTTTTATTG 1250
   CTTGTTGGAT TTTTGTCTTG TGTTTCTTTA GTTTTTGTGA AGTTTAATTG 1300
   ACTTATTTAT ATAAATTTTT TTTGTTTCAT ATTGATGTGT GTCTAGGCAG 1350
   GACCTGTGGC CAAGTTCTTA GTTGCTGTAT GTCTCGTGGT AGGACTGTAG 1400
   AAAAGGGAAC TGAACATTCC AGAGCGTGTA GTGAATCACG TAAAGCTAGA 1450
   AATGATCCCC AGCTGTTTAT GCATAGATAA TCTCTCCATT CCCGTGGAAC 1500
   GTTTTTCCTG TTCTTAAGAC GTGATTTTGC TGTAGAAGAT GGCACTTATA 1550
   ACCAAAGCCC AAAGTGGTAT AGAAATGCTG GTTTTTCAGT TTTCAGGAGT 1600
   GGGTTGATTT CAGCACCTAC AGTGTACAGT CTTGTATTAA GTTGTTAATA 1650
   AAAGTACATG TTAAACTTAA AAAAAAAAAA AAAAAAAAAA AAAAAAAAAA 1700
   AAAAAAAAAA AAAGCGGCCG CCAGCACACT GGAATTC 1737
(2) INFORMATION FOR SEQ ID NO:3:
   (i) SEQUENCE CHARACTERISTICS:
      (A) LENGTH: 1679 bases
      (B) TYPE: nucleic acid
      (C) STRANDEDNESS: single
      (D) TOPOLOGY: linear
   (xi) SEQUENCE DESCRIPTION: SEQ ID NO:3:

## Description

This invention relates to the field of assaying platelet factor 4 superfamily members (hereafter "PF4A") and the preparation of agonists and antagonists to the members of this family.

### BACKGROUND OF THE INVENTION

While interleukin-8 was initially identified as a chemoattractant for neutrophils, and was known to bind a receptor on neutrophils⁸⁻¹⁰, it has in addition a wide range of pro-inflammatory activities including the stimulation of degranulation and the upregulation of the cell adhesion molecule MAC-1 and of the complement receptor CR1¹. IL-8 can also mediate the inhibition of the adherence of neutrophils to activated endothelial cells².

IL-8 is a member of a family of ten or more pro-inflammatory cytokines with an M,~10,000¹. This larger family of proteins is called the platelet factor 4 superfamily (Wolpe *et al.,* FASEB J. **3**:2565-73 [1989]). Some members of the platelet factor 4 superfamily, in general the subset referred to as CXC peptides (including IL-8), possess neutrophil agonist activity, e.g. NAP-2, MIP-2, platelet factor 4 and NAP-3 (MGSA/gro). Other members of this family, the C-C peptides, are not neutrophil agonists. Hereafter "PF4A" means the PF4 superfamily.

It is an object of this invention to identify receptors for the PF4A superfamily (hereinafter "PF4AR").

It is another object of this invention to obtain DNA encoding or hybridizing to these receptors, and to express the receptors in host cells.

It is an additional object of this invention to provide isolates of PF4AR for diagnostic and therapeutic purposes.

A still further object is to obtain DNA encoding variants of such receptors and to prepare such variants in recombinant cell culture.

These and other objects of this invention will be apparent from the specification as a whole.

### SUMMARY OF THE INVENTION

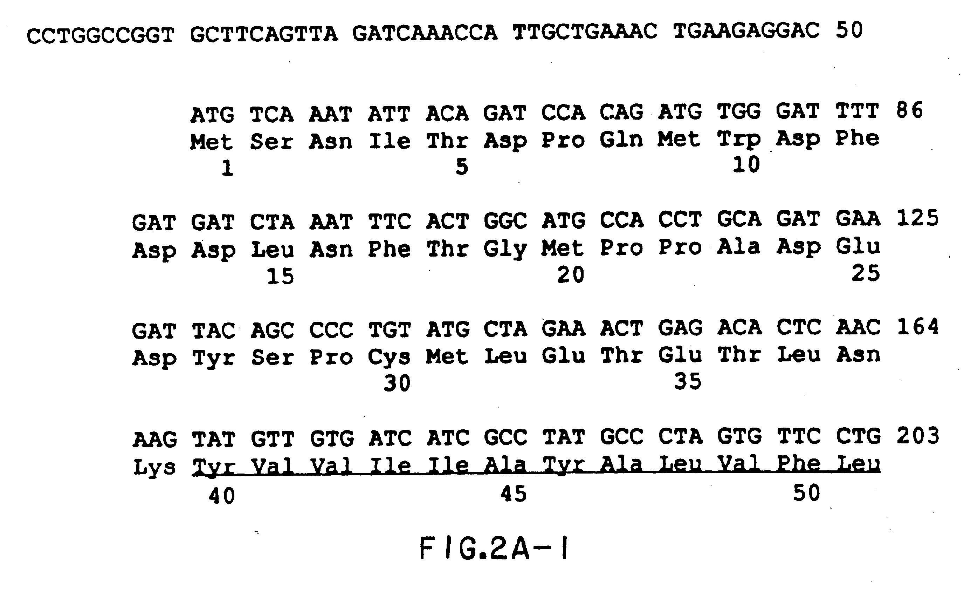

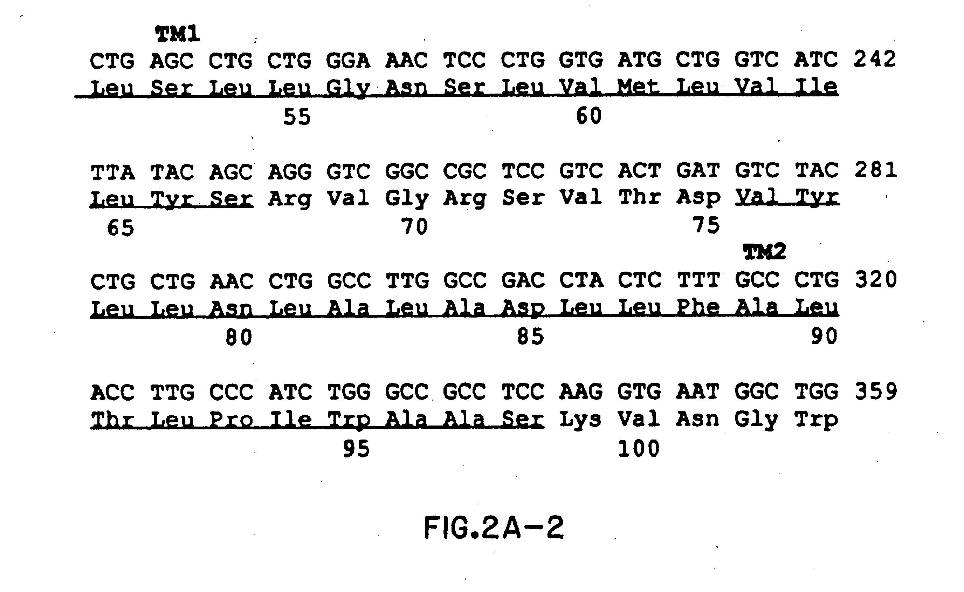

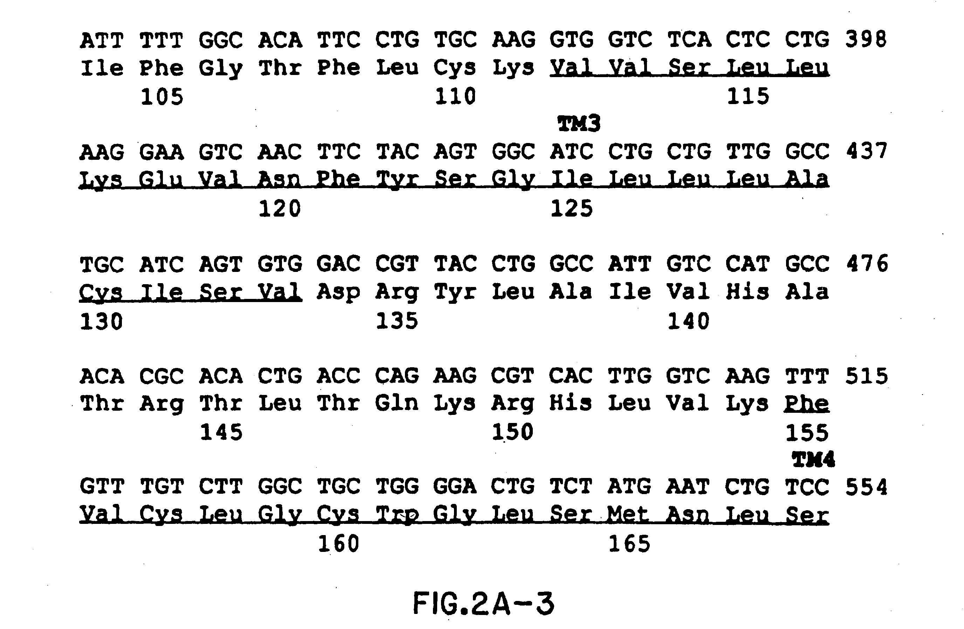

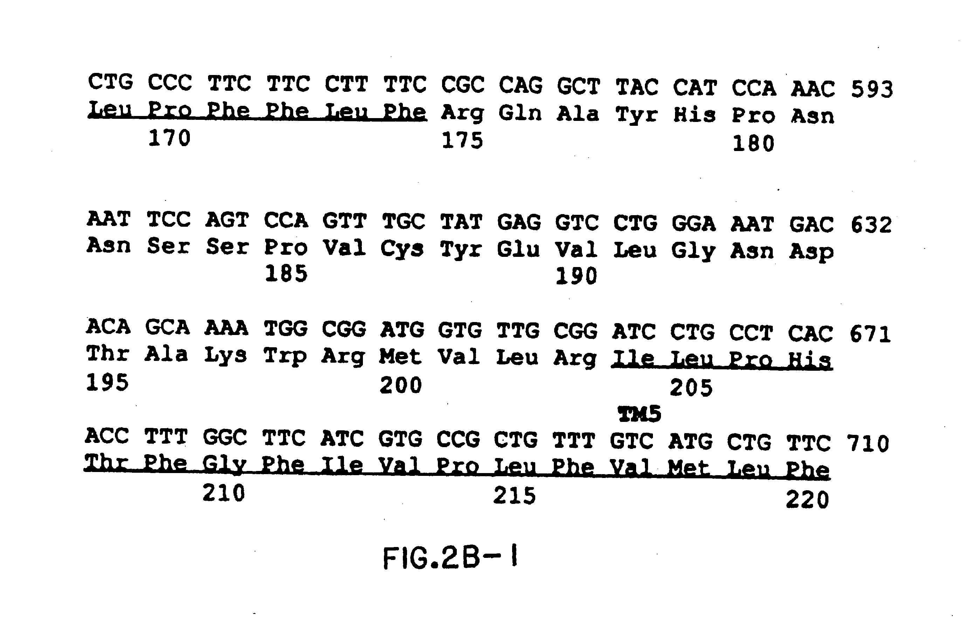

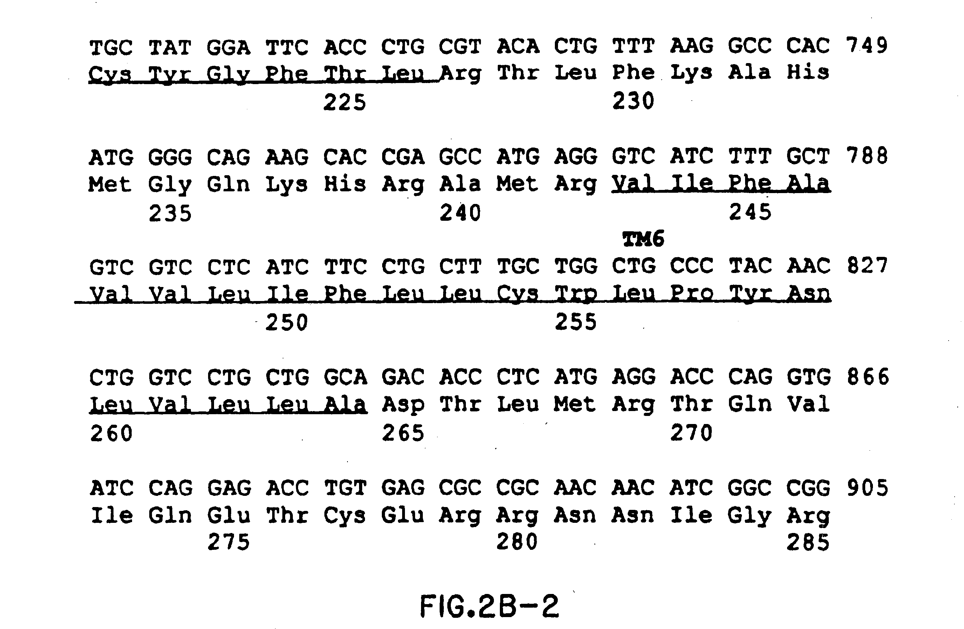

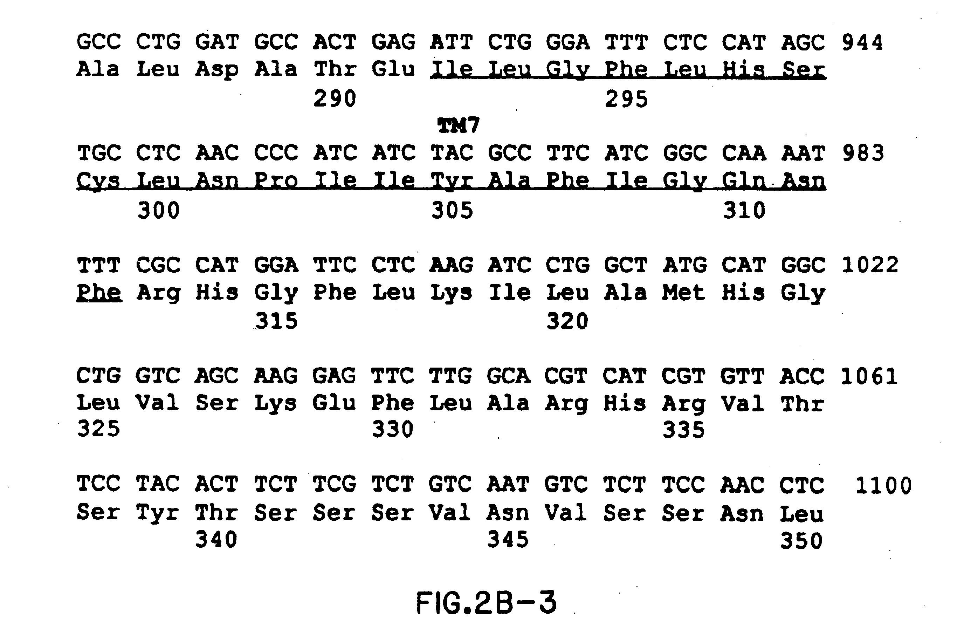

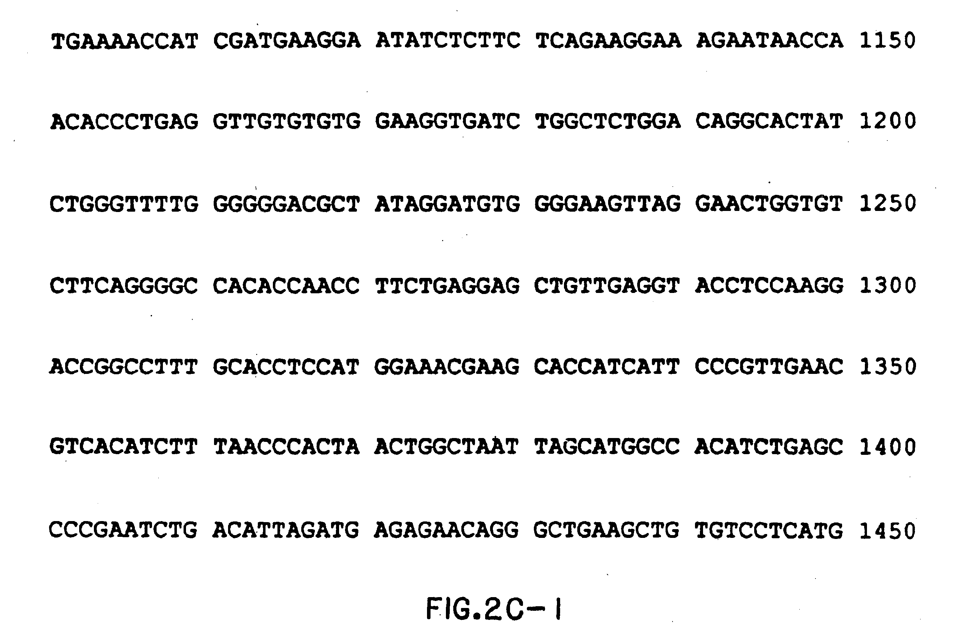

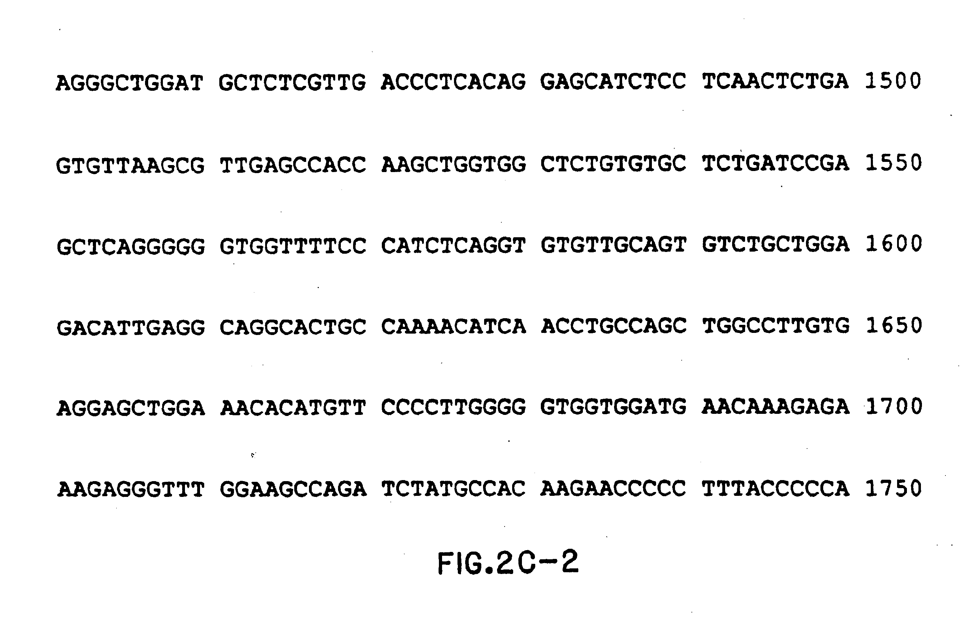

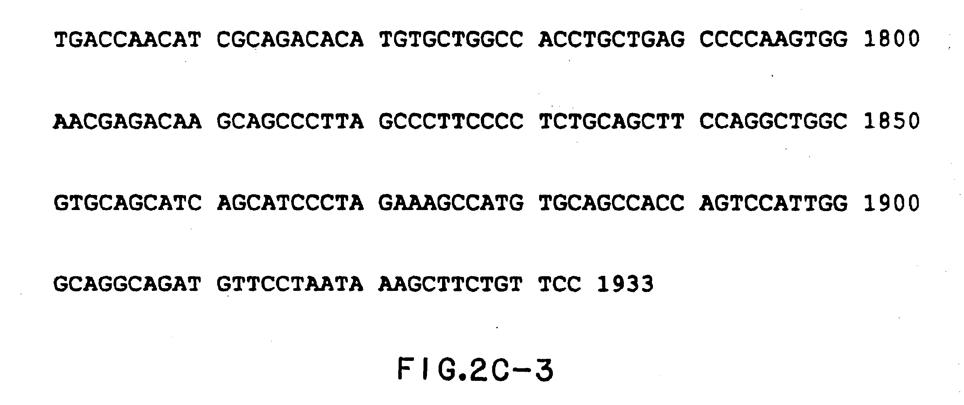

In a first aspect of the present invention there is provided an isolated PF4AR polypeptide having at least 85% amino acid sequence homology with the translated amino acid sequence of figure 2, 4 or 5.

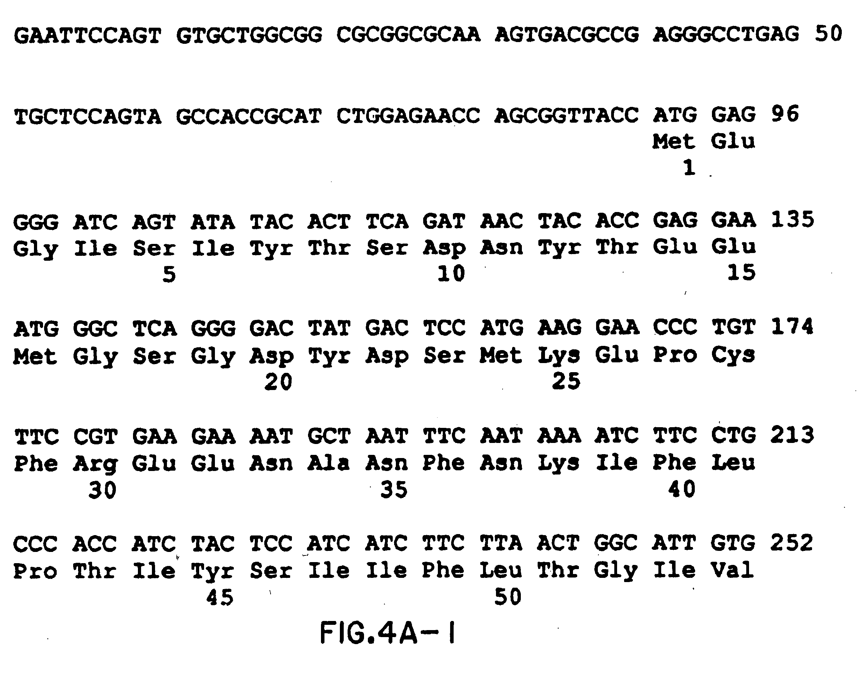

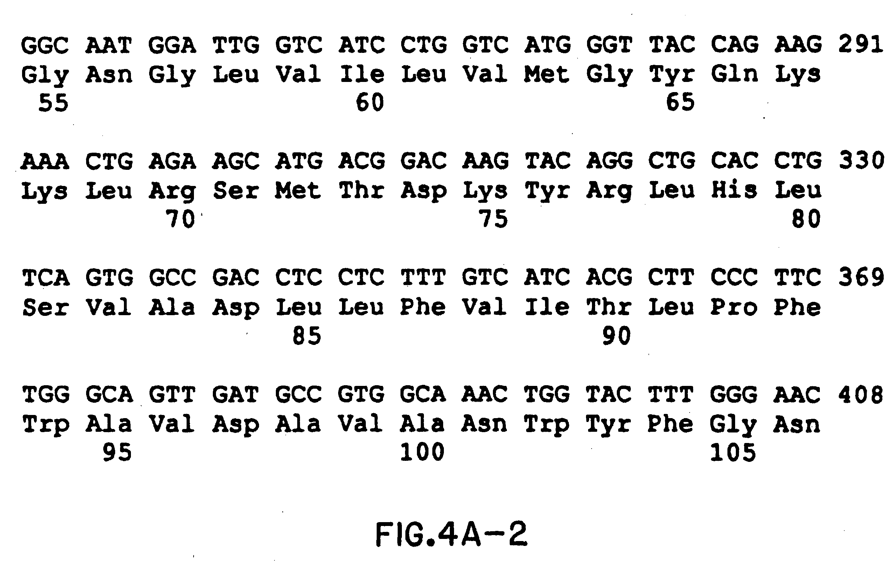

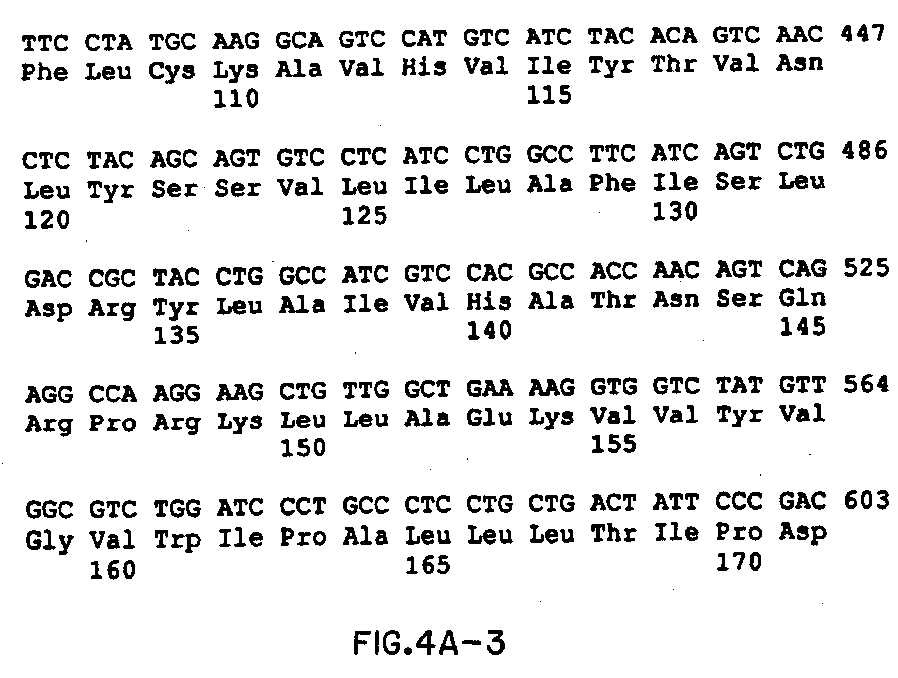

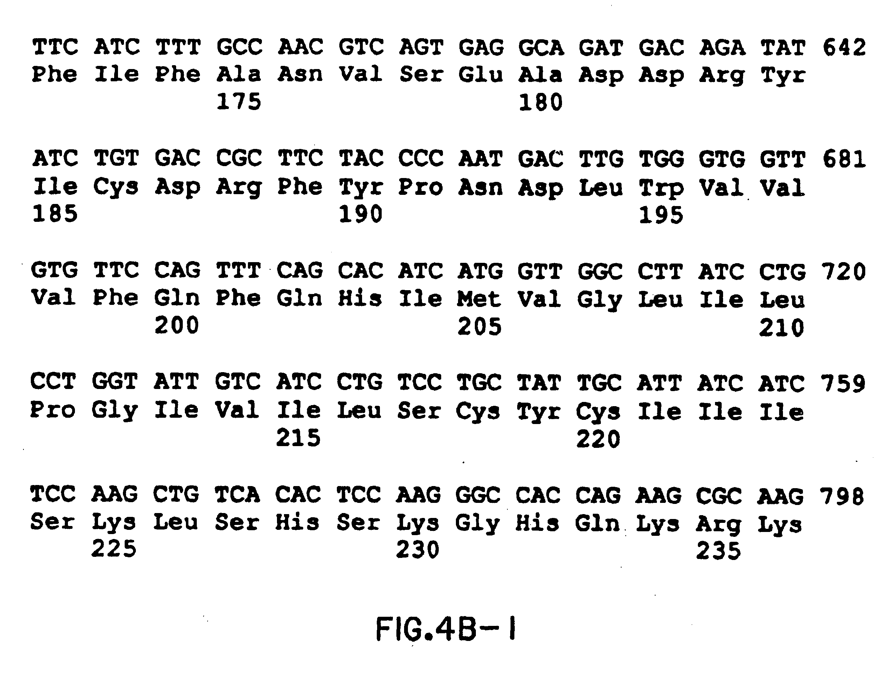

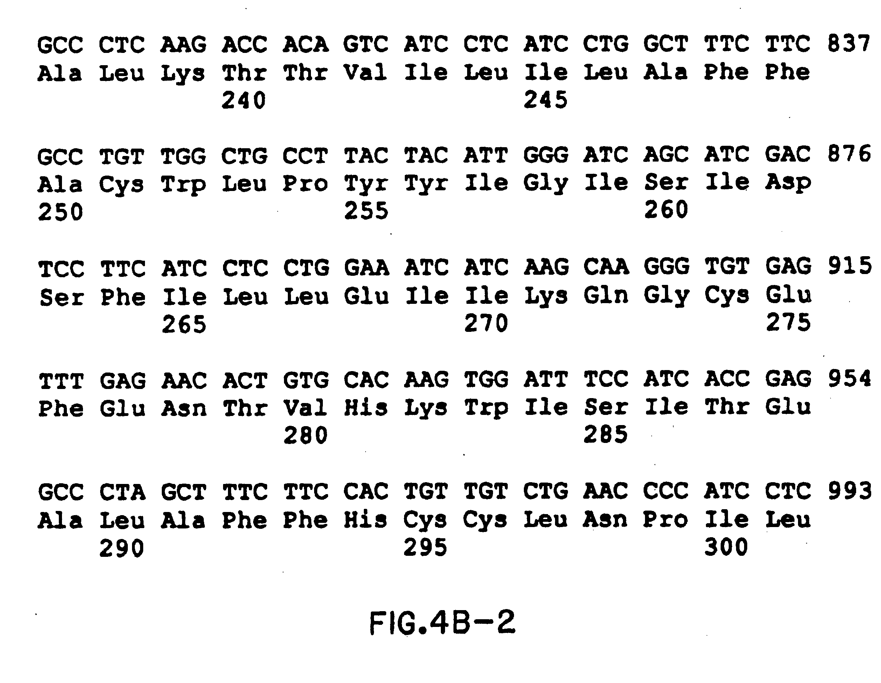

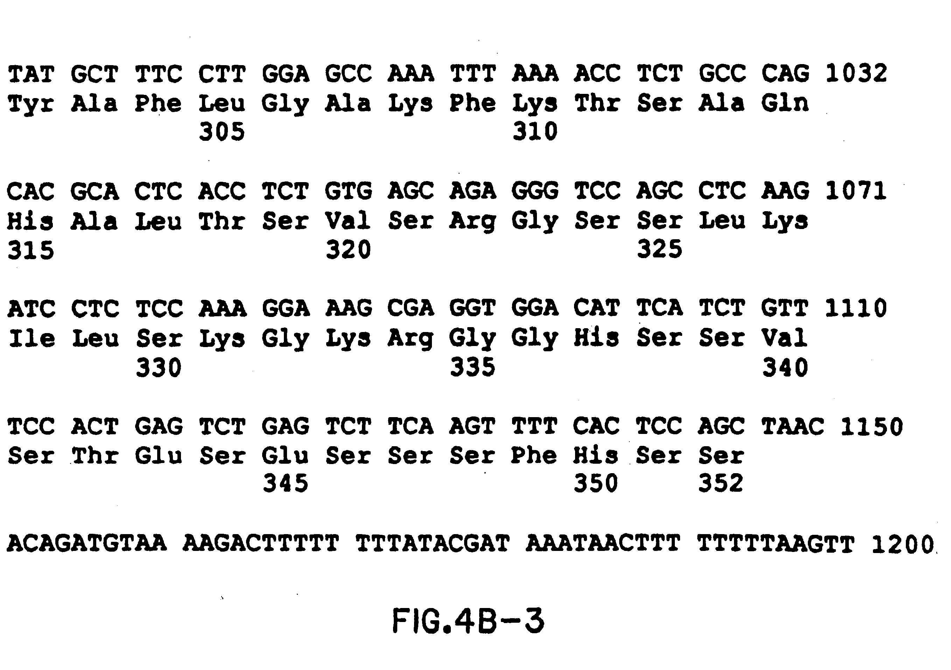

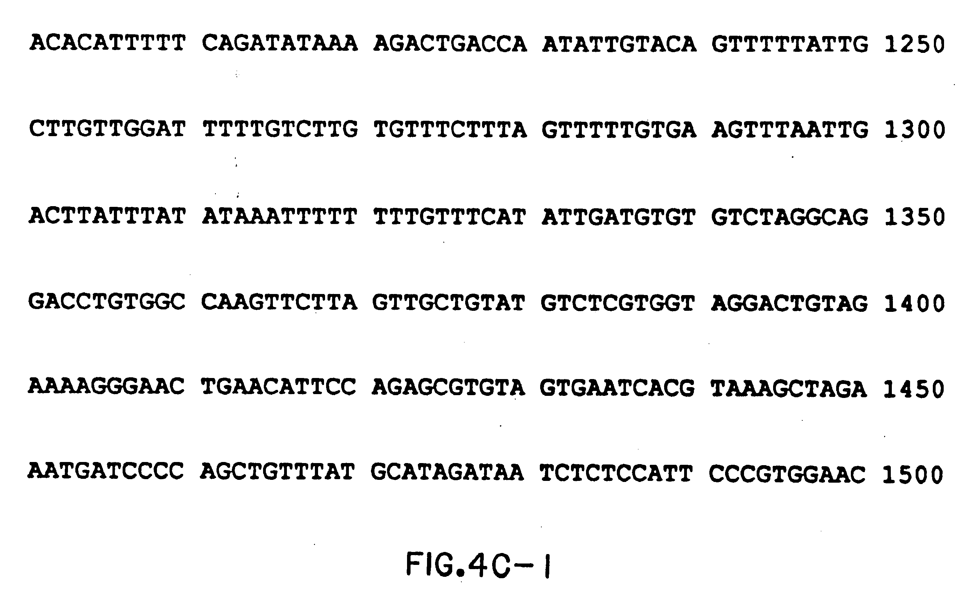

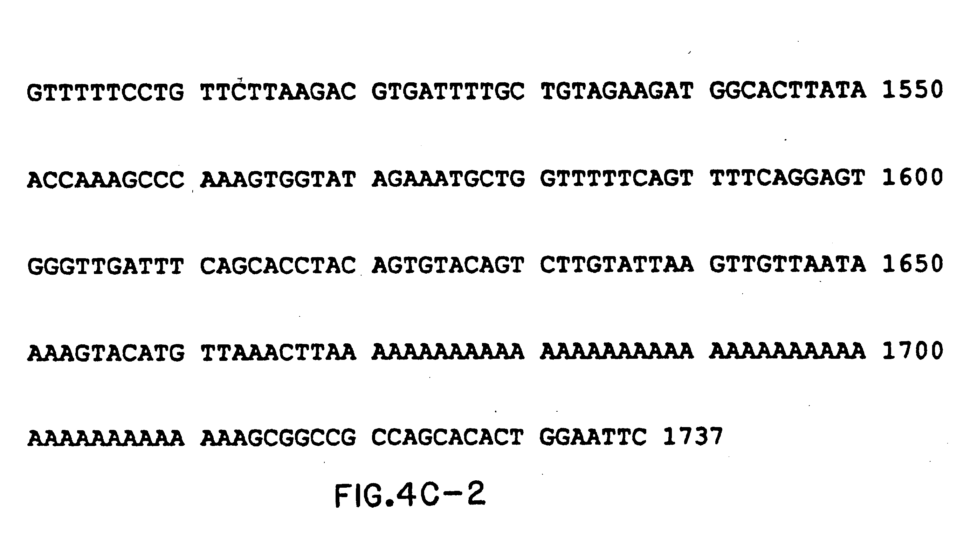

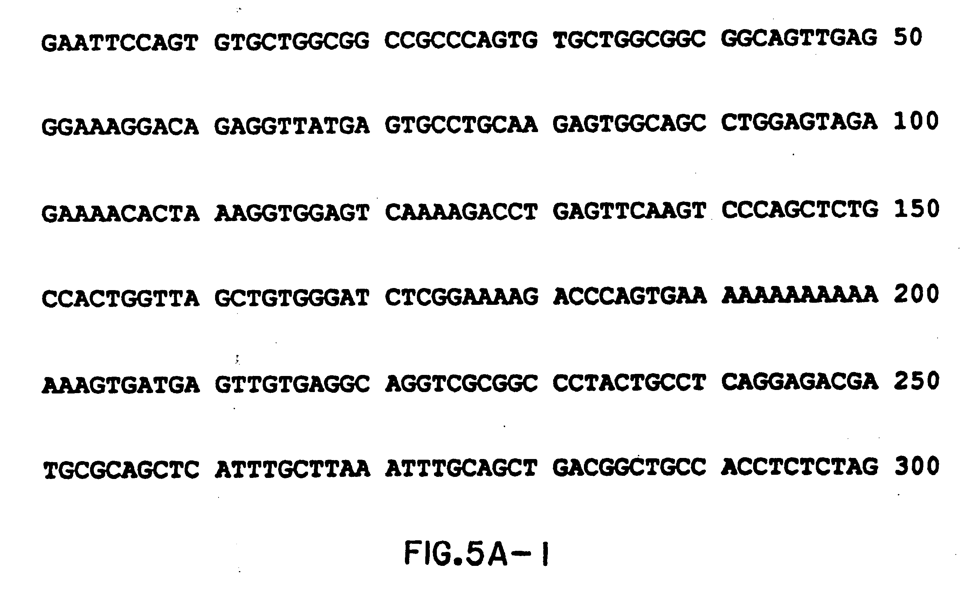

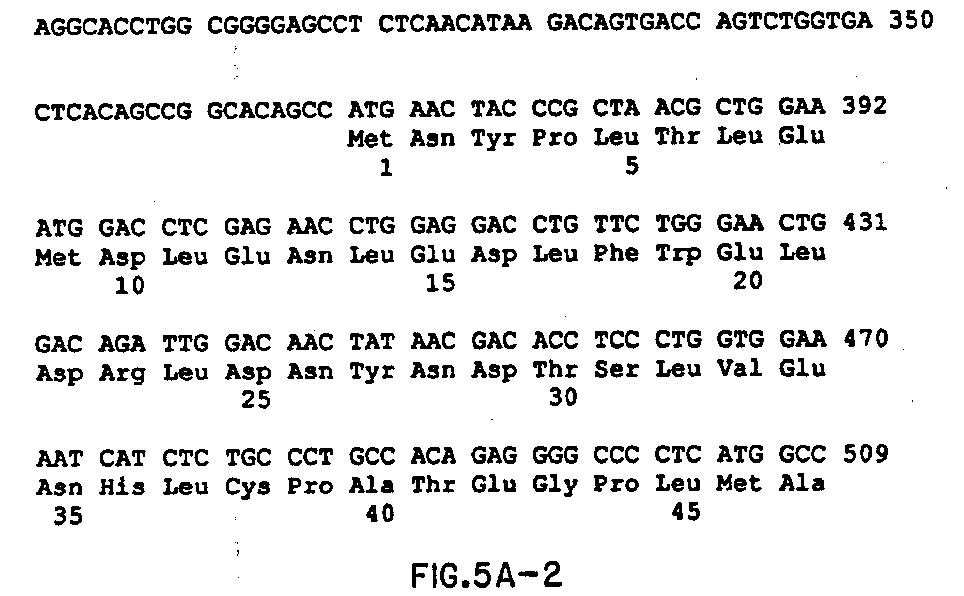

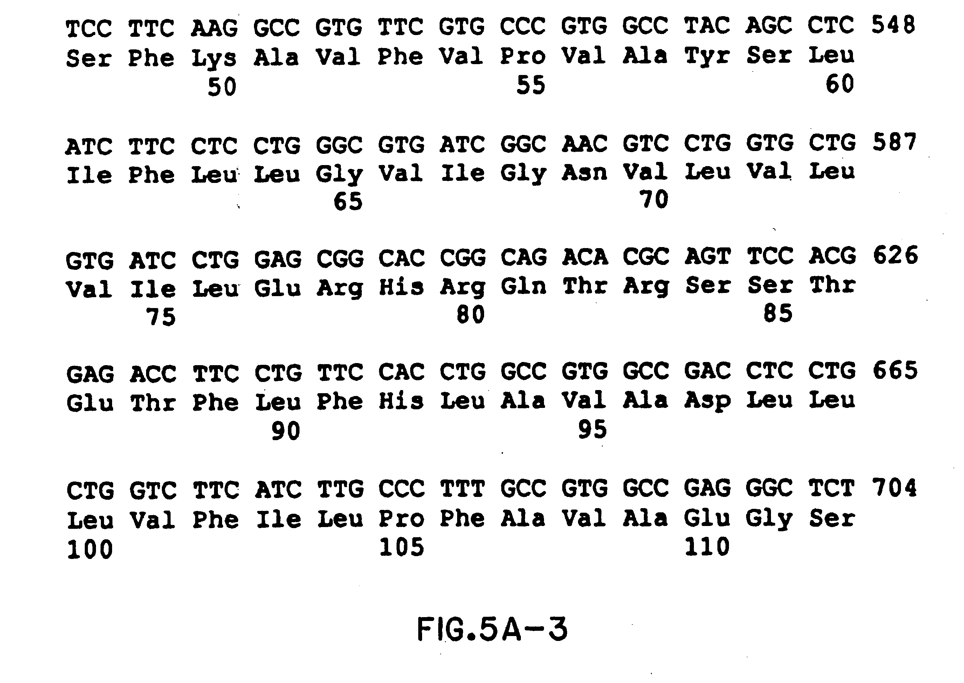

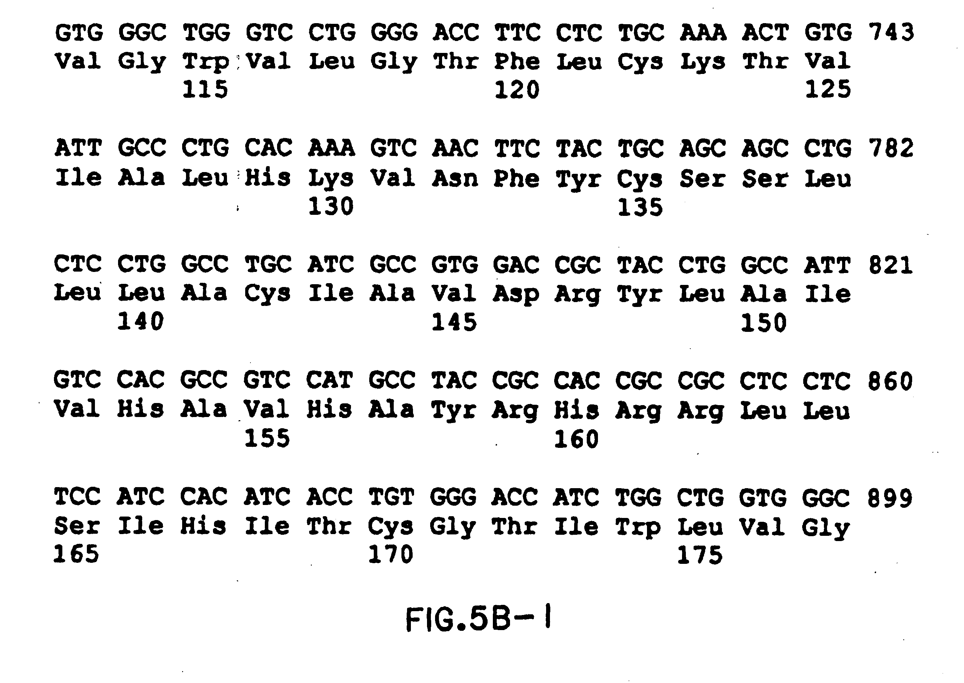

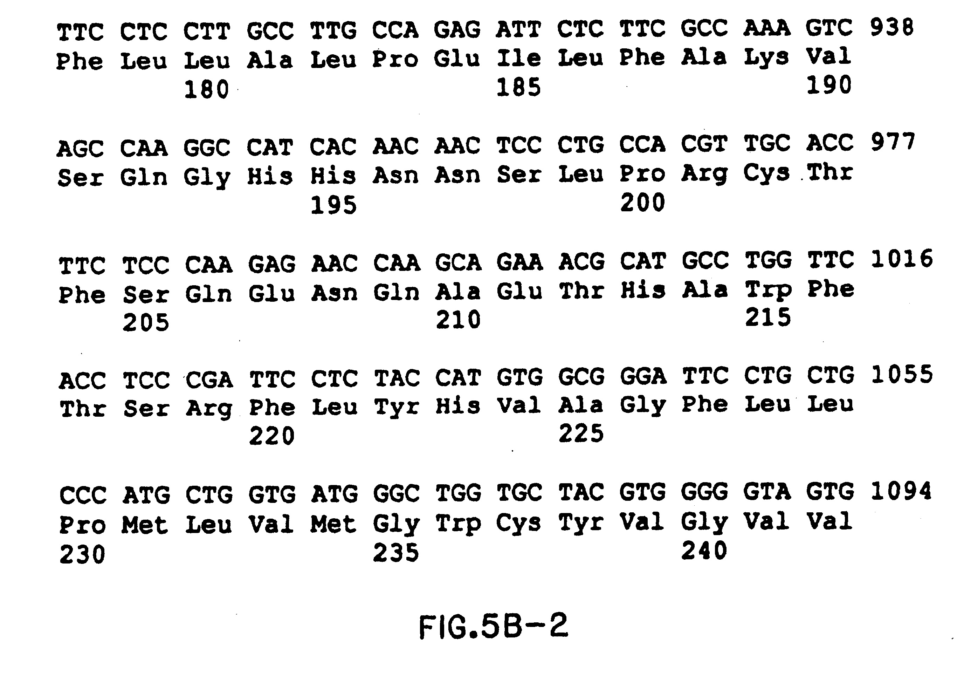

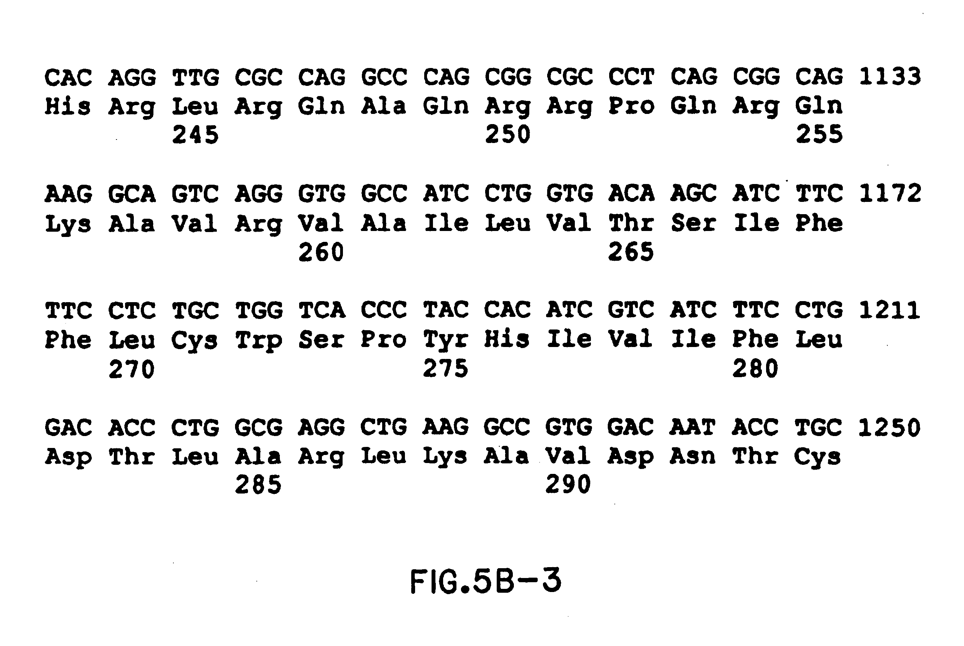

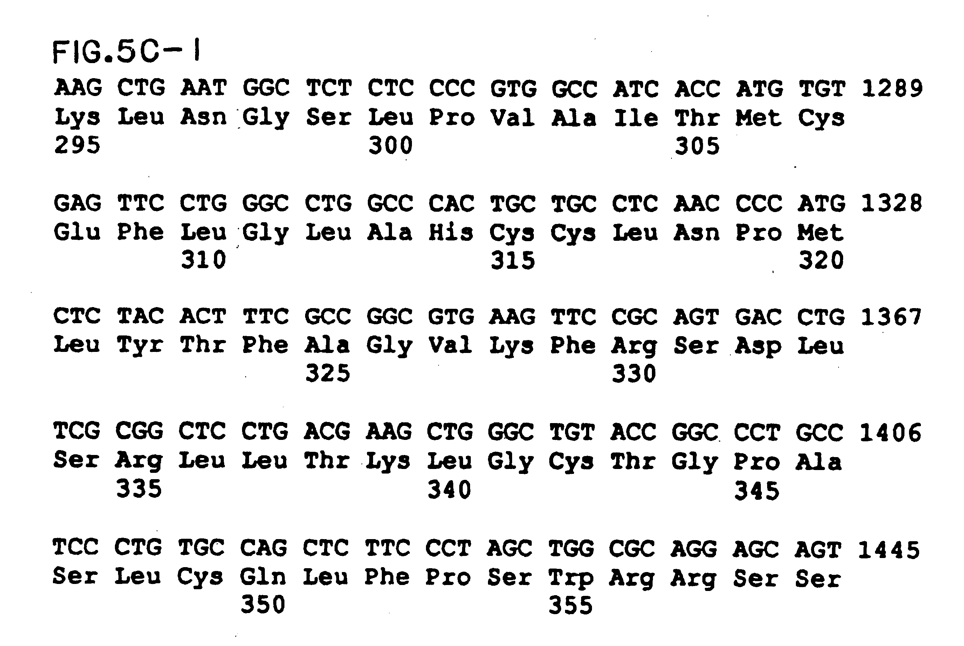

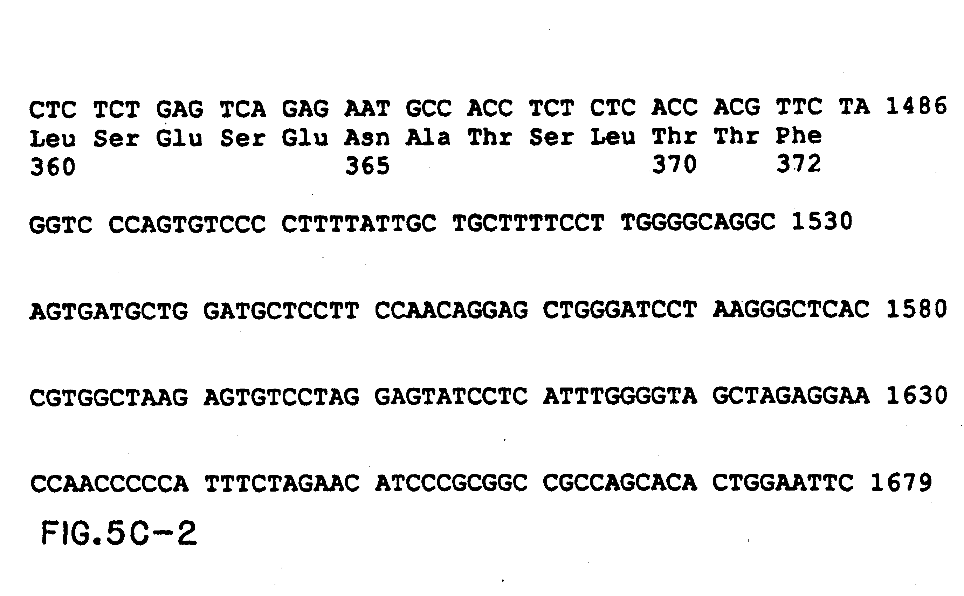

In a further aspect there is provided an isolated PF4AR polypeptide wherein the nucleic acid encoding the PF4AR polypeptide hybridises with the complement of the nucleic acid encoding the polypeptide of figures 4 or 5 under high stringency conditions.

In another aspect, the invention provides a composition comprising the PF4AR that is free of contaminating polypeptides of the animal species from which the PF4AR is derived.

The PF4AR or fragments thereof (which also may be synthesized by chemical methods) are fused (by recombinant expression or *in vitro* covalent methods) to an immunogenic polypeptide and this fusion polypeptide, in turn, is used to immunize an animal to raise antibodies against a PF4AR epitope. Anti-PF4AR antibodies are recovered from the serum of immunized animals. Alternatively, monoclonal antibodies are prepared from cells of the immunized animal in conventional fashion.

Anti-PF4AR antibodies are useful particularly in the diagnosis (*in vitro* or *in vivo*) or (when immoblilized on an insoluble matrix) the purification of the PF4AR.

Substitutional, deletional, or insertional variants of the PF4AR are prepared by *in vitro* or recombinant methods and screened for immuno-crossreactivity with the PF4AR and for PF4AR antagonist or agonist activity.

The PF4AR also is derivatized *in vitro* to prepare immobilized PF4AR and labeled PF4AR, particularly for purposes of diagnosis of PF4AR or its antibodies, or for affinity purification of PF4AR antibodies.

The PF4AR, its derivatives, or its antibodies are formulated into physiologically acceptable vehicles, especially for therapeutic use. Such vehicles include sustained-release formulations of the PF4AR.

In still other aspects, the invention provides an isolated nucleic acid molecule encoding the PF4AR, labeled or unlabeled, and a nucleic acid sequence that is complementary to, or hybridizes under suitable conditions to a nucleic acid sequence encoding the PF4AR.

In addition, the invention provides a replicable vector comprising the nucleic acid molecule encoding the PF4AR operably linked to control sequences recognized by a host transformed by the vector; host cells transformed with the vector; and a method of using a nucleic acid molecule encoding the PF4AR to effect the production of PF4AR, comprising expressing the nucleic acid molecule in a culture of the transformed host cells and recovering the PF4AR from the host cell culture. The nucleic acid sequence is also useful in hybridization assays for PF4AR nucleic acid. The recombinant host cells are particularly useful in assaying the appropriate PF4A members.

In further embodiments, the invention provides a method for producing PF4AR comprising inserting into the DNA of a cell containing the nucleic acid encoding the PF4AR a transcription modulatory element in sufficient proximity and orientation to the PF4AR nucleic acid to influence or destroy transcription of DNA encoding a biologically active PF4AR, with an optional further step comprising culturing the cell containing the transcription modulatory element and the PF4AR nucleic acid.

### BRIEF DESCRIPTION OF THE DRAWINGS

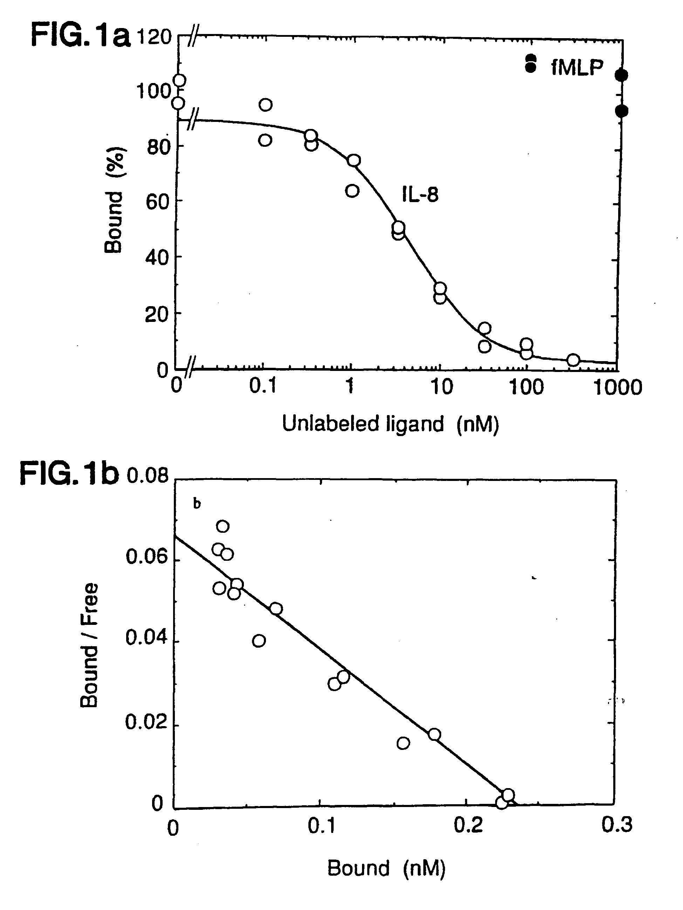

Fig. 1 depicts the high affinity binding of IL-8 to COS cells transfected with clone pRKSB.il8r1.1. a, Competition with unlabelled IL-8 or fMLP. b, Scatchard analysis of the IL-8 competition data; apparent Kd = 3.6 nM, average of 820,000 binding sites/cell. Similar competitions with human neutrophils gave Kd = 1.1 nM, 31000 binding sites/cell.
Figs. 2a-2c (SEQ ID NO. 1) (hereinafter referred to collectively as Fig. 2) depicts the amino acid and nucleotide sequences of the IL-8 receptor cDNA insert from clone pRKSB.il8rl.1. The seven putative transmembrane domains are shown. There are 4 extracellular segments and 4 intracellular segments, each being separated by one of the transmembrane domains. The extracellular segments are approximately delineated by residues 1-39, 99-111, 134-154, 175-203 and 265-290. The IL-8 receptor contains 3 potential N-linked glycosylation sites in the first extracellular region and 3 more in the third extracellular loop.

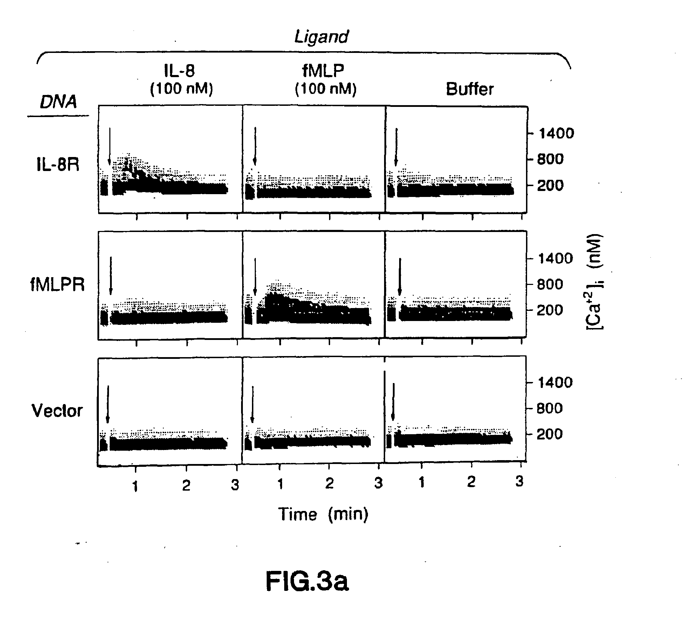

Fig. 3a depicts the flow cytometry determination of the intracellular Ca⁺⁺ response of transfected human IL-8 and fMLP receptors to their ligands. Human embryonic kidney 293 cells were transtected by electroporation¹⁹ with IL-8 receptor (clone pRK5B.il8r1.1), fMLP receptor (human fMLP receptor cDNA³ in the vector pRK5), or vector (pRK5B¹⁸) DNA. After two days, the cells were loaded with 2 µM indo-1 acetoxymethyl ester in RPMI medium for 30 mm at 37°. Intracellular Ca + + was measured with a Coulter 753 flow cytometer using the ratio of 405 and 525 nm fluorescence²³.

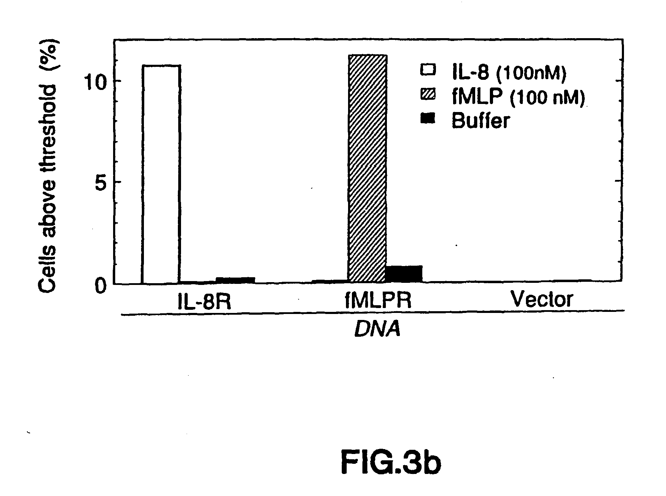

Fig. 3b illustrates the percent of cells above 400 nM Caᵢ⁺⁺ for the time period after addition of IL-8 (about 15 sec. into each run).
Figs. 4 (SEQ ID NO.2) and 5 (SEQ ID NO.3) depict the DNA and an imputed polypeptide sequences for two additional PF4AR members identified by probing lambda libraries from a human monocyte-like cell line (HL-60) and human PBLs using a large fragment of the IL-8 receptor DNA.

### Detailed Description of the Invention

We have isolated by expression cloning a cDNA encoding the human neutrophil IL-8 receptor together with two other homologous receptors. The amino acid sequence shows that the IL-8 receptor is a member of the G-protein coupled receptor family with clear similarity (29 % amino acid identity) to the human neutrophil receptors for the chemoattractants f-Met-Leu-Phe³ and C5a⁴. Although the IL-8 receptor sequence may be the human homologue of what has been identified as the isoform of the rabbit f-Met-Leu-Phe receptor⁵, we show that when transfected into mammalian cells, this receptor clone confers high affinity binding to IL-8 and produces a transient Ca⁺⁺ mobilization in response to IL-8 with no binding or response to f-Met-Leu-Phe.

A COS cell expression cloning strategy^{6.7} was used to isolate clones encoding the IL-8 receptor. A cDNA library constructed from human neutrophil mRNA in the mammalian expression vector pRK5B was transfected into COS-7 cells as pools of 2500 clones, and the cells screened for the binding of ¹²⁵I-IL-8. One positive pool from the first 58 transfections was partitioned into smaller pools until a pure clone (pRK5B.il8r1.1) was obtained. Figure 1 shows the competition of ¹²⁶I-IL-8 binding by unlabelled IL-8 to COS cells transfected with the isolated clone. Analysis of this data gives a Kd of 3.6 nM for IL-8 binding which is within the range of 0.8 to 4 nM reported for IL-8 binding to human neutrophils⁸⁻¹⁰. There is no competition of the IL-8 binding by the chemotactic peptide f-Met-Leu-Phe (fMLP).

The DNA sequence of the isolated cDNA clone (Fig. 2) contains a single long open reading frame beginning with a methionine residue that matches the consensus expected for a translation initiation site¹¹. This open reading frame encodes a protein of 350 amino acids (translated M, 39.5 kD). The amino add sequence shares several features with the G-protein coupled receptors of the rhodopsin superfamily including seven hydrophobic domains that are presumed to span the cell membrane and N-linked glycosylation sites near the N-terminus¹² (see below).

The encoded amino acid sequence is the most similar to a recently cloned sequence for the rabbit fMLP receptor⁵. The similarity is sufficiently high (79% amino acid identity overall with multiple stretches of more than 20 contiguous amino add matches) that these two sequences may well be species homologs of the same receptor. The human fMLP receptor has also been cloned³; it has only 26% amino acid identity with the rabbit fMLP receptor (and 29% identity to the human IL-8 receptor presented here). The considerable divergence between the rabbit and human fMLP receptor amino acid sequences has lead to the suggestion in the art (now believed to be possibly erroneous) that these may be two isoforms of the fMLP receptor⁵.

Neutrophils respond to the chemoattractants IL-8 and fMLP with a rapid, transient increase in the intracellular free Ca⁺⁺ concentration^{1,13}. In order to verify the identification of the clone isolated here as the IL-8 receptor, we have determined the intracellular Ca⁺⁺ response of transfected cells to added IL-8 as well as fMLP We have used parallel experiments with transfected human fMLP receptor or with the expression vector as controls. Flow cytometer analysis shows a clear transient increase in intracellular Ca⁺⁺ for the transfected IL-8 receptor in response to IL-8. No response is found to fMLP. Conversely, cells transfected with the human fMLP receptor respond to fMLP but not to IL-8. No response to either chemoattractant is found in vector transfected cells. Only a subset of the cells are expected to respond in these experiments as the transfection efficiency is estimated to be 15-25%. Binding experiments¹⁴ also failed to detect any binding of ³H-fMLP to the expressed IL-8 receptor or ¹²⁶I-IL-8 to the expressed human fMLP receptor. These experiments clearly demonstrate the specificity of the two receptors for their respective ligands; a result expected based on the lack of binding competition between IL-8 and fMLP for neutrophils¹. These results also demonstrate that the cloned receptors function in second message signaling in response to ligand binding.

Blot hybridization of the cloned IL-8 receptor cDNA to human neutrophil mRNA, shows strong bands of 2.4 and 3.0 kb as well as a fainter band at 3.5 kb. While it is clear from the DNA sequence data presented in Fig. 2 that the mRNA for the receptor has a long 3' untranslated region, additional work will be needed to establish whether the multiple RNA bands are due to multiple polyadenylation sites. No hybridization was detected to mRNA from U266 or Jurkat cell lines, which are of the B cell and T cell lineages. No hybridization was found for mRNA from the monocyte cell line U937 as well, in spite of the reports of low levels of IL-8 binding to these cells^{9,10}.

Alignment of the receptor sequences for the three neutrophil chemoattractants IL-8, fMLP³, and C5a⁴ shows that they form a subfamily of the G-protein coupled receptors with 29-34 % amino acid identity. This subfamily has a short third intracellular loop as compared with other G-protein coupled receptors such as the β-adrenergic¹² or muscarinic acetylcholine receptors¹⁵. This loop contains determinants at least partially responsible for the binding of G-proteins to the receptors¹². The intracellular C-terminal region of the IL-8 receptor, while not very similar to that of the fMLP and C5a receptors does preserve a high number of serine and threonine residues that may function as phosphorylation sites. As has been noted for the C5a receptor⁴, the N-terminal extracellular region for the IL-8 receptor has several acidic residues. These may aid in the binding of IL-8 which is quite basic (pl ~ 9.5).

### I. Definitions

In general, the following words or phrases have the indicated definition when used in the description, examples, and claims:

The term "PF4AR" is defined as a polypeptide having a qualitative biological activity in common with the polypeptides of Figs. 2. 4, or 5. Optionally PF4AR will have at least 30% and ordinarily 75% amino acid sequence identity with any of the polypeptides of Figs. 2, 4 or 5. PF4AR excludes the rabbit fMLP receptor⁵, the human fMLP receptor³, the human C5a receptor¹.

Identity or homology with respect to a PF4AR is defined herein to be the percentage of amino acid residues in the candidate sequence that are identical with the residues in Figs. 2, 4 or 5 after aligning the sequences and introducing gaps, if necessary, to achieve the maximum percent homology, and not considering any conservative substitutions as representing residue identity. No N- nor C-terminal extensions, deletions nor insertions shall be construed as reducing identity or homology.

PF4AR qualitative biological activity is defined as any one of (1) immunological cross-reactivity with at least one epitope of a polypeptide set forth in Figs. 2, 4, or 5; (2) the ability to specifically bind to a member of the PF4 super-family; or (3) any effector or functional activity of the Figs. 2, 4 or 5 polypeptides as found in nature, including their ability to bind any ligands other than superfamily members.

Immunologically cross-reactive as used herein means that the candidate polypeptide is capable of competitively inhibiting the binding of a PF4AR to polyclonal antibodies or antisera raised against a PF4AR. Such antibodies and antisera are prepared in conventional fashion by injecting an animal such as a goat or rabbit, for example, subcutaneously with the known native PF4AR in complete Freund's adjuvant, followed by booster intraperitoneal or subcutaneous injection in incomplete Freund's.

Included within the scope of the PF4AR as that term is used herein are polypeptides having the amino acid sequences described in Figs. 2, 4 or 5, amino acid sequence variants of such amino acid sequences, glycosylation variants of the polypeptides and covalent modifications of the polypeptides. Each of these are described in more detail below.

"Isolated" PF4AR nucleic acid or polypeptide is a PF4AR nucleic acid or polypeptide that is identified and separated from at least one contaminant (nucleic acid or polypeptide respectively) with which it is ordinarily associated in nature, such as from the animal or human source of the PF4AR nucleic acid or polypeptide. In preferred embodiments, the PF4AR will be isolated to pharmaceutically acceptable levels of purity with respect to proteins of its species of origin. In preferred embodiments, PF4AR protein will be purified (1) to greater than 95% by weight of protein as determined by the Lowry method, and most preferably more than 99% by weight, (2) to a degree sufficient to obtain at least 15 residues of N-terminal or internal amino acid sequence by an amino acid sequenator commercially available on the filing date hereof, or (3) to homogeneity by conventional nonreducing SDS-PAGE using Coomassie blue or, preferably, silver stain. Isolated PF4AR includes PF4AR in situ within recombinant cells since, in this instance, at least one component of the PF4AR natural environment will not be present. Isolated PF4AR includes PF4AR from one species in a recombinant cell culture of another species since the receptor in such circumstances will be devoid of source polypeptides. Ordinarily, however, isolated receptor will be prepared by at least one purification step.

Isolated PF4AR nucleic acid includes a nucleic acid that is identified and separated from at least one containment nucleic acid with which it is ordinarily associated in the natural source of the receptor nucleic acid. Isolated PF4AR nucleic acid thus is present in other than in the form or setting in which it is found in nature. However, isolated receptor-encoding nucleic acid includes PF4AR nucleic acid in ordinarily receptor-expressing cells where the nucleic acid is in a chromosomal location different from that of natural cells or is otherwise flanked by a different DNA sequence than that found in nature.

The nucleic acid or polypeptide may be labeled for diagnostic and probe purposes, using a label as described and defined further below in the discussion of diagnostic assays.

PF4AR "nucleic acid" is defined as RNA or DNA containing greater than ten bases that encodes a polypeptide sequence within Figs. 2, 4 or 5, is complementary to nucleic acid sequence of Figs. 2, 4 or 5, hybridizes to such nucleic acid and remains stably bound to it under low stringency conditions, or encodes a polypeptide sharing at least 30% sequence identity, preferably at least 75%, and more preferably at least 85%, with the translated amino acid sequence shown in Figs. 2, 4 or 5 or a fragment thereof. Preferably the DNA which hybridizes to the nucleic acid of Figs. 2, 4 or 5 contain at least 20, more preferably 40, and more preferably 60 bases. Most preferably, the hybridizing DNA or RNA contains 45 or even more preferably 90 bases. Such hybridizing or complementary nucleic acid, however, is defined further as being novel and unobvious over any prior art nucleic acid including that which encodes, hybridizes under low stringency conditions, or is complementary to nucleic acid encoding rabbit fMLP receptor⁵, human fMLP receptor or (optionally) the IL-8 receptor of Murphy *et al.* (*supra*).

"High stringency conditions" are any of those that (1) employ low ionic strength and high temperature for washing, for example, 0.015 M NaCl/0.0015 M sodium citrate/0.1% NaDodSO₄ at 50°C; (2) employ during hybridization 50% (vol/vol) formamide with 0.1% bovine serum albumin/0.1% Ficoll/0.1% polyvinylpyrrolidone/50mM sodium phosphate buffer at pH 6.5 with 750 mM NaCl, 75 mM sodium citrate at 42°C; or (3) employ hybridization with 50% formamide, 5 x SSC (0.75 M NaCl, 0.075 M sodium citrate), 50 mM sodium phosphate (pH 6.8), 0.1% sodium pyrophosphate, 5 x Denhardt's solution, sonicated salmon sperm DNA (50 µg/ml), 0.1% SDS, and 10% dextran sulfate at 42°C, with washes at 42°C in 0.2 x SSC and 0.1% SDS. Conditions of low stringency are set forth in Example 2.

The term "control sequences" refers to DNA sequences necessary for the expression of an operably linked coding sequence in a particular host organism. The control sequences that are suitable for prokaryotes, for example, include a promoter, optionally an operator sequence, and a ribosome binding site. Eukaryotic cells are known to utilize promoters, polyadenylation signals, and enhancers.

Nucleic acid is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous and, in the case of a secretory leader, contiguous and in reading phase. However enhancers do not have to be contiguous. Linking is accomplished by ligation at convenient restriction sites. If such sites do not exist, then synthetic oligonucleotide adaptors or linkers are used in accord with conventional practice.

The starting plasmids herein are commercially available, are publicly available on an unrestricted basis, or can be constructed from such available plasmids in accord with published procedures. In addition, other equivalent plasmids are known in the art and will be apparent to the ordinary artisan. Methods for restriction enzyme digestion, recovery or isolation of DNA, hybridization analysis, and ligation are conventional and by this time well known to the ordinary artisan.

"Recovery" or "isolation" of a given fragment of DNA from a restriction digest means separation of the digest on polyacrylamide or agarose gel by electrophoresis, identification of the fragment of interest by comparison of its mobility versus that of marker DNA fragments of known molecular weight, removal of the gel section containing the desired fragment, and separation of the gel from DNA. This procedure is known generally. For example, see Lawn *et al.,* Nucleic Acids Res. **9**: 6103-6114 (1981), and Goeddel *et al.,* Nucleic Acids Res. **8**:4057 (1980).

### II. Suitable Methods for Practicing the Invention

### 1. Preparation of Native PF4AR and Variants

### A. Isolation of DNA Encoding PF4AR

The DNA encoding of the PF4AR may be obtained from any cDNA library prepared from tissue believed to contain the PF4AR mRNA, generally HL60 or PBL libraries. The PF4AR gene may also be obtained from a genomic library. Libraries are screened with probes designed to identify the gene of interest or the protein encoded by it. We have described the entire cDNA for the IL-8 receptor and two homologous receptors. Nucleic acid encoding this family of receptors is readily obtained under low stringency conditions from genomic DNA or neutrophil cDNA libraries using probes having oligonucleotide sequences from the receptor gene sequences of Figs. 2, 4 or 5. These probes usually will contain about 500 or more bases. Since the probes will hybridize perfectly to the three exemplified DNAs, there is no need to use probe pools containing degenerate sequences. Screening with the probes to identify the Figs. 2, 4 or 5 receptors is more efficient if performed under conditions of high stringency.

Other PF4ARs other than those in Figs. 2, 4 or 5 are believed to exist and to contain regions of homology to the exemplified receptors. Thus probes having the sequences of the DNAs in Figs. 2, 4 or 5 can be used to screen for these receptors as well. The best candidates for probes are long sequences (greater than about 100 bases) that represent sequences that are highly homologous among the three exemplified human receptors. IL-8 cDNA encoding the IL-8 residues 15-34, 78-94, 176-193, 264-282 and 299-312 (and comparable probes from other receptors of the IL-8R family) are useful, particularly in probing for IL-8 receptor DNA. Probes useful for the receptor of Fig. 4 (and isolated proteins characteristic of the Fig. 5 receptor) are represented by sequences comprising residues 1-48, 77-92, 107-137, 156-177, 189-226, 239-257 and 271-315. Homologous probes and residues of the Fig. 4 receptor also are useful, i.e. residues 1-35, 64-78, 94-124, 143-164, 176-197, 219-239 and 251-295. cDNAs comprising cDNA encoding the following regions of the Figs. 2, 4 or 5 polypeptides are useful in probing for other receptors: 92-106, 57-72, 138-154, 314-329 and 57-154.

In general, one first identifies a cell which is capable of specifically binding or which is activated by a given PF4A, typically by *in vitro* bioassays and, optionally, by cell binding analysis using the labelled PF4A. Cells identified by this process (and some are already known for individual PF4As) therefore are expressing a receptor for this PF4A. A cDNA library is prepared from such cells and is screened using the receptor probes by procedures that are conventional per se. In this instance, however, it is preferred to use low stringency conditions (such as those in Example 2) and then analyze the resulting positive clones for homology to the Figs. 2, 4 or 5 receptors. In general, candidate human PF4ARs will exhibit greater than about 30% amino acid sequence homology to the Figs. 2, 4 or 5 receptors and bear a similar transmembrane loop structure.

Assays are then conducted to confirm that the hybridizing full length genes are the desired PF4AR. The candidate is simply inserted into an expression vector and transformed into a host cell that ordinarily does not bind to the candidate PF4A ligand. Transformants that acquire the ability to bind the ligand thus bear the desired receptor gene. In Example 2, we show that two additional homologous polypeptide sequences representing PR4ARs are identified using IL-8R DNA encoding residues 23-314, although the particular probe is not believed to be critical.

An alternative means to isolate genes encoding additional PF4ARs is to use polymerase chain reaction (PCR) methodology (U.S. Patent 4,683,195; Erlich, ed., PCR Technology, 1989) to amplify the target DNA or RNA, e.g. as described in section 14 of Sambrook *et al.,* supra. This method requires the use of oligonucleotide primers that will be expected to hybridize to the PF4AR, and these readily are selected from the receptor cDNAs of Figs. 2, 4 or 5. Strategies for selection of oligonucleotide primers are described above.

cDNA libraries may be screened from various tissues, preferably mammalian PBL, monocyte, placental, fetal, brain, and carcinoma cell lines in order to obtain DNA encoding the receptors of Figs. 2, 4 or 5, or homologous receptors. More preferably, human or rabbit placental, fetal, brain, and carcinoma cell line cDNA libraries are screened with labelled oligonucleotide probes.

Another method for obtaining the gene of interest is to chemically synthesize it using one of the methods described in Engels *et al.* (Agnew. Chem. Int. Ed. Engl. **28**:716-734 [1989]). These methods include triester, phosphite, phosphoramidite and H-phosphonate methods, typically proceeding by oligonucleotide synthesis on solid supports. These methods may be used if the entire amino acid or nucleic acid sequence of the gene is known, or the sequence of the nucleic acid complementary to the coding strand is available. If the desired amino acid sequence is known, one may infer potential nucleic acid sequences using known and preferred coding residues for each amino acid residue.

### B. Amino Acid Sequence Variants of the PF4AR

Amino acid sequence variants of the PF4AR are prepared by introducing appropriate nucleotide changes into the PF4AR DNA, or by *in vitro* synthesis of the desired PF4AR polypeptide. Such variants include, for example, deletions from, or insertions or substitutions of, residues within the amino acid sequence shown for the receptors in Figs. 2, 4 or 5. Any combination of deletion, insertion, and substitution can be made to arrive at the final construct, provided that the final construct possesses the desired characteristics.

The amino acid changes also may alter post-translational processing of the PF4AR, such as changing the number or position of glycosylation sites or by altering its membrane anchoring characteristics. Excluded from the scope of this invention are PF4AR variants or polypeptide sequences that are not statutorily novel and unobvious over the prior art.

In designing amino acid sequence variants of PF4ARs, the location of the mutation site and the nature of the mutation will depend on the PF4AR characteristic(s) to be modified. The sites for mutation can be modified individually or in series, e.g., by (1) substituting first with conservative amino acid choices and then with more radical selections depending upon the results achieved, (2) deleting the target residue, or (3) inserting residues of the same or a different class adjacent to the located site, or combinations of options 1-3.

A useful method for identification of certain residues or regions of the PF4AR polypeptide that are preferred locations for mutagenesis is called "alanine scanning mutagenesis" as described by Cunningham and Wells (Science **244**:1081-1085 [1989]). Here, a residue or group of target residues are identified (e.g., charged residues such as arg, asp, his, lys, and glu) and replaced by a neutral or negatively charged amino acid (most preferably alanine or poly-alanine) to affect the interaction of the amino acids with the surrounding aqueous environment in or outside the cell. Those domains demonstrating functional sensitivity to the substitutions then are refined by introducing further or other variants at or for the sites of substitution. Thus, while the site for introducing an amino acid sequence variation is predetermined, the nature of the mutation *per se* need not be predetermined. For example, to optimize the performance of a mutation at a given site, ala scanning or random mutagenests may be conducted at the target codon or region and the expressed PF4AR variants are screened for the optimal combination of desired activity.

In general, the regions of the PF4AR molecule preferred for alterations are non-hydrophic regions or regions that are not highly conserved. Such regions are those in which sequences of 5 or more residues are not substantially conserved in the homologous positions in the rabbit fMLP receptor, the human fMLP receptor, the human C5a receptor and the receptors of Figs. 2, 4 and 5.

PF4AR variants will exhibit at least a biological activity of the parental sequence, for example ligand binding activity or antigenic activity. Antigenically active PF4AR is a polypeptide that binds with an affinity of at least about 10⁻⁹ 1/mole to an antibody raised against a naturally occurring PF4AR sequence. Ordinarily the polypeptide binds with an affinity of at least about 10⁻⁸ 1/mole. Most preferably, the antigenically active PF4AR is a polypeptide that binds to an antibody raised against the receptor in its native conformation, "native conformation" generally meaning the receptor as found in nature which has not been denatured by chaotropic agents, heat or other treatment that substantially modifies the three dimensional structure of the receptor (this can be determined, for example, by migration on nonreducing, non-denaturing sizing gels). Antibody used in determination of antigenic activity is rabbit polyclonal antibody raised by formulating the native non-rabbit receptor in Freund's complete adjuvant, subcutaneously injecting the formulation, and boosting the immune response by intraperitoneal injection of the formulation until the titer of anti-receptor antibody plateaus.

One group of variants are deletion mutants, or fragments of the sequences set forth in Figs. 2, 4, 5 or other PF4AR. In general, the fragments are those which constitute the extracellular regions of the receptors (these receptors are unlike most in that they are believed to contain a plurality of hydrophobic, trans-memberan domains separated by hydrophilic sequences believed to loop into the ectoplasm). Particularly of interest are the N-terminal extracellular region containing acidic amino acid residues. However, any sequence which is capable of raising an antibody that will cross-react with the intact receptor, or which will bind to a member of the PF4 superfamily, is useful. These fragments typically will contain a consecutive sequence of at least about 5 (and ordinarily at least about 10) residues.

Amino acid sequence deletions generally range from about 1 to 30 residues, more preferably about 1 to 10 residues, and typically are contiguous. Deletions may be introduced into regions of low homology among the receptors of Figs. 2, 4 and 5 to modify the activity of the receptors. Such deletions will be more likely to modify the biological activity of the receptors more significantly than deletions made elsewhere. The number of consecutive deletions will be selected so as to preserve the tertiary structure of the PF4AR in the affected domain, e.g.. beta-pleated sheet or alpha helix.

Amino acid sequence insertions include amino- and/or carboxyl-terminal fusions ranging in length from one residue to polypeptides containing a hundred or more residues, as well as intrasequence insertions of single or multiple amino acid residues. Intrasequence insertions (i.e., insertions within the PF4AR sequence) may range generally from about 1 to 10 residues, more preferably 1 to 5, most preferably 1 to 3.

Insertional variants of the PF4AR or its extracellular segments include the fusion to the N- or C-terminus of the PF4AR of immunogenic polypeptides, e.g., bacterial polypeptides such as beta-lactamase or an enzyme encoded by the *E. coli trp* locus, or yeast protein, and C-terminal fusions with proteins having a long half-life such as immunoglobulin constant regions (or other immunoglobulin regions), albumin, or ferritin, as described in WO 89/02922 published 6 April 1989.

Another group of variants are amino acid substitution variants. These variants have at least one amino acid residue in the PF4AR molecule removed and a different residue inserted in its place. The sites of greatest interest for substitutional mutagenesis include sites identified as the active site(s) of the PF4AR, and sites where the amino acids found in the PF4AR from various species are substantially different in terms of side-chain bulk, charge, and/or hydrophobicity.

Other sites of interest are those in which particular residues of the PF4ARs of Figs. 2. 4 and 5 are identical. These positions may be important for the biological activity of the PF4AR. These sites, especially those falling within a sequence of at least three other identically conserved sites, are substituted in a relatively conservative manner. Such conservative substitutions are shown in Table 1 under the heading of preferred substitutions. If such substitutions result in a change in biological activity, then more substantial changes, denominated exemplary substitutions in Table 1, or as further described below in reference to amino acid classes, are introduced and the products screened.

**Table 1**

| Original Residue | Exemplary Substitutions | Preferred Substitutions |
|---|---|---|
| Ala (A) | val; feu; ile | val |
| Arg (R) | lys; gln; asn | lys |
| Asn (N) | gln; his; lys; arg | gln |
| Asp (D) | glu | glu |
| Cys (C) | ser | ser |
| Gln (Q) | asn | asn |
| Glu (E) | asp | asp |
| Gly (G) | pro | pro |
| His (H) | asn; gln; lys; arg | arg |
| Ile (I) | leu; val; met; ala; phe; norleucine | leu |
| Leu (L) | norleucine; ile; val; met; ala; phe | ile |
| Lys (K) | arg; gln; asn | arg |
| Met (M) | leu; phe; ile | leu |
| Phe (F) | leu; val; ile; ala | leu |
| Pro (P) | gly | gly |
| Ser (S) | thr | thr |
| Thr (T) | ser | ser |
| Trp (W) | tyr | tyr |
| Tyr (Y) | trp; phe; thr; ser | phe |
| Val (V) | ile; leu; met; phe; ala; norleucine | leu |

Substantial modifications in function or immunological identity of the PF4AR are accomplished by selecting substitutions that differ significantly in their effect on maintaining (a) the structure of the polypeptide backbone in the area of the substitution, for example, as a sheet or helical conformation, (b) the charge or hydrophobicity of the molecule at the target site, or (c) the bulk of the side chain. Naturally occurring residues are divided into groups based on common side chain properties:
(1) hydrophobic: norleucine, met, ala, val, leu, ile;
(2) neutral hydrophilic: cys, ser, thr;
(3) acidic: asp, glu;
(4) basic: asn, gln, his, lys, arg;
(5) residues that influence chain orientation: gly, pro; and
(6) aromatic: trp tyr, phe.

Non-conservative substitutions will entail exchanging a member of one of these classes for another. Such substituted residues may be introduced into regions of the PF4AR that are homologous with other PF4ARs, or, more preferably, into the non-homologous regions of the molecule.

Any cysteine residues not involved in maintaining the proper conformation of the PF4AR may be substituted, generally with serine, to improve the oxidative stability of the molecule and prevent aberrant crosslinking.

DNA encoding amino acid sequence variants of the PF4AR is prepared by a variety of methods known in the an. These methods include, but are not limited to, isolation from a natural source (in the case of naturally occurring amino acid sequence variants) or preparation by oligonucleotide-mediated (or site-directed) mutagenesis, PCR mutagenesis, and cassette mutagenesis of an earlier prepared variant or a non-variant version of the PF4AR. These techniques may utilize PF4AR nucleic acid (DNA or RNA), or nucleic acid complementary to the PF4AR nucleic acid.

Oligonucleotide-mediated mutagenesis is a preferred method for preparing substitution, deletion, and insertion variants of PF4AR DNA. This technique is well known in the art, for example as described by Adelman *et al.,* DNA **2**:183 (1983). Briefly, the PF4AR DNA is altered by hybridizing an oligonucleotide encoding the desired mutation to a DNA template, where the template is the single-stranded form of a plasmid or bacteriophage containing the unaltered or native DNA sequence of the PF4AR. After hybridization, a DNA polymerase is used to synthesize an entire second complementary strand of the template that will thus incorporate the oligonucleotide primer, and will code for the selected alteration in the PF4AR DNA.

Generally, oligonucleoxides of at least 25 nucleotides in length are used. An optimal oligonucleotide will have 12 to 15 nucleotides that are completely complementary to the template on either side of the nucleotide(s) coding for the mutation. This ensures that the oligonucleotide will hybridize properly to the single-stranded DNA template molecule. The oligonucleotides are readily synthesized using techniques known in the art such as that described by Crea *et al.* (Proc. Natl. Acad. Sci. USA **75**:5765 [1978]).

Single-stranded DNA template may also be generated by denaturing double-stranded plasmid (or other) DNA using standard techniques.

For alteration of the native DNA sequence (to generate amino acid sequence variants, for example), the oligonucleotide is hybridized to the single-stranded template under suitable hybridization conditions. A DNA polymerizing enzyme, usually the Klenow fragment of DNA polymerase I, is then added to synthesize the complementary strand of the template using the oligonucleotide as a primer for synthesis. A heteroduplex molecule is thus formed such that one strand of DNA encodes the mutated form of the PF4AR, and the other strand (the original template) encodes the native, unaltered sequence of the PF4AR. This heteroduplex molecule is then transformed into a suitable host cell, usually a prokaryote such as *E*. *coli* JM101. The cells are plated onto agarose plates, and screened using the oligonucleotide primer radiolabeled with 32-phosphate to identify the bacterial colonies that contain the mutated DNA. The mutated region is then removed and placed in an appropriate vector for protein production, generally an expression vector of the type typically employed for transformation of an appropriate host.

The method described immediately above may be modified such that a homoduplex molecule is created wherein both strands of the plasmid contain the mutation(s). The modifications are as follows: The single-stranded oligonucleotide is annealed to the single-stranded template as described above. A mixture of three deoxyribonucleotides, deoxyriboadenosine (dATP), deoxyriboguanosine (dGTP), and deoxyribothymidine (dTTP), is combined with a modified thio-deoxyribocytosine called dCTP-(aS) (which can be obtained from Amersham Corporation). This mixture is added to the template-oligonucleotide complex. Upon addition of DNA polymerase to this mixture, a strand of DNA identical to the template except for the mutated bases is generated. In addition, this new strand of DNA will contain dCTP-(aS) instead of dCTP, which serves to protect it from restriction endonuclease digestion. After the template strand of the double-stranded heteroduplex is nicked with an appropriate restriction enzyme, the template strand can be digested with ExoIII nuclease or another appropriate nuclease past the region that contains the site(s) to be mutagenized. The reaction is then stopped to leave a molecule that is only partially single-stranded. A complete double-stranded DNA homoduplex is then formed using DNA polymerase in the presence of all four deoxyribonucleotide triphosphates, ATP, and DNA ligase. This homoduplex molecule can then be transformed into a suitable host cell such as *E. coli* JM101, as described above.

DNA encoding PF4AR mutants at more than one site may be generated in one of several ways. If the amino acids are located close together in the polypeptide chain, they may be mutated simultaneously using one oligonucleotide that codes for all of the desired amino acid substitutions. If, however, the amino acids are located some distance from each other (separated by more than about ten amino acids), it is more difficult to generate a single oligonucleotide that encodes all of the desired changes. Instead, one of two alternative methods may be employed.

In the first method, a separate oligonucleotide is generated for each amino add to be substituted. The oligonucleotides are then annealed to the single-stranded template DNA simultaneously, and the second strand of DNA that is synthesized from the template will encode all of the desired amino acid substitutions.

The alternative method involves two or more rounds of mutagenesis to produce the desired mutant. The first round is as described for the single mutants: wild-type DNA is used for the template, an oligonucleotide encoding the first desired amino acid substitution(s) is annealed to this template, and the heteroduplex DNA molecule is then generated. The second round of mutagenesis utilizes the mutated DNA produced in the first round of mutagenesis as the template. Thus, this template already contains one or more mutations. The oligonucleotide encoding the additional desired amino acid substitution(s) is then annealed to this template, and the resulting strand of DNA now encodes mutations from both the first and second rounds of mutagenesis. This resultant DNA can be used as a template in a third round of mutagenesis, and so on.

PCR mutagenesis is also suitable for making amino acid variants of the PF4AR. While the following discussion refers to DNA, it is understood that the technique also finds application with RNA. The PCR technique generally refers to the following procedure (see Erlich, *supra,* the chapter by R. Higuchi, p. 61-70): When small amounts of template DNA are used as starting material in a PCR, primers that differ slightly in sequence from the corresponding region in a template DNA can be used to generate relatively large quantities of a specific DNA fragment that differs from the template sequence only at the positions where the primers differ from the template. For introduction of a mutation into a plasmid DNA, one of the primers is designed to overlap the position of the mutation and to contain the mutation; the sequence of the other primer must be identical to a stretch of sequence of the opposite strand of the plasmid, but this sequence can be located anywhere along the plasmid DNA. It is preferred, however, that the sequence of the second primer is located within 200 nucleotides from that of the first, such that in the end the entire amplified region of DNA bounded by the primers can be easily sequenced. PCR amplification using a primer pair like the one just described results in a population of DNA fragments that differ at the position of the mutation specified by the primer, and possibly at other positions, as template copying is somewhat error-prone.

If the ratio of template to product material is extremely low, the vast majority of product DNA fragments incorporate the desired mutation(s). This product material is used to replace the corresponding region in the plasmid that served as PCR template using standard DNA technology. Mutations at separate positions can be introduced simultaneously by either using a mutant second primer, or performing a second PCR with different mutant primers and ligating the two resulting PCR fragments simultaneously to the vector fragment in a three (or more)-part ligation.

Another method for preparing variants, cassette mutagenesis, is based on the technique described by Wells *et al.* (Gene, **34**:315 [1985]).

### C. Insertion of DNA into a Cloning Vehicle

The cDNA or genomic DNA encoding native or variant PF4AR is inserted into a replicable vector for further cloning (amplification of the DNA) or for expression. Many vectors are available, and selection of the appropriate vector will depend on (1) whether it is to be used for DNA amplification or for DNA expression, (2) the size of the DNA to be inserted into the vector, and (3) the host cell to be transformed with the vector. Each vector contains various components depending on its function (amplification of DNA or expression of DNA) and the host cell for which it is compatible. The vector components generally include, but are not limited to, one or more of the following: a signal sequence, an origin of replication, one or more marker genes, an enhancer element, a promoter, and a transcription termination sequence.

### (i) Signal Sequence Component

In general, a signal sequence may be a component of the vector, or it may be a part of the PF4AR DNA that is inserted into the vector. The native pro PF4AR DNA is directed to the cell surface in our recombinant cells but it does not contain a conventional signal and no N-terminal polypeptide is cleaved during post-translational processing of the polypeptide during membrane insertion of the PF4AR.

### (ii) Origin of Replication Component

Both expression and cloning vectors contain a nucleic acid sequence that enables the vector to replicate in one or more selected host cells. Generally in cloning vectors this sequence is one that enables the vector to replicate independently of the host chromosomal DNA, and includes origins of replication or autonomously replicating sequences. Such sequences are well known for a variety of bacteria, yeast, and viruses. The origin of replication from the plasmid pBR322 is suitable for most Gram-negative bacteria, the 2µ plasmid origin is suitable for yeast, and various viral origins (SV40, polyoma, adenovirus, VSV or BPV) are useful for cloning vectors in mammalian cells. Generally, the origin of replication component is not needed for mammalian expression vectors (the SV40 origin may typically be used only because it contains the early promoter).

Most expression vectors are "shuttle" vectors, i.e. they are capable of replication in at least one class of organisms but can be transfected into another organism for expression. For example, a vector is cloned in *E*. *coli* and then the same vector is transfected into yeast or mammalian cells for expression even though it is not capable of replicating independently of the host cell chromosome.

DNA may also be amplified by insertion into the host genome. This is readily accomplished using *Bacillus* species as hosts, for example, by including in the vector a DNA sequence that is complementary to a sequence found in *Bacillus* genomic DNA. Transfection of *Bacillus* with this vector results in homologous recombination with the genome and insertion of the PF4AR DNA. However, the recovery of genomic DNA encoding the PF4AR is more complex than that of an exogenously replicated vector because restriction enzyme digestion is required to excise the PF4AR DNA.

### (iii) Selection Gene Component

Expression and cloning vectors should contain a selection gene, also termed a selectable marker. This gene encodes a protein necessary for the survival or growth of transformed host cells grown in a selective culture medium. Host cells not transformed with the vector containing the selection gene will not survive in the culture medium. Typical selection genes encode proteins that (a) confer resistance to antibiotics or other toxins, e.g. ampicillin, neomycin, methotrexate, or tetracycline, (b) complement auxotrophic deficiencies, or (c) supply critical nutrients not available from complex media, e.g. the gene encoding D-alanine racemase for *Bacilli.*

One example of a selection scheme utilizes a drug to arrest growth of a host cell. Those cells that are successfully transformed with a heterologous gene express a protein conferring drug resistance and thus survive the selection regimen. Examples of such dominant selection use the drugs neomycin (Southern *et al.,* J. Molec. Appl. Genet. **1**:327 (19821), mycophenolic acid (Mulligan *et al.,* Science **209**:1422 [1980]) or hygromycin (Sugden *et al.,* Mol. Cell. Biol. **5**:410-413 [1985]). The three examples given above employ bacterial genes under eukaryotic control to convey resistance to the appropriate drug G418 or neomycin (geneticin), xgpt (mycophenolic acid), or hygromycin, respectively.

Another example of suitable selectable markers for mammalian cells are those that enable the identification of cells competent to take up the PF4AR nucleic acid, such as dihydrofolate reductase (DHFR) or thymidine kinase. The mammalian cell transformants are placed under selection pressure which only the transformants are uniquely adapted to survive by virtue of having taken up the marker. Selection pressure is imposed by culturing the transformants under conditions in which the concentration of selection agent in the medium is successively changed, thereby leading to amplification of both the selection gene and the DNA that encodes the PF4AR. Amplification is the process by which genes in greater demand for the production of a protein critical for growth are reiterated in tandem within the chromosomes of successive generations of recombinant cells. Increased quantities of the PF4AR are synthesized from the amplified DNA.

For example, cells transformed with the DHFR selection gene are first identified by culturing all of the transformants in a culture medium that contains methotrexate (Mtx), a competitive antagonist of DHFR. An appropriate host cell when wild-type DHFR is employed is the Chinese hamster ovary (CHO) cell line deficient in DHFR activity, prepared and propagated as described by Urlaub and Chasm, Proc. Natl. Acad. Sci. USA. **77**:4216 [1980]. The transformed cells are then exposed to increased levels of methotrexate. This leads to the synthesis of multiple copies of the DHFR gene, and, concomitantly, multiple copies of other DNA comprising the expression vectors, such as the DNA encoding the PF4AR. This amplification technique can be used with any otherwise suitable host, e.g., ATCC No. CCL61 CHO-K1, notwithstanding the presence of endogenous DHFR if, for example, a mutant DHFR gene that is highly resistant to Mtx is employed (EP 117,060). Alternatively, host cells (particularly wild-type hosts that contain endogenous DHFR) transformed or co-transformed with DNA sequences encoding the PF4AR, wild-type DHFR protein, and another selectable marker such as aminoglycoside 3' phosphotransferase (APH) can be selected by cell growth in medium containing a selection agent for the selectable marker such as an aminoglycosidic antibiotic, e.g., kanamycin, neomycin, or G418. See U.S. Pat. No. 4,965,199.

A suitable selection gene for use in yeast is the *trp*1 gene present in the yeast plasmid YRp7 (Stinchcomb *et al.,* Nature **282**:39 [1979]; Kingsman *et al.,* Gene **7**:141 [1979]; or Tschemper *et al.*, Gene **10**:157 (1980]). The *trp*1 gene provides a selection marker for a mutant strain of yeast lacking the ability to grow in tryptophan, for example, ATCC No. 44076 or PEP4-1 (Jones, Genetics **85**:12 [1977]). The presence of the trp1 lesion in the yeast host cell genome then provides an effective environment for detecting transformation by growth in the absence of tryptophan. Similarly, *Leu*2-deficient yeast strains (ATCC 20,622 or 38,626) are complemented by known plasmids bearing the *Leu*2 gene.

### (iv) Promoter Component

Expression vectors usually contain a promoter that is recognized by the host organism and is operably linked to the PF4AR nucleic acid. Promoters are untranslated sequences located upstream (5') to the start codon of a structural gene (generally within about 100 to 1000 bp) that control the transcription and translation of a particular nucleic acid sequence, such as the PF4AR, to which they are operably linked. Such promoters typically fall into two classes, inducible and constitutive. Inducible promoters are promoters that initiate increased levels of transcription from DNA under their control in response to some change in culture conditions, e.g. the presence or absence of a nutrient or a change in temperature. At this time a large number of promoters recognized by a variety of potential host cells are well known. These promoters are operably linked to DNA encoding the PF4AR by removing the promoter from the source DNA by restriction enzyme digestion and inserting the isolated promoter sequence into the vector. Both the native PF4AR promoter sequence and many heterologous promoters may be used to direct amplification and/or expression of the PF4AR DNA. However, heterologous promoters are preferred, as they generally permit greater transcription and higher yields of expressed PF4AR as compared to the native PF4AR promoter.

Promoters suitable for use with prokaryotic hosts include the β-lactamase and lactose promoter systems (Chang *et al.,* Nature. **275**:615 [1978]; and Goeddel *et al.,* Nature. **281**:544 [1979]), alkaline phosphatase, a tryptophan (trp) promoter system (Goeddel, Nucleic Acids Res.. **8**:4057 [1980] and EP 36,776) and hybrid promoters such as the tac promoter (deBoer *et al.,* Proc. Natl. Acad. Sci. USA. **80**:21-25 [1983]). However, other known bacterial promoters are suitable. Their nucleotide sequences have been published, thereby enabling a skilled worker operably to ligate them to DNA encoding the PF4AR (Siebenlist *et al.,* Cell. **20**:269 (1980]) using linkers or adaptors to supply any required restriction sites. Promoters for use in bacterial systems also generally will contain a Shine-Dalgarno (S.D.) sequence operably linked to the DNA encoding the PF4AR.

Suitable promoting sequences for use with yeast hosts include the promoters for 3-phosphoglycerate kinase (Hitzeman *et al,* J. Biol. Chem., **255**:2073 [1980]) or other glycolytic enzymes (Hess *et al.,* J. Adv. Enzyme Reg., **7**:149 [1968]; and Holland, Biochemistry. 17:4900[1978]), such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase.

Other yeast promoters, which are inducible promoters having the additional advantage of transcription controlled by growth conditions, are the promoter regions for alcohol dehydrogenase 2, isocytochrome C, acid phosphatase, degradative enzymes associated with nitrogen metabolism, metallothionein, glyceraldehyde-3-phosphate dehydrogenase, and enzymes responsible for maltose and galactose utilization. Suitable vectors and promoters for use in yeast expression are further described in Hitzeman *et al.,* EP 73,657A. Yeast enhancers also are advantageously used with yeast promoters.

Promoter sequences are known for eukaryotes. Virtually all eukaryotic genes have an AT-rich region located approximately 25 to 30 bases upstream from the site where transcription is initiated. Another sequence found 70 to 80 bases upstream from the start of transcription of many genes is a CXCAAT region where X may be any nucleotide. At the 3' end of most eukaryotic genes is an AATAAA sequence that may be the signal for addition of the poly A tail to the 3' end of the coding sequence. All of these sequences are suitably inserted into mammalian expression vectors.

PF4AR transcription from vectors in mammalian host cells is controlled by promoters obtained from the genomes of viruses such as polyoma virus, fowlpox virus (UK 2,211,504 published 5 July 1989), adenovirus (such as Adenovirus 2), bovine papilloma virus, avian sarcoma virus, cytomegalovirus, a retrovirus, hepatitis-B virus and most preferably Simian Virus 40 (SV40), from heterologous mammalian promoters, e.g. the actin promoter or an immunoglobulin promoter, from heat-shock promoters, and from the promoter normally associated with the PF4AR sequence, provided such promoters are compatible with the host cell systems.

The early and late promoters of the SV40 virus are conveniently obtained as an SV40 restriction fragment that also contains the SV40 viral origin of replication. Fiers *et al.,* Nature. **273**:113 (1978); Mulligan and Berg, Science, **209**:1422-1427 (1980); Pavlakis et *al*., Proc. Natl. Acad. Sci. USA, **78**:7398-7402 (1981). The immediate early promoter of the human cytomegalovirus is conveniently obtained as a HindIII E restriction fragment Greenaway *et al.,* Gene, **18**:355-360 (1982). A system for expressing DNA in mammalian hosts using the bovine papilloma virus as a vector is disclosed in U.S. 4,419,446. A modification of this system is described in U.S. 4,601,978. See also Gray *et al.,* Nature. **295**:503-508 (1982) on expressing cDNA encoding immune interferon in monkey cells; Reyes *et al.,* Nature. **297**:598-601 (1982) on expression of human β-interferon cDNA in mouse cells under the control of a thymidine kinase promoter from herpes simplex virus, Canaani and Berg, Proc. Natl. Acad. Sci. USA. **79**:5166-5170 (1982) on expression of the human interferon β1 gene in cultured mouse and rabbit cells, and Gorman *et al.,* Proc. Natl. Acad. Sci. USA. **79**:6777-6781 (1982) on expression of bacterial CAT sequences in CV-1 monkey kidney cells, chicken embryo fibroblasts, Chinese hamster ovary cells, HeLa cells, and mouse NIH-3T3 cells using the Rous sarcoma virus long terminal repeat as a promoter.

### (v) Enhancer Element Component

Transcription of a DNA encoding the PF4AR of this invention by higher eukaryotes is often increased by inserting an enhancer sequence into the vector. Enhancers are cis-acting elements of DNA, usually about from 10-300 bp, that act on a promoter to increase its transcription. Enhancers are relatively orientation and position independent having been found 5' (Laimins *et al.,* Proc. Natl. Acad. Sci. USA, **78**:993 [1981]) and 3' (Lusky *et al.,* Mol. Cell Big., **3**:1108 [1983]) to the transcription unit, within an intron (Banerji *et al.,* Cell, **33**:729 [1983]) as well as within the coding sequence itself (Osborne *et al.,* Mol. Cell Bio., **4**:1293 [1984]). Many enhancer sequences are now known from mammalian genes (globin, elastase, albumin, α-fetoprotein and insulin). Typically, however, one will use an enhancer from a eukaryotic cell virus. Examples include the SV40 enhancer on the late side of the replication origin (bp 100-270), the cytomegalovirus early promoter enhancer, the polyoma enhancer on the late side of the replication origin, and adenovirus enhancers. See also Yaniv, Nature, **297**:17-18 (1982) on enhancing elements for activation of eukaryotic promoters. The enhancer may be spliced into the vector at a position 5' or 3' to the PF4AR DNA, but is preferably located at a site 5' from the promoter.

### (vi) Transcription Termination Component

Expression vectors used in eukaryotic host cells (yeast, fungi, insect, plant, animal, human, or nucleated cells from other multicellular organisms) will also contain sequences necessary for the termination of transcription and for stabilizing the mRNA. Such sequences are commonly available from the 5' and, occasionally 3' untranslated regions of eukaryotic or viral DNAs or cDNAs. These regions contain nucleotide segments transcribed as polyadenylated fragments in the untranslated portion of the mRNA encoding the PF4AR. The 3' untranslated regions also include transcription termination sites.

Suitable vectors containing one or more of the above listed components and the desired coding and control sequences are constructed by standard ligation techniques. Isolated plasmids or DNA fragments are cleaved, tailored, and religated in the form desired to generate the plasmids required.

For analysis to confirm correct sequences in plasmids constructed, the ligation mixtures are used to transform *E. coli* K12 strain 294 (ATCC 31,446) and successful transformants selected by ampicillin or tetracycline resistance where appropriate. Plasmids from the transformants are prepared, analyzed by restriction endonuclease digestion, and/or sequenced by the method of Messing *et al.,* Nucleic Acids Res., **9**:309 (1981) or by the method of Maxam *et al.,* Methods in Enzymology, **65**:499 (1980).

Particularly useful in the practice of this invention are expression vectors that provide for the transient expression in mammalian cells of DNA encoding the PF4AR. In general, transient expression involves the use of an expression vector that is able to replicate efficiently in a host cell, such that the host cell accumulates many copies of the expression vector and, in turn, synthesizes high levels of a desired polypeptide encoded by the expression vector.
Transient expression systems, comprising a suitable expression vector and a host cell, allow for the convenient positive identification of polypeptides encoded by cloned DNAs, as well as for the rapid screening of such polypeptides for desired biological or physiological properties. Thus, transient expression systems are particularly useful in the invention for purposes of identifying analogs and variants of the PF4AR that have PF4AR-like activity.

Other methods, vectors, and host cells suitable for adaptation to the synthesis of the PF4AR in recombinant vertebrate cell culture are described in Gething *et al*., Nature, **293**:620-625 [1981]; Mantei *et at.,* Nature, 281:40-46 [1979]; Levinson *et al.;* EP 117,060; and EP 117,058. A particularly useful plasmid for mammalian cell culture expression of the PF4AR is pRK5 (EP pub. no. 307,247).

### D. Selection and Transformation of Host Cells

Suitable host cells for cloning or expressing the vectors herein are the prokaryote, yeast, or higher eukaryote cells described above. Suitable prokaryotes include eubacteria, such as Gram-negative or Gram-positive organisms, for example, *E. coli, Bacilli* such as *B. subtilis, Pseudomonas* species such as *P. aeruginosa, Salmonella typhimurium,* or *Serratia marcescens.* One preferred *E. coli* cloning host is *E. coli* 294 (ATCC 31,446), although other strains such as *E. coli* B. *E. coli _{X}*1776 (ATCC 31,537), and *E. coli* W3110 (ATCC 27,325) are suitable. These examples are illustrative rather than limiting. Preferably the host cell should secrete minimal amounts of proteolytic enzymes. Alternatively *in vitro* methods of cloning, e.g. PCR or other nucleic acid polymerase reactions, are suitable.

In addition to prokaryotes, eukaryotic microbes such as filamentous fungi or yeast are suitable hosts for vectors containing PF4AR DNA. *Saccharomyces cerevisiae,* or common baker's yeast, is the most commonly used among lower eukaryotic host microorganisms. However, a number of other genera, species, and strains are commonly available and useful herein, such as *S*. *pombe* [Beach and Nurse, Nature, **290**:140 (1981)], *Kluyveromyces lactis* [Louvencourt *et al.,* J. Bacteriol., **737** (1983)], *yarrowia* [EP 402,226], *Pichia pastoris* [EP 183,070], *Trichoderma reesia* [EP 244,234], *Neurospora crassa* [Case *et al.,* Proc. Natl. Acad. Sci. USA, **76**:5259-5263 (1979)], and *Aspergillus* hosts such as *A*. *nidulans* [Ballance *et al.,* Biochem: Biophys. Res. Commun.. **112**:284-289 (1983); Tilburn *et al.,* Gene, **26**:205-221 (1983); Yelton *et al.,* Proc. Natl. Acad. Sci. USA, **81**:1470-1474 (1984)] and A. *niger* [Kelly and Hynes, EMBO J., **4**:475-479 (1985)].

Suitable host cells for the expression of glycosylated PF4AR polypeptide are derived from multicellular organisms. Such host cells are capable of complex processing and glycosylation activities. In principle, any higher eukaryotic cell culture is workable, whether from vertebrate or invertebrate culture. Examples of invertebrate cells include plant and insect cells. Numerous baculoviral strains and variants and corresponding permissive insect host cells from hosts such as *Spodoptera frugiperda* (caterpillar), *Aedes aegypti* (mosquito), *Aedes albopictus* (mosquito), *Drosophila melanogaster* (fruitfly), and *Bombyx mori* host cells have been identified. See, e.g., Luckow *et al.,* Bio/Technology, **6**:47-55 (1988); Miller *et al.,* in Genetic Engineering, Setlow, J.K. *et al.,* **8**:277-279 (Plenum Publishing, 1986), and Maeda *et al.,* Nature. **315**:592-594 (1985). A variety of such viral strains are publicly available, e.g., the L-1 variant of *Autographa californica* NPV and the Bm-5 strain of *Bombyx mori* NPV, and such viruses may be used as the virus herein according to the present invention, particularly for transfection of *Spodoptera frugiparda* cells. Plant cell cultures of cotton, corn, potato, soybean, petunia, tomato, and tobacco can be utilized as hosts. Typically, plant cells are transfected by incubation with certain strains of the bacterium *Agrobactanium tumefaciens,* which has been previously manipulated to contain the PF4AR DNA. During incubation of the plant cell culture with A. *tumafacians,* the DNA encoding PF4AR is transferred to the plant cell host such that it is transfected, and will, under appropriate conditions, express the PF4AR DNA. In addition, regulatory and signal sequences compatible with plant cells are available, such as the nopaline synthase promoter and polyadenylation signal sequences. Depicker *et al.,* J. Mol. Anol. Gen., 1:561 (1982). In addition, DNA segments isolated from the upstream region of the T-DNA *780* gene are capable of activating or increasing transcription levels of plant-expressible genes in recombinant DNA-containing plant tissue. See EP 321,196 published 21 June 1989.

However, interest has been greatest in vertebrate cells, and propagation of vertebrate cells in culture (tissue culture) has become a routine procedure in recent years [Tissue Culture, Academic Press, Kruse and Patterson, editors (1973)]. Examples of useful mammalian host cell lines are monkey kidney CV1 line transformed by SV40 (COS-7, ATCC CRL 1651); human embryonic kidney line (293 or 293 cells subcloned for growth in suspension culture, Graham *et al.,* J. Gen Virol., **36**:59 [1977]); baby hamster kidney cells (BHK, ATCC CCL 10); Chinese hamster ovary cells/-DHFR (CHO, Urlaub and Chasin, Proc. Natl. Acad. Sci. USA, **77**:4216 [1980]); mouse sertoli cells (TM4, Mather, Biol. Reprod., **23**:243-251 [1980]); monkey kidney cells (CV1 ATCC CCL 70); African green monkey kidney cells (VERO-76, ATCC CRL-1587); human cervical carcinoma cells (HELA, ATCC CCL 2); canine kidney cells (MDCK, ATCC CCL 34); buffalo rat liver cells (BRL 3A, ATCC CRL 1442); human lung cells (W138, ATCC CCL 75); human liver cells (Hep G2, HB 8065); mouse mammary tumor (MMT 060562, ATCC CCL51); TRI cells (Mather *et al.,* Annals N.Y Acad. Sci., **383**:44-68 [1982]); MAC 5 cells; FS4 cells; and a human hepatoma cell line (Hep G2). Preferred host cells are human embryonic kidney 293 and Chinese hamster ovary cells.

Host cells are transfected and preferably transformed with the above-described expression or cloning vectors of this invention and cultured in conventional nutrient media modified as appropriate for inducing promoters, selecting transformants, or amplifying the genes encoding the desired sequences.

Transfection refers to the taking up of an expression vector by a host cell whether or not any coding sequences are in fact expressed. Numerous methods of transfection are known to the ordinarily skilled artisan, for example, CaPO₄ and electroporation. Successful transfection is generally recognized when any indication of the operation of this vector occurs within the host cell.

Transformation means introducing DNA into an organism so that the DNA is replicable, either as an extra-chromosomal element or by chromosomal integrant Depending on the host cell used, transformation is done using standard techniques appropriate to such cells. The calcium treatment employing calcium chloride, as described in section 1.82 of Sambrook *et al*., supra, is generally used for prokaryotes or other cells that contain substantial cell-wall barriers. Infection with *Agrobacterium tumefeciens* is used for transformation of certain plant cells, as described by Shaw *et al.,* Gene, **23**:315 (1983) and WO 89/05859 published 29 June 1989. For mammalian cells without such cell walls, the calcium phosphate precipitation method described in sections 16.30-16.37 of Sambrook *et al., supra,* is preferred. General aspects of mammalian cell host system transformations have been described by Axel in U.S. 4,399,216 issued 16 August 1983. Transformations into yeast are typically carried out according to the method of Van Solingen et al., J.
Bact., **130**:946 (1977) and Hsiao *et al.,* Proc. Natl. Acad. Sci. (USA), **76**:3829 (1979). However, other methods for introducing DNA into cells such as by nuclear injection, electroporation, or by protoplast fusion may also be used.

### E. Culturing the Host Cells

Prokaryotic cells used to produce the PF4AR polypeptide of this invention are cultured in suitable media as described generally in Sambrook *et al., supra.*

The mammalian host cells used to produce the PF4AR of this invention may be cultured in a variety of media. Commercially available media such as Ham's F10 (Sigma), Minimal Essential Medium ([MEM], Sigma), RPMI-1640 (Sigma), and Dulbecco's Modified Eagle's Medium ([DMEM], Sigma) are suitable for culturing the host cells. In addition, any of the media described in Ham and Wallace, Meth. Enz., **58**:44 (1979), Barnes and Sato, Anal. Biochem., **102**:255 (1980), U.S. 4,767,704; 4,657,866; 4,927,762; or 4,560,655; WO 90/03430; WO 87/00195; or U.S. Pat. Re. 30,985, may be used as culture media for the host cells. Any of these media may be supplemented as necessary with hormones and/or other growth factors (such as insulin, transferrin, or epidermal growth factor), salts (such as sodium chloride, calcium, magnesium, and phosphate), buffers (such as HEPES), nucleosides (such as adenosine and thymidine), antibiotics (such as Gentamycin^{™} drug), trace elements (defined as inorganic compounds usually present at final concentrations in the micromolar range), and glucose or an equivalent energy source. Any other necessary supplements may also be included at appropriate concentrations that would be known to those skilled in the art. The culture conditions, such as temperature, pH, and the like, are those previously used with the host cell selected for expression, and will be apparent to the ordinarily skilled artisan.

The host cells referred to in this disclosure encompass cells in *in vitro* culture as well as cells that are within a host animal.

It is further envisioned that the PF4AR of this invention may be produced by homologous recombination, or with recombinant production methods utilizing control elements introduced into cells already containing DNA encoding the PF4AR. For example, a powerful promoter/enhancer element, a suppressor, or an exogenous transcription modulatory element is inserted in the genome of the intended host cell in proximity and orientation sufficient to influence the transcription of DNA encoding the desired PF4AR. The control element does not encode the PF4AR of this invention, but the DNA is present in the host cell genome. One next screens for cells making the PF4AR of this invention, or increased or decreased levels of expression, as desired.

### F. Detecting Gene Amplification/Expression

Gene amplification and/or expression may be measured in a sample directly, for example, by conventional Southern blotting, northern blotting to quantitate the transcription of mRNA (Thomas, Proc. Natl. Acad. Sci. USA, **77**:5201-5205 [1980]), dot blotting (DNA analysis), or *in situ* hybridization, using an appropriately labeled probe, based on the sequences provided herein. Various labels may be employed, most commonly radioisotopes, particularly ³²P.
However, other techniques may also be employed, such as using biotin-modified nucleotides for introduction into a polynucleotide. The biotin then serves as the site for binding to avidin or antibodies, which may be labeled with a wide variety of labels, such as radionuclides, fluorescers, enzymes, or the like. Alternatively, antibodies may be employed that can recognize specific duplexes, including DNA duplexes, RNA duplexes, and DNA-RNA hybrid duplexes or DNA-protein duplexes. The antibodies in turn may be labeled and the assay may be carried out where the duplex is bound to a surface, so that upon the formation of duplex on the surface, the presence of antibody bound to the duplex can be detected.

Gene expression, alternatively, may be measured by immunological methods, such as immunohistochemical staining of tissue sections and assay of cell culture or body fluids, to quantitate directly the expression of gene product With immunohistochemical staining techniques, a cell sample is prepared, typically by dehydration and fixation, followed by reaction with labeled antibodies specific for the gene product coupled, where the labels are usually visually detectable, such as enzymatic labels, fluorescent labels, luminescent labels, and the like. A particularly sensitive staining technique suitable for use in the present invention is described by Hsu *et al*., Am. J. Clin. Path., **75**:734-738 (1980).

Antibodies useful for immunohistochemical staining and/or assay of sample fluids may be either monoclonal or polyclonal, and may be prepared in any mammal. Conveniently, the antibodies may be prepared against a native PF4AR polypeptide or against a synthetic peptide based on the DNA sequences provided herein as described further in Section 4 below.

### G. Purification of The PF4AR Polypeptide

The PF4AR is recovered from the culture cells by solubilizing cell membrane in detergent.

When a human PF4AR is expressed in a recombinant cell other than one of human origin, the PF4AR is completely free of proteins or polypeptides of human origin. However, it is necessary to purify the PF4AR from recombinant cell proteins or polypeptides to obtain preparations that are substantially homogeneous as to the PF4AR. As a first step, the cells are centrifuged to separate them from culture medium. The membrane and soluble protein fractions are then separated. The PF4AR may then be purified from the membrane fraction of the culture lysate by solubilization with detergents followed by suitable purification procedures: fractionation on immunoaffinity or ion-exchange columns; ethanol precipitation; reverse phase HPLC; chromatography on silica or on a cation exchange resin such as DEAE; chromatofocusing; SDS-PAGE; ammonium sulfate precipitation; gel filtration using, for example, Sephadex G-75; hydrophobic affinity resins and ligand affinity using the appropriate PF4A immobilized on a matrix.

PF4AR variants in which residues have been deleted, inserted or substituted are recovered in the same fashion as the native PF4AR, taking account of any substantial changes in properties occasioned by the variation. For example, preparation of a PF4AR fusion with another protein or polypeptide, e.g. a bacterial or viral antigen, facilitates purification; an immunoaffinity column containing antibody to the antigen can be used to adsorb the fusion. Immunoaffinity columns such as a rabbit polyclonal anti-PF4AR column can be employed to absorb the PF4AR variant by binding it to at least one remaining immune epitope. A protease inhibitor such as phenyl methyl sulfonyl fluoride (PMSF) also may be useful to inhibit proteolytic degradation during purification, and antibiotics may be included to prevent the growth of adventitious contaminants. One skilled in the art will appreciate that purification methods suitable for native PF4AR may require modification to account for changes in the character of the PF4AR or its variants upon expression in recombinant cell culture.

### H. Covalent Modifications of PF4AR Polypeptides

Covalent modifications of PF4AR polypeptides are included within the scope of this invention. Both native PF4ARs and amino acid sequence variants of the PF4AR may be covalently modified. Covalent modifications of the PF4AR, fragments thereof or antibodies thereto are introduced into the molecule by reacting targeted amino acid residues of the PF4AR, fragments thereof, or PF4AR antibody with an organic derivatizing agent that is capable of reacting with selected side chains or the N- or C-terminal residues. Most commonly, PF4AR and its antibodies are covalently bonded to detectable groups used in diagnosis, e.g. enzymes, radio isotopes, spin labels, antigens, fluorescent or chemiluminescent groups and the like.

Cysteinyl residues most commonly are reacted with α-haloacetates (and corresponding amines), such as chloroacetic acid or chloroacetamide, to give carboxymethyl or carboxyamidomethyl derivatives. Cysteinyl residues also are derivatized by reaction with bromotrifluoroacetone, α-bromo-β-(5-imidazole)propionic acid, chloroacetyl phosphate, N-alkylmaleimides, 3-nitro-2-pyridyl disulfide, methyl 2-pyridyl disulfide, p-chloromercuribenzoate, 2-chloromercuri-4-nitrophenol, or chloro-7-nitrobenzo-2-oxa-1,3-diazole.

Histidyl residues are derivatized by reaction with diethylpyrocarbonate at pH 5.5-7.0 because this agent is relatively specific for the histidyl side chain. Para-bromophenacyl bromide also is useful; the reaction is preferably performed in 0.1 M sodium cacodylate at pH 6.0.

Lysinyl and amino terminal residues are reacted with succinic or other carboxylic acid anhydrides. Derivatization with these agents has the effect of reversing the charge of the lysinyl residues. Other suitable reagents for derivatizing α-amino-containing residues include imidoesters such as methyl picolinimidate; pyridoxal phosphate; pyridoxal; chloroborohydride; trinitrobenzenesulfonic acid; O-methylisourea; 2,4-pentanedione; and transaminase-catalyzed reaction with glyoxylate.

Arginyl residues are modified by reaction with one or several conventional reagents, among them phenylglyoxal, 2,3-butanedione, 1,2-cyclohexanedione, and ninhydrin. Derivatization of arginine residues requires that the reaction be performed in alkaline conditions because of the high pKₐ of the guanidine functional group. Furthermore, these reagents may react with the groups of lysine as well as the arginine epsilon-amino group.

The specific modification of tyrosyl residues may be made, with particular interest in introducing spectral labels into tyrosyl residues by reaction with aromatic diazonium compounds or tetranitromethane. Most commonly, N-acetylimidizole and tetranitromethane are used to form O-acetyl tyrosyl species and 3-nitro derivatives, respectively. Tyrosyl residues are iodinated using ¹²⁵I or ¹³¹I to prepare labeled proteins for use in radioimmunoassay, the chloramine T method described above being suitable.

Carboxyl side groups (aspartyl or glutamyl) are selectively modified by reaction with carbodiimides (R'-N = C = N-R'), where R and R' are different alkyl groups, such as 1-cyclohexyl-3-(2-morpholinyl-4-ethyl) carbodiimide or 1-ethyl-3-(4-azonia-4,4-dimethylpentyl) carbodiimide. Furthermore, aspartyl and glutamyl residues are converted to asparaginyl and glutaminyl residues by reaction with ammonium ions.

Derivatization with bifunctional agents is useful for crosslinking PF4AR, its fragments or antibodies to a water-insoluble support matrix or surface for use in methods for purifying anti-PF4AR antibodies, and vice versa. Immobilized PF4AR also is useful in screening for the PF4 superfamily members to which the receptor binds. Commonly used crosslinking 4, agents include, e.g., 1,1-bis(diazoacetyl)-2-phenylethane, glutaraldehyde, N-hydroxysuccinimide esters, for example, esters with 4-azidosalicylic acid, homobifunctional imidoesters, including disuccinimidylesters such as 3,3'-dithiobis(succinimidylpropionate), and bifunctional maleimides such as bis-N-maleimido-1,8-octane. Derivatizing agents such as methyl-3-[(p-azidophenyl)dithio]propioimidate yield photoactivatable intermediates that are capable of forming crosslinks in the presence of light. Alternatively, reactive water-insoluble matrices such as cyanogen bromide-activated carbohydrates and the reactive substrates described in U.S. 3,969,287; 3,691,016; 4,195,128; 4,247,642; 4,229,537; and 4,330,440 are employed for protein immobilization.

Glutaminyl and asparaginyl residues are frequently deamidated to the corresponding glutamyl and aspartyl residues, respectively. Alternatively, these residues are deamidated under mildly acidic conditions. Either form of these residues falls within the scope of this invention.

Other modifications include hydroxylation of proline and lysine, phosphorylation of hydroxyl groups of seryl or threonyl residues, methylation of the α-amino groups of lysine, arginine, and histidine side chains (TE. Creighton, Proteins: Structure and Molecular Properties. W.H. Freeman & Co., San Francisco, pp. 79-86 [1983]), acetylation of the N-terminal amine, and amidation of any C-terminal carboxyl group.

Another type of covalent modification of the beta-8 polypeptide included within the scope of this invention comprises altering the native glycosylation pattern of the polypeptide. By altering is meant deleting one or more carbohydrate moieties found in the native receptor, and/or adding one or more glycosylation sites that are not present in the native receptor.

Glycosylation of polypeptides is typically either N-linked or O-linked. N-linked refers to the attachment of the carbohydrate moiety to the side chain of an asparagine residue. The tri-peptide sequences asparagine-X-serine and asparagine-X-threonine, where X is any amino acid except praline, are the recognition sequences for enzymatic attachment of the carbohydrate moiety to the asparagine side chain. Thus, the presence of either of these tri-peptide sequences in a polypeptide creates a potential glycosylation site. O-linked glycosylation refers to the attachment of one of the sugars N-acetylgalactosamine, galactose, or xylose, to a hydroxyamino acid, most commonly serine or threonine, although 5-hydroxyproline or 5-hydroxylysine may also be used. As noted above, the OL-8 receptor contains 6 putative N-linked glycosylation sites.

Addition of glycosylation sites to the PF4AR polypeptide is conveniently accomplished by altering the amino acid sequence such that it contains one or more of the above-described tri-peptide sequences (for N-linked glycosylation sites). The alteration may also be made by the addition of, or substitution by, one or more serine or threonine residues to the native PF4AR sequence (for O-linked glycosylation sites). For ease, the PF4AR amino acid sequence is preferably altered through changes at the DNA level, particularly by mutating the DNA encoding the PF4AR polypeptide at preselected bases such that codons are generated that will translate into the desired amino acids. The DNA mutation(s) may be made using methods described above under the heading of "Amino Acid Sequence Variants of PF4AR Polypeptide".

Another means of increasing the number of carbohydrate moieties on the PF4AR polypeptide is by chemical or enzymatic coupling of glycosides to the polypeptide. These procedures are advantageous in that they do not require production of the polypeptide in a host cell that has glycosylation capabilities for N- and O- linked glycosylation.
Depending on the coupling mode used, the sugar(s) may be attached to (a) arginine and histidine, (b) free carboxyl groups, (c) free sulfhydryl groups such as those of cysteine, (d) free hydroxyl groups such as those of serine, threonine, or hydroxyproline, (e) aromatic residues such as those of phenylalanine, tyrosine, or tryptophan, or (f) the amide group of glutamine. These methods are described in WO 87/05330 published 11 September 1987, and in Aplin and Wriston (CRC Crit. Rev. Biochem., pp. 259-306 [1981]).

Removal of carbohydrate moieties present on the native PF4AR polypeptide may be accomplished chemically or enzymatically. Chemical deglycosylation requires exposure of the polypeptide to the compound trifluoromethanesulfonic acid, or an equivalent compound. This treatment results in the cleavage of most or all sugars except the linking sugar (N-acetylglucosamine or N-acetytgalactosamine), while leaving the polypeptide intact. Chemical deglycosylaxion is described by Hakimuddin *et al.* (Arch. Biochem. Biophys., **259**:52 [1987]) and by Edge *et al.* (Anal. Biochem., **118**:131 [1981]). Enzymatic cleavage of carbohydrate moieties on polypeptides can be achieved by the use of a variety of endo- and exoglycosidases as described by Thotakura *et al.* (Meth. Enzymol., **138**:350 [1987]).

Glycosylation at potential glycosylation sites may be prevented by the use of the compound tunicamycin as described by Duskin *et al.* (J. Biol. Chem., **257**:3105 [1982]). Tunicamycin blocks the formation of protein-N-glycoside linkages.

The PF4AR also may be entrapped in microcapsules prepared, for example, by coacervation techniques or by interfacial polymerization (for example, hydroxymethylcellulose or gelaxin-microcapsuies and poly-[methylmethacylate] microcapsules, respectively), in colloidal drug delivery systems (for example, liposomes, albumin microspheres, microemulsions, nano-particles and nanocapsules), or in macroemulsions. Such techniques are disclosed in Remington's Pharmaceutical Sciences 16th edition, Osol, A., Ed., (1980).

PF4AR preparations are also useful in generating antibodies, for use as standards in assays for the PF4AR (e.g. by labeling the PF4AR for use as a standard in a radioimmunoassay, enzyme-linked immunoassay, or radioreceptor assay), in affinity purification techniques, and in competitive-type receptor binding assays when labeled with radio-iodine, enzymes, fluorophores, spin labels, and the like.

Since it is often difficult to predict in advance the characteristics of a variant PF4AR, it will be appreciated that some screening of the recovered variant will be needed to select the optimal variant. For example, a change in the immunological character of the PF4AR molecule, such as affinity for a given antibody, is measured by a competitive-type immunoassay. The variant is assayed for changes in the suppression or enhancement of its activity by comparison to the activity observed for native PF4AR in the same assay. Other potential modifications of protein or polypeptide properties such as redox or thermal stability, hydrophobicity, susceptibility to proteolytic degradation, or the tendency to aggregate with carriers or into multimers are assayed by methods well known in the art.

### 3. Therapeutic Compositions and Administration of PF4AR

Therapeutic formulations of PF4AR (including its PF4AR binding fragments) or antibodies thereto are prepared for storage by mixing PF4AR having the desired degree of purity with optional physiologically acceptable carriers, excipients, or stabilizers (Remington's Pharmaceutical Sciences, *supra*), in the form of lyophilized cake or aqueous solutions. Acceptable carriers, excipients or stabilizers are nontoxic to recipients at the dosages and concentrations employed, and include buffers such as phosphate, citrate, and other organic acids; antioxidants including ascorbic acid; low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, arginine or lysine; monosaccharides, disaccharides, and other carbohydrates including glucose, mannose, or dextrins; chelating agents such as EDTA; sugar alcohols such as mannitol or sorbitol; salt-forming counterions such as sodium; and/or nonionic surfactants such as Tween, Pluronics or polyethylene glycol (PEG).

The PF4AR, or antibody to be used for *in vivo* administration must be sterile. This is readily accomplished by filtration through sterile filtration membranes, prior to or following lyophilization and reconstitution. The PF4AR ordinarily will be stored in lyophilized form or in solution.

Therapeutic PF4AR or antibody compositions generally are placed into a container having a sterile access port, for example, an intravenous solution bag or vial having a stopper pierceable by a hypodermic injection needle.

The route of PF4AR or antibody administration is in accord with known methods, e.g. injection or infusion by intravenous, intraperitoneal, intracerebral, intramuscular, intraocular, intraarterial, or intralesional routes, or by sustained release systems as noted below.

Suitable examples of sustained-release preparations include semipermeable polymer matrices in the form of shaped articles, e.g. films, or microcapsules. Sustained release matrices include polyesters, hydrogels, polylactides (U.S. 3,773,919, EP58,481), copolymers of L-glutamic acid and gamma ethyl-L-glutamate (Sidman *et al.,* Biopolymers. **22**:547-556 [1983]), poly (2-hydroxyethyl-methacrylate) (Langer *et al.,* J. Biomed. Mater. Res., **15**:167-277 [1981] and Langer, Chem. Tech., **12**:98-105 [1982]), ethylene vinyl acetate (Langer *et al., supra*) or poly-D-(-)-3-hydroxybutyric acid (EP 133,988). Sustained-release PF4AR or antibody compositions also include liposomally entrapped PF4AR or antibody. Liposomes containing PF4AR or antibody are prepared by methods known *per se:* DE 3,218,121; Epstein *et al*, Proc. Natl. Acad. Sci. USA. **82**:3688-3692 (1985); Hwang *et al.,* Proc. Natl. Acad. Sci. USA. **77**:4030-4034 (1980); EP 52,322; EP 36,676; EP 88,046; EP 143,949; EP 142,641; Japanese patent application 83-118008; U.S. 4,485,045 and 4,544,545; and EP 102,324. Ordinarily the liposomes are of the small (about 200-800 Angstroms) unilamelar type in which the lipid content is greater than about 30 mol. % cholesterol, the selected proportion being adjusted for the optimal PF4AR or antibody therapy.

An effective amount of PF4AR or antibody to be employed therapeutically will depend, for example, upon the therapeutic objectives, the route of administration, and the condition of the patient. Accordingly, it will be necessary for the therapist to titer the dosage and modify the route of administration as required to obtain the optimal therapeutic effect. Typically, the clinician will administer the PF4AR or antibody until a dosage is reached that achieves the desired effect. The progress of this therapy is easily monitored by conventional assays.

### 4. PF4AR Antibody Preparation

Polyclonal antibodies to the PF4AR generally are raised in animals by multiple subcutaneous (sc) or intraperitoneal (ip) injections of the PF4AR and an adjuvant Immunization with recombinant cells transformed with the PF4AR (e.g. mouse or CHO cells transformed with huPF4AR) may be satisfactory, or it may be useful to separate the PF4AR nad conjugate it or a fragment containing the target amino acid sequence to a protein that is immunogenic in the species to be immunized, e.g., keyhole limpet hemocyanin, serum albumin, bovine thyroglobulin, or soybean trypsin inhibitor using a bifunctional or derivatizing agent, for example, maleimidobenzoyl sulfosuccinimide ester (conjugation through cysteine residues), N-hydroxysuccinimide (throughlysine residues), glutaraldehyde, succinic anhydride, SOCl₂, or R¹N = C = NR, where R and R¹ are different alkyl groups.

Animals ordinarily are immunized against the cells or immunogenic conjugates or derivatives by combining 1 mg or 1 µg of PF4AR of Freund's complete adjuvant and injecting the solution intradermally at multiple sites. One month later the animals are boosted with 1/5 to 1/10 the original amount of conjugate in Freund's complete adjuvant by subcutaneous injection at multiple sites. 7 to 14 days later animals are bled and the serum is assayed for anti-PF4AR titer. Animals are boosted until the titer plateaus. Preferably, the animal is boosted with the conjugate of the same PF4AR, but conjugated to a different protein and/or through a different cross-linking agent. Conjugates also can be made in recombinant cell culture as protein fusions. Also, aggregating agents such as alum are used to enhance the immune response.

Another option is to employ combinatorial variable domain libraries and screening methods to identify the desired anti-PF4AR antibodies.

Monoclonal antibodies are prepared by recovering spleen cells from immunized animals and immortalizing the cells in conventional fashion, e.g. by fusion with myeloma cells or by Epstein-Barr (EB)-virus transformation and screening for clones expressing the desired antibody.

The monoclonal antibody preferably is specific for each target PF4AR polypeptide, and will not cross-react with rabbit fMLP receptor⁵, human fMLP receptor, human C5a receptor the low affinity IL-8 receptor or other members of the PF4AR family Antibodies specific for the receptor of Figs. 2, 4 or 5 are preferred. The antibody is selected to be either agonistic, antagonistic or to have no effect on the activity of a PF4 super-family member in binding to or activating the receptor.

Murphy et al. (supra) describe a receptor having a high degree of homology to the receptor of Fig. 2. The Murphy et al. characterized their receptor in recombinant oocytes as being a "low affinity" receptor for IL-8 and having little capability to bind MGSA, thus suggesting that it plays a minor role in IL-8 and MGSA biological activity in vivo. Our studies, however, have shown that the Murphy et al. receptor exhibits IL-8 affinity as high or higher than the receptor of Fig. 2 and that as well it shows high affinity (about 1-10nM) for MGSA. Thus, antagonism of the IL-8 and/or MGSA response of lymphoid cells will likely require that both receptors be inhibited or blocked. For example, one should select an IL-8 antagonist antibody that binds to an epitope of the Fig. 2 receptor that is shared by the Murphy et al. receptor.
This could be readily accomplished by routine screening methods. For example, the candidate antibodies can be assayed for their ability to compete against labelled IL-8 for binding to cells bearing the Fig. 2 receptor, and then the same study conducted with cells bearing the Murphy et al. receptor. Antibodies that inhibit IL-8 activation or binding to both cells are then selected as therapeutic candidates. On the other hand, antibodies that can discriminate between the Fig. 2 and Murphy et al. receptors and bind only to one or the other are useful in diagnosis. The receptor of Fig. 2 binds MGSA poorly, in contrast to the Murphy et al. receptor.

### 5. Uses of PF4AR its nucleic acid and its Antibodies

The nucleic acid encoding the PF4AR may be used as a diagnostic for tissue specific typing. For example, such procedures as *in situ* hybridization, and northern and Southern blotting, and PCR analysis may be used to determine whether DNA and/or RNA encoding the PF4AR are present in the cell type(s) being evaluated. These receptors typically are diagnostic of PBL or monocytic cells.

Isolated PF4AR polypeptide may be used in quantitative diagnostic assays as a standard or control against which samples e.g. from PBL or monocytic cells, containing unknown quantities of PF4AR may be compared. Recombinant cells which express the IL-8 receptor can be used in assays for PF4A ligands in the same fashion as for example neutrophils are used in IL-8 assays. The PF4AR polypeptides, fragments or cells (as such, or derivatized) also can be used as immunogens in the production of antibodies to PF4AR, for the purification of such antibodies from ascites or recombinant cell culture media or for use as competitive anatagonists for superfamily ligands, e.g. IL-8.

The PF4AR are useful in screening for amino acid sequence or other variants of PF4 superfamily members.
For example, a bank of candidate IL-8 amino acid sequence variants are prepared by site directed mutagenesis. These are incubated in competition with labelled native IL-8 for cells bearing the IL-8 receptor of Fig. 2 in order identify agonist or antagonist IL-8 variants. Binding or cell activation are suitable assay endpoints. Alternatively, the receptor is recovered in cell-free form and binding of IL-8 and candidate variants assayed.

PF4AR antibodies are useful in diagnostic assays for PF4AR expression in specific cells or tissues wherein the antibodies are labeled in the same fashion as the PF4AR described above and/or are immobilized on an insoluble matrix. PF4AR antibodies also are useful for the affinity purification of the PF4AR from recombinant cell culture or natural sources. The PF4AR antibodies that do not detectably cross-react with other PF4ARs can be used to purify each PF4AR free from other homologous receptors. PF4AR antibodies that are PF4 antagonists are useful as anti-inflammatory agents or in the treatment of other PF4 superfamily-mediated disorders.

Suitable diagnostic assays for the PF4AR and its antibodies are well known *per se.* Such assays include competitive and sandwich assays, and steric inhibition assays. Competitive and sandwich methods employ a phase-separation step as an integral part of the method while steric inhibition assays are conducted in a single reaction mixture. Fundamentally, the same procedures are used for the assay of the PF4AR and for substances that bind the PF4AR, although certain methods will be favored depending upon the molecular weight of the substance being assayed. Therefore, the substance to be tested is referred to herein as an analyte, irrespective of its status otherwise as an antigen or antibody, and proteins that bind to the analyte are denominated binding partners, whether they be antibodies, cell surface receptors, or antigens.

Analytical methods for the PF4AR or its antibodies all use one or more of the following reagents: labeled analyte analogue, immobilized analyte analogue, labeled binding partner, immobilized binding partner and steric conjugates. The labeled reagents also are known as "tracers."

The label used (and this is also useful to label PF4AR nucleic acid for use as a probe) is any detectable functionality that does not interfere with the binding of analyte and its binding partner. Numerous labels are known for use in immunoassay, examples including moieties that may be detected directly, such as fluorochrome, chemiluminescent, and radioactive labels, as well as moieties, such as enzymes, that must be reacted or derivatized to be detected. Examples of such labels include the radioisotopes ³²P, ¹⁴C, ¹²⁶I ³H, and ¹³¹I, fluorophores such as rare earth chelates or fluorescein and its derivatives, rhodamine and its derivatives, dansyl, umbelliferone, luceriferases, e.g., firefly luciferase and bacterial luciferase (U.S. Pat. No. 4,737,456), luciferin, 2,3-dihydrophthalazinediones, horseradish peroxidase (HRP), alkaline phosphatase, β-galactosidase, glucoamylase, lysozyme, saccharide oxidases, e.g., glucose oxidase, galactose oxidase, and glucose-6-phosphate dehydrogenase, heterocyclic oxidases such as uricase and xanthine oxidase, coupled with an enzyme that employs hydrogen peroxide to oxidize a dye precursor such as HRP, lactoperoxidase, or microperoxidase, biotin/avidin, spin labels, bacteriophage labels, stable free radicals, and the like.

Conventional methods are available to bind these labels covalently to proteins or polypeptides. For instance, coupling agents such as dialdehydes, carbodiimides, dimaleimides, bis-imidates, bis-diazotized benzidine, and the like may be used to tag the antibodies with the above-described fluorescent, chemiluminescent, and enzyme labels. See, for example, U.S. Pat. Nos. 3,940,475 (fluorimetry) and 3,645,090 (enzymes); Hunter *et al.,* Nature, **144**:946 (1962); David *et al.,* Biochemistry. **13**:1014-1021 (1974); Pain *et al.,* J. Immunol. Methods. **40**:219-230 (1981); and Nygren, J. Histochem. and Cytochem., **30**:407-412 (1982). Preferred labels herein are enzymes such as horseradish peroxidase and alkaline phosphatase.

The conjugation of such label, including the enzymes, to the antibody is a standard manipulative procedure for one of ordinary skill in immunoassay techniques. See, for example, O'Sullivan *et al.,* "Methods for the Preparation of Enzyme-antibody Conjugates for Use in Enzyme immunoassay," in Methods in Enzymology, ed. J.J. Langone and H. Van Vunakis. Vol. 73 (Academic Press, New York, New York, 1981), pp. 147-166. Such bonding methods are suitable for use with PF4AR or its antibodies, all of which are proteinaceous.

Immobilization of reagents is required for certain assay methods. Immobilization entails separating the binding partner from any analyte that remains free in solution. This conventionally is accomplished by either insolubilizing the binding partner or analyte analogue before the assay procedure, as by adsorption to a water-insoluble matrix or surface (Bennich *et al.,* U.S. 3,720,760), by covalent coupling (for example, using glutaraldehyde cross-linking), or by insolubilizing the partner or analogue afterward, e.g., by immunoprecipitation.

Other assay methods, known as competitive or sandwich assays, are well established and widely used in the commercial diagnostics industry.

Competitive assays rely on the ability of a tracer analogue to compete with the test sample analyte for a limited number of binding sites on a common binding partner. The binding partner generally is insolubilized before or after the competition and then the tracer and analyte bound to the binding partner are separated from the unbound tracer and analyte. This separation is accomplished by decanting (where the binding partner was preinsolubilized) or by centrifuging (where the binding partner was precipitated after the competitive reaction). The amount of test sample analyte is inversely proportional to the amount of bound tracer as measured by the amount of marker substance. Dose-response curves with known amounts of analyte are prepared and compared with the test results to quantitatively determine the amount of analyte present in the test sample. These assays are called ELISA systems when enzymes are used as the detectable markers.

Another species of competitive assay, called a "homogeneous" assay, does not require a phase separation. Here, a conjugate of an enzyme with the analyte is prepared and used such that when anti-analyte binds to the analyte the presence of the anti-analyte modifies the enzyme activity. In this case, the PF4AR or its immunologically active fragments are conjugated with a bifunctional organic bridge to an enzyme such as peroxidase. Conjugates are selected for use with anti-PF4AR so that binding of the anti-PF4AR inhibits or potentiates the enzyme activity of the label. This method per se is widely practiced under the name of EMIT.

Steric conjugates are used in steric hindrance methods for homogeneous assay. These conjugates are synthesized by covalently linking a low-molecular-weight hapten to a small analyte so that antibody to hapten substantially is unable to bind the conjugate at the same time as anti-analyte. Under this assay procedure the analyte present in the test sample will bind anti-analyte, thereby allowing anti-hapten to bind the conjugate, resulting in a change in the character of the conjugate hapten, e.g.. a change in fluorescence when the hapten is a fluorophore.

Sandwich assays particularly are useful for the determination of PF4AR or PF4AR antibodies. In sequential sandwich assays an immobilized binding partner is used to adsorb test sample analyte, the test sample is removed as by washing, the bound analyte is used to adsorb labeled binding partner, and bound material is then separated from residual tracer. The amount of bound tracer is directly proportional to test sample analyte. In "simultaneous" sandwich assays the test sample is not separated before adding the labeled binding partner. A sequential sandwich assay using an anti-PF4AR monoclonal antibody as one antibody and a polyclonal anti-PF4AR antibody as the other is useful in testing samples for PF4AR activity.

The foregoing are merely exemplary diagnostic assays for PF4AR and antibodies. Other methods now or hereafter developed for the determination of these analytes are included within the scope hereof, including the bioassays described above.

The polypeptides set forth in Figs. 4 and 5 are believed to represent receptors for different and as yet undetermined members of the PF4 superfamily (which includes both the C-C and CXC subfamilies). Like the IL-8 receptor of Fig. 2 they are members of the G-protein-coupled superfamily and bear greater similarity to the IL-8 receptor than other receptors. In preliminary experiments, recombinant cells bearing these receptors do not respond to Rantes, MCP1, IL-8 or MGSA, although they may ultimately be shown to bind other members of the PF4 superfamily or presently unknown ligands. However, whether or not the Figs. 4 or 5 polypeptides bind to members of the PF4 superfamily, the polypeptides are useful for preparing antibodies for diagnostic use in determining the tissue distribution of the receptors and thus as an immunohistochemical diagnostic for such tissues, in particular as a diagnostic for monocytic cells or PBLs since it is known that such cells express the receptors of Figs. 4 and 5. Of course, once the PF4 super-family members are identified which bind to these receptors then the receptors can be used to diagnose the presence of the identified members or for their purification in specific affinity procedures. The DNA in Figs. 4 and 5 also is useful in diagnostics for the presence of DNA or RNA encoding the IL-8 receptor when low stringency conditions are employed.

The following examples are offered by way of illustration and not by way of limitation.

### EXAMPLE 1

To obtain the clone pRKSB.il8r1.1, a cDNA¹⁶ library of 1,000,000 clones was constructed from human neutrophil mRNA¹⁷ in the vector pRK5B using bstXI linkers. The cDNA is produced in blunted form. Hemi-kinase bstXI linkers are ligated to the CDNA, and the linkers ligated into the PRK5B vector that had been bstXI digested, phosphatased, and the long vector fragment isolated. PRK5B is a derivative of PRK5¹⁸ that contains a cytomegalovirus promoter followed by a 5' intron, bstXl cloning site, and an SV40 early polyadenylation signal, although it will be understood that any mammalian cell expression vector will be satisfactory, 58 pools of 2500 clones each were transfected into COS-7 cells by electroporation¹⁹ of 20 µg of DNA into 3,750,000 cells After 2 days of growth on 150-mm dishes in medium (50:50::Ham's F12:DMEM) containing 10% fetal calf serum, ¹²⁵I-IL-8 binding was performed. Purified human 72 amino acid IL-8 made in E. coli²⁰ was labeled by the lactoperoxidase method²¹ to about 1100 Ci/mmol and was at least 85 % bindable. Dishes were rinsed twice with phosphate-buffered saline, and binding was performed with 8 ml per dish of growth medium containing 2.5 % fetal calf serum and about 0.5 nM ¹²⁵I-IL-8. After 2 hr at 37°, the plates were rinsed three times with phosphate-buffered saline, the bottoms cut out²², and autoradiographed. Each positive pool of 2500 cDNA clones was subsequently partitioned into pools of 800 clones, and each of these was transfected and assayed. Each positive pool in turn was subdivided through pools of 185, 30 and finally a single clone(s) until single positive clones were identified to obtain the pure isolate. Since only a portion of each pool was used for transfection it was unnecessary to rescue clones from transformants.

Binding competition was performed with electroporated COS-7 cells after 1 day of expression in 6-well dishes (about 175,000 cells/dish). Binding was performed with radioiodinated wild type IL-8 in binding medium Ca²⁺ and Mg²⁺ -free Hanks buffered with 25 nM Hepes and supplemented with 0.5% BSA) at 4° for about 2 hr. Wells were then washed, the cells harvested with trypsin, and counted. No specific binding was found in parallel wells containing cells transfected with DNA from the vector pRK5B. Neutrophil binding was performed as described²² but for 2 hr at 4°.

### EXAMPLE 2

Existing λgt10 cDNA libraries from the human cell line, HL60, and from human peripheral blood lymphocytes were screened at low stringency with a probe from the coding region of the cloned high-affinity human IL-8 receptor (Fig. 2). The probe was the 874 bp PstI/NcoI fragment of the receptor containing the coding region for amino acids 23-314. Hybridization was in 20% formamide, 4 x SSC, 50 mM sodium phosphate buffer, pH 7, 0.2 g/l sonicated salmon sperm DNA, 5 x Denhardts, 10% dextran sulfate, at 42°C with a wash at 1 x SSC, 0.1 % SDS at 50°C. A number of duplicate spots of varying intensity (about 60) were picked, plaque purified, subcloned into plasmid vectors, and sequenced. Nucleic acid sequencing began with the selection of spots of greatest intensity. Sufficient sequence was obtained for a given spot (phage) to determine whether or not evidence of structural or sequence homology with the IL-8 receptor existed. If it did, then the remainder of the gene was obtained (if necessary) and sequenced in its entirety. To avoid sequences all hybridizing clone the sequence was then used to probe the parental collection of IL-8 receptor DNA hybridizing clones under high stringency conditions in order to identify and discard other spots containing the same hybridizing gene. This technique was highly effective in reducing the sequencing burden. For example, one clone was represented by about one third of the initial 60 clones, and on this result alone the negative screen was able to reduce considering the work involved in sequencing the clones.

From this screen, two new gene sequences were found that are clearly related to the IL-8 receptor. The coding region for one new gene was split between two clones (8rr.20 and 8rr.15). The combined sequence of this gene (8rr.20.15) is shown in Figure 4. The complete coding region for the second gene is found on clone 8rr.9 (Figure 5). The predicted amino acid sequence of 8rr.20.15 is 34% identical with both the high and low affinity IL-8 receptor sequences. The sequence of 8rr.9 is 36% and 38% identical with the high and low affinity IL-8 receptor sequences, respectively (W.E. Holmes *et al.,* Science **253**, 1278 (1991), and P.M. Murphy *et al.,* Science **253**, 1280 (1991). The amino acid sequence of 8rr.20.15 and 8rr.9 are 31 % identical. Use of this probe under low stringency conditions did not produce detectable hybridization to the fMLP receptor genes that were expected to be found in these libraries.

### References

1. Oppenheim, J.J. *et al.,* Annu. Rev. Immunol., **9**:617-648 (1991).
2. Gimbrone, M.A.Jr. *et al.,* Science, **246**:1601-3 (1989).
3. Boulay, F. *et al.,* Biochem. Biophys. Res. Comm., **168**:1103-1109 (1990).
4. Gerard, N.P & Gerard, C., Nature, **349**:614-617 (1991).
5. Thomas, K.M., Pyun, H.Y & Navarro, J., J. Biol. Chem., **265**:20061-20064 (1990).
6. Sims, J.E. *et al.,* Science. **241**:585-589 (1988).
7. D'Andrea, A.D., Lodish, H.F & Wong, G.G., Cell. **57**:277-285 (1989).
8. Samanta, A.K., Oppenheim, J.J. & Matsushima, K., J. Exp. Med., **169**:1185-1189 (1989).
9. Besemer, J., Hujber, A. & Kuhn, B., J. Biol. Chem., **264**:17409-17415 (1989).
10. Grob, P.M. *et al,* J. Biol. Chem., **265**:8311-8316 (1990).
11. Kozak, M., Nucleic Acid Res., **12**:857-872 (1984).
12. Dixon, R.A.F., Sigal, I.S. & Strader, C.D., Cold Spring Har. Sym. Quant. Biol., **53**:487-497 (1988).
13. Korchak, H.M. *et al.,* J. Biol Chem., **259**:4076-4082 (1984).
14. Tennenberg, S.D., Zemlan, F.P. & Solomkin J.S., J. Immunol., **141**:3937-3944 (1988).
15. Ramachandran, J. *et al.,* BigEssays, **10**:54-57 (1989).
16. Gubler, U. & Hoffman, B.J. *Gene,* **25**, 263-269, (1983).
17. Chirgwin, J.J. *et al.* Biochem., **18**:5294-5299 (1979).
18. EP 307,247.
19. Gearing, D.P. *et al.* EMBO J., **8**:3667-3676 (1989).
20. Hebert, C.A. et al. J. Immunol., **145**:3033-3040 (1991).
21. Morrison M. & Bayse, G.S., Biochem., **9**:2995-3000 (1970).
22. Pacholcryk, T., Blakely, R.D. & Amara, S.G., BioTechniques, **9**:556-558 (1990).
23. Grynkiewicz, G., Poenie, M. & Tsien, R.Y., J. Biol. Chem., **260**:3440-3450 (1985).

## Claims (Claims for the following Contracting State(s): AT, BE, CH, DE, DR, FR, GB, IT, LI, LU, MC, NL, SE)

1. An isolated platelet factor 4 superfamily receptor (PF4AR) polypeptide having at least an 85% amino acid sequence homology with the translated amino acid sequence of figure 2.

2. The PF4AR polypeptide of claim 1 that comprises the polypeptide of figure 2.

3. An isolated polypeptide comprising the PF4AR polypeptide of any one of the preceding claims fused to a polypeptide heterologous to the PF4AR polypeptide.

4. The PF4AR polypeptide of any one of the preceding claims wherein the polypeptide binds IL-8 with no binding or response to f-Met-Leu-Phe.

5. The PF4AR polypeptide of any one of the preceding claims that is human.

6. An isolated nucleic acid molecule comprising a nucleic acid sequence encoding the PF4AR polypeptide of any one of claims 1 to 5.

7. The nucleic acid molecule of claim 6 which is DNA and has the translated DNA sequence shown in figure 2.

8. The nucleic acid molecule of claim 6 or claim 7 further comprising a promoter operably linked to the nucleic acid sequence encoding the PF4AR polypeptide.

9. An expression vector comprising the nucleic acid sequence of any one of claims 6 to 7 operably linked to control sequences recognized by a host cell transformed with the vector.

10. A host cell transformed with the vector of claim 9.

11. A method of using a nucleic acid molecule containing a nucleic acid sequence encoding the PF4AR polypeptide of any one of claims 1 to 5, comprising expressing the nucleic acid molecule in a cultured host cell transformed with a vector comprising the nucleic acid sequence encoding the PF4AR polypeptide operably linked to control sequences recognized by the host cell transformed with the vector, thereby producing the PF4AR polypeptide, and recovering the PF4AR polypeptide from the host cell.

12. The method of claim 11 wherein the PF4AR polypeptide is recovered from the host cell membranes.

13. A monoclonal antibody that is capable of specifically binding the PF4AR polypeptide according to claim 1.

14. A monoclonal antibody that is capable of specifically binding the PF4AR polypeptide of figure 2.

15. The monoclonal antibody of claim 14 which is capable of specifically binding an N-terminal extracellular region of the PF4AR polypeptide.

16. A composition comprising the monoclonal antibody of any one of claims 13 to 15 and a pharmaceutically acceptable carrier.

17. A monoclonal antibody of any one of claims 13 to 15 for use in therapy or diagnosis.

18. An isolated platelet factor 4 superfamily receptor (PF4AR) polypeptide having at least an 85% amino acid sequence homology with the translated amino acid sequence of figure 4 or figure 5.

19. An isolated PF4AR polypeptide wherein the nucleic acid encoding the PF4AR polypeptide hybridises with the complement of the nucleic acid encoding the polypeptide of figures 4 or 5 under high stringency conditions.

20. An isolated PF4AR polypeptide comprising an amino acid sequence that is at least 10 residues in length and is contained in an extracellular region of the polypeptide of figure 4 or 5 and is capable of raising an antibody that will cross-react with the polypeptide of figure 4 or 5.

21. The PF4AR polypeptide of claim 20 wherein the extracellular region is the N-terminal extracellular region of figure 4 or 5.

22. An isolated polypeptide comprising the PF4AR polypeptide of any one of claims 18 to 21 fused to a polypeptide heterologous to the PF4AR polypeptide.

23. An isolated nucleic acid molecule comprising a nucleic acid sequence encoding the PF4AR polypeptide of any one of claims 18 to 22.

24. The nucleic acid molecule of claim 23 which is DNA and has the translated DNA sequence shown in figure 4 or figure 5.

25. The nucleic acid molecule of claim 23 or claim 24 further comprising a promoter operably linked to the nucleic acid sequence encoding the PF4AR polypeptide.

26. An expression vector comprising the nucleic acid sequence of any one of claims 23 to 25 operably linked to control sequences recognized by a host cell transformed with the vector.

27. A host cell transformed with the vector of claim 26.

28. A method of using a nucleic acid molecule containing a nucleic acid sequence encoding the PF4AR polypeptide of any one of claims 18 to 22, comprising expressing the nucleic acid molecule in a cultured host cell transformed with a vector comprising the nucleic acid sequence encoding the PF4AR polypeptide operably linked to control sequences recognized by the host cell transformed with the vector, thereby producing the PF4AR polypeptide, and recovering the PF4AR polypeptide from the host cell.

29. The method of claim 28 wherein the PF4AR polypeptide is recovered from the host cell membranes.

30. A monoclonal antibody that is capable of specifically binding the PF4AR polypeptide according to any one of claims 18 to 21.

31. The monoclonal antibody of claim 30 which is capable of specifically binding an N-terminal extracellular region of the PF4AR polypeptide.

32. The monoclonal antibody of claim 30 or 31 that is capable of specifically binding the PF4AR polypeptide of figure 4.

## Claims (Claims for the following Contracting State(s): ES, GR)

1. An isolated platelet factor 4 superfamily receptor (PF4AR) polypeptide having at least an 85% amino acid sequence homology with the translated amino acid sequence of figure 2.

2. The PF4AR polypeptide of claim 1 that comprises the polypeptide of figure 2.

3. An isolated polypeptide comprising the PF4AR polypeptide of any one of the preceding claims fused to a polypeptide heterologous to the PF4AR polypeptide.

4. The PF4AR polypeptide of any one of the preceding claims wherein the polypeptide binds IL-8 with no binding or response to f-Met-Leu-Phe.

5. The PF4AR polypeptide of any one of the preceding claims that is human.

6. An isolated nucleic acid molecule comprising a nucleic acid sequence encoding the PF4AR polypeptide of any one of claims 1 to 5.

7. The nucleic acid molecule of claim 6 which is DNA and has the translated DNA sequence shown in figure 2.

8. The nucleic acid molecule of claim 6 or claim 7 further comprising a promoter operably linked to the nucleic acid sequence encoding the PF4AR polypeptide.

9. An expression vector comprising the nucleic acid sequence of any one of claims 6 to 8 operably linked to control sequences recognized by a host cell transformed with the vector.

10. A host cell transformed with the vector of claim 9.

11. A method of using a nucleic acid molecule containing a nucleic acid sequence encoding the PF4AR polypeptide of any one of claims 1 to 5, comprising expressing the nucleic acid molecule in a cultured host cell transformed with a vector comprising the nucleic acid sequence encoding the PF4AR polypeptide operably linked to control sequences recognized by the host cell transformed with the vector, thereby producing the PF4AR polypeptide, and recovering the PF4AR polypeptide from the host cell.

12. The method of claim 11 wherein the PF4AR polypeptide is recovered from the host cell membranes.

13. A monoclonal antibody that is capable of specifically binding the PF4AR polypeptide according to claim 1.

14. A monoclonal antibody that is capable of specifically binding the PF4AR polypeptide of figure 2.

15. The monoclonal antibody of claim 14 which is capable of specifically binding an N-terminal extracellular region of the PF4AR polypeptide.

16. A composition comprising the monoclonal antibody of any one of claims 13 to 15 and a pharmaceutically acceptable carrier.

17. A monoclonal antibody of any one of claims 13 to 15 for use in therapy or diagnosis.

18. A method of using a PF4AR polypeptide as defined in any one of claims 1 to 9, the method comprising:
using a recombinant cell expressing the PF4AR polypeptide, or the PF4AR polypeptide and an adjuvant, or the PF4AR polypeptide conjugated to an immunogenic peptide, to immunise an animal to raise antibodies against a PF4AR epitope; and
preparing monoclonal antibodies from cells of the immunised animal,
wherein the monoclonal antibodies are capable of specifically binding a PF4AR polypeptide as defined in claim 1.

19. A method of using a PF4AR polypeptide as defined in any one of claims 1 to 5, the method comprising:
using a recombinant cell expressing the PF4AR polypeptide, or the PF4AR polypeptide and an adjuvant, or the PF4AR polypeptide conjugated to an immunogenic peptide, to immunise an animal to raise antibodies against a PF4AR epitope; and
preparing monoclonal antibodies from cells of the immunised animal,
wherein the monoclonal antibodies are capable of specifically binding the PF4AR polypeptide of figure 2.

20. A method according to claim 19 wherein the antibodies are capable of specifically binding an N-terminal extracellular region of the PF4AR polypeptide.

21. A method according to any one of claims 18 to 20 further comprising mixing the antibodies with a pharmaceutically acceptable carrier.

22. A method of preparing a therapeutic formulation of a PF4AR polypeptide as defined in any one of claims 1 to 5 or a monoclonal antibody as defined in any one of claims 13 to 15, the method comprising mixing the polypeptide or antibody with physiologically acceptable carriers, excipients or stabilizers.

23. A method according to claim 22 wherein the antibody is an antibody according to claim 15.

24. An isolated platelet factor 4 superfamily receptor (PF4AR) polypeptide having at least an 85% amino acid sequence homology with the translated amino acid sequence of figure 4 or figure 5.

25. An isolated PF4AR polypeptide wherein the nucleic acid encoding the PF4AR polypeptide hybridises with the complement of the nucleic acid encoding the polypeptide of figures 4 or 5 under high stringency conditions.

26. An isolated PF4AR polypeptide comprising an amino acid sequence that is at least 10 residues in length and is contained in an extracellular region of the polypeptide of figure 4 or 5 and is capable of raising an antibody that will cross-react with the polypeptide of figure 4 or 5.

27. The PF4AR polypeptide of claim 26 wherein the extracellular region is the N-terminal extracellular region of figure 4 or 5.

28. An isolated polypeptide comprising the PF4AR polypeptide of any one of claims 24 to 27 fused to a polypeptide heterologous to the PF4AR polypeptide.

29. An isolated nucleic acid molecule comprising a nucleic acid sequence encoding the PF4AR polypeptide of any one of claims 24 to 28.

30. The nucleic acid molecule of claim 29 which is DNA and has the translated DNA sequence shown in figure 4 or figure 5.

31. The nucleic acid molecule of claim 29 or claim 30 further comprising a promoter operably linked to the nucleic acid sequence encoding the PF4AR polypeptide.

32. An expression vector comprising the nucleic acid sequence of any one of claims 29 to 31 operably linked to control sequences recognized by a host cell transformed with the vector.

33. A host cell transformed with the vector of claim 32.

34. A method of using a nucleic acid molecule containing a nucleic acid sequence encoding the PF4AR polypeptide of any one of claims 24 to 28, comprising expressing the nucleic acid molecule in a cultured host cell transformed with a vector comprising the nucleic acid sequence encoding the PF4AR polypeptide operably linked to control sequences recognized by the host cell transformed with the vector, thereby producing the PF4AR polypeptide, and recovering the PF4AR polypeptide from the host cell.

35. The method of claim 34 wherein the PF4AR polypeptide is recovered from the host cell membranes.

36. A monoclonal antibody that is capable of specifically binding the PF4AR polypeptide according to any one of claims 24 to 27.

37. The monoclonal antibody of claim 36 which is capable of specifically binding an N-terminal extracellular region of the PF4AR polypeptide.

38. The monoclonal antibody of claim 36 or 37 that is capable of specifically binding the PF4AR polypeptide of figure 4.

39. A method of using a PF4AR polypeptide as defined in any one of claims 24 to 28, the method comprising:
using a recombinant cell expressing the PF4AR polypeptide, or the PF4AR polypeptide and an adjuvant, or the PF4AR polypeptide conjugated to an immunogenic peptide, to immunise an animal to raise antibodies against a PF4AR epitope; and
preparing monoclonal antibodies from cells of the immunised animal,
wherein the monoclonal antibodies are capable of specifically binding a PF4AR polypeptide as defined in any one of claims 24 to 27.

40. A method of using a PF4AR polypeptide as defined in any one of claims 24 to 28, the method comprising:
using a recombinant cell expressing the PF4AR polypeptide, or the PF4AR polypeptide and an adjuvant, or the PF4AR polypeptide conjugated to an immunogenic peptide, to immunise an animal to raise antibodies against a PF4AR epitope; and
preparing monoclonal antibodies from cells of the immunised animal,
wherein the monoclonal antibodies are capable of specifically binding the PF4AR polypeptide of figure 4 or 5.

41. A method according to claim 40 wherein the antibodies are capable of specifically binding an N-tenninal extracellular region of the PF4AR polypeptide.

42. A method according to claim 40 or 41 wherein the antibodies are capable of specifically binding the PF4AR polypeptide of figure 4.

## Patentansprüche (Patentansprüche für folgende(n) Vertragsstaat(en): AT, BE, CH, DE, DR, FR, GB, IT, LI, LU, MC, NL, SE)

1. Isoliertes Blutplättchen-Faktor-4-Überfamilien-Rezeptor- (PF4AR-) Polypeptid mit zumindest 85%iger Aminosäuresequenz-Homologie mit der translatierten Aminosäuresequenz aus Fig. 2.

2. PF4AR-Polypeptid nach Anspruch 1, welches das Polypeptid aus Fig. 2 umfasst.

3. Isoliertes Polypeptid, welches das PF4AR-Polypeptid nach einem der vorangegangenen Ansprüche an ein Polypeptid fusioniert umfasst, das zum PF4AR-Polypeptid heterolog ist.

4. PF4AR-Polypeptid nach einem der vorangegangenen Ansprüche, worin das Polypeptid IL-8 bindet, ohne sich an f-Met-Leu-Phe zu binden oder darauf anzusprechen.

5. PF4AR-Polypeptid nach einem der vorangegangenen Ansprüche, das ein humanes ist.

6. Isoliertes Nucleinsäuremolekül, das eine Nucleinsäuresequenz umfasst, die für das PF4AR-Polypeptid nach einem der Ansprüche 1 bis 5 kodiert.

7. Nucleinsäuremolekül nach Anspruch 6, das DNA ist und die in Fig. 2 gezeigte translatierte DNA-Sequenz aufweist.

8. Nucleinsäuremolekül nach Anspruch 6 oder Anspruch 7, das weiters einen Promotor umfasst, der an die für das PF4AR-Polypeptid kodierende Nucleinsäuresequenz operabel gebunden ist.

9. Expressionsvektor, der die Nucleinsäuresequenz nach einem der Ansprüche 6 bis 7 operabel an Kontrollsequenzen gebunden umfasst, die von einer mit dem Vektor transformierten Wirtszelle erkannt werden.

10. Mit dem Vektor nach Anspruch 9 transformierte Wirtszelle.

11. Verfahren zur Verwendung eines Nucleinsäuremoleküls, das eine für das PF4AR-Polypeptid nach einem der Ansprüche 1 bis 5 kodierende Nucleinsäuresequenz enthält, umfassend das Exprimieren des Nucleinsäuremoleküls in einer kultivierten Wirtszelle, die mit einem Vektor transformiert ist, der die Nucleinsäuresequenz umfasst, die für das PF4AR-Polypeptid kodiert und an Kontrollsequenzen operabel gebunden ist, die von der mit dem Vektor transformierten Wirtszelle erkannt werden, wodurch das PF4AR-Polypeptid produziert wird, sowie das Gewinnen des PF4AR-Polypeptids aus der Wirtszelle.

12. Verfahren nach Anspruch 11, worin das PF4AR-Polypeptid aus den Wirtszellmembranen gewonnen wird.

13. Monoklonaler Antikörper, der fähig ist, das PF4AR-Polypeptid nach Anspruch 1 spezifisch zu binden.

14. Monoklonaler Antikörper, der fähig ist, das PF4AR-Polypeptid aus Fig. 2 spezifisch zu binden.

15. Monoklonaler Antikörper nach Anspruch 14, der fähig ist, eine N-terminale extrazelluläre Region des PF4AR-Polypeptids spezifisch zu binden.

16. Zusammensetzung, die den monoklonalen Antikörper nach einem der Ansprüche 13 bis 15 und einen pharmazeutisch annehmbaren Träger umfasst.

17. Monoklonaler Antikörper nach einem der Ansprüche 13 bis 15 zur Verwendung bei der Therapie oder Diagnose.

18. Isoliertes Blutplättchen-Faktor-4-Überfamilien-Rezeptor- (PF4AR-) Polypeptid mit zumindest 85%iger Aminosäuresequenz-Homologie mit der translatierten Aminosäuresequenz aus Fig. 4 oder Fig. 5.

19. Isoliertes PF4AR-Polypeptid, worin die Nucleinsäure, die für das PF4AR-Polypeptid kodiert, unter hochstringenten Bedingungen mit dem Komplement der für das Polypeptid aus Fig. 4 oder 5 kodierenden Nucleinsäure hybridisiert.

20. Isoliertes PF4AR-Polypeptid, das eine Aminosäuresequenz mit einer Länge von zumindest 10 Resten umfasst, die in einer extrazellulären Region des Polypeptids aus Fig. 4 oder 5 enthalten ist und fähig ist, einen Antikörper hervorzubringen, der mit dem Polypeptid aus Fig. 4 oder 5 kreuzreagiert.

21. PF4AR-Polypeptid nach Anspruch 20, worin die extrazelluläre Region die N-terminale extrazelluläre Region aus Fig. 4 oder 5 ist.

22. Isoliertes Polypeptid, welches das PF4AR-Polypeptid nach einem der Ansprüche 18 bis 21 an ein Polypeptid fusioniert umfasst, das zum PF4AR-Polypeptid heterolog ist.

23. Isoliertes Nucleinsäuremolekül, das eine Nucleinsäuresequenz umfasst, die für das PF4AR-Polypeptid nach einem der Ansprüche 18 bis 22 kodiert.

24. Nucleinsäuremolekül nach Anspruch 23, das DNA ist und die in Fig. 4 oder Fig. 5 gezeigte translatierte DNA-Sequenz aufweist.

25. Nucleinsäuremolekül nach Anspruch 23 oder Anspruch 24, das weiters einen Promotor umfasst, der an die für das PF4AR-Polypeptid kodierende Nucleinsäuresequenz operabel gebunden ist.

26. Expressionsvektor, der die Nucleinsäuresequenz nach einem der Ansprüche 23 bis 25 operabel an Kontrollsequenzen gebunden umfasst, die von einer mit dem Vektor transformierten Wirtszelle erkannt werden.

27. Mit dem Vektor nach Anspruch 26 transformierte Wirtszelle.

28. Verfahren zur Verwendung eines Nucleinsäuremoleküls, das eine für das PF4AR-Polypeptid nach einem der Ansprüche 18 bis 22 kodierende Nucleinsäuresequenz enthält, umfassend das Exprimieren des Nucleinsäuremoleküls in einer kultivierten Wirtszelle, die mit einem Vektor transformiert ist, der die Nucleinsäuresequenz umfasst, die für das PF4AR-Polypeptid kodiert und an Kontrollsequenzen operabel gebunden ist, die von der mit dem Vektor transformierten Wirtszelle erkannt werden, wodurch das PF4AR-Polypeptid produziert wird, sowie das Gewinnen des PF4AR-Polypeptids aus der Wirtszelle.

29. Verfahren nach Anspruch 28, worin das PF4AR-Polypeptid aus den Wirtszellmembranen gewonnen wird.

30. Monoklonaler Antikörper, der fähig ist, das PF4AR-Polypeptid nach einem der Ansprüche 18 bis 21 spezifisch zu binden.

31. Monoklonaler Antikörper nach Anspruch 30, der fähig ist, eine N-terminale extrazelluläre Region des PF4AR-Polypeptids spezifisch zu binden.

32. Monoklonaler Antikörper nach Anspruch 30 oder 31, der fähig ist, das PF4AR-Polypeptid aus Fig. 4 spezifisch zu binden.

## Patentansprüche (Patentansprüche für folgende(n) Vertragsstaat(en): ES, GR)

1. Isoliertes Blutplättchen-Faktor-4-Überfamilien-Rezeptor- (PF4AR-) Polypeptid mit zumindest 85%iger Aminosäuresequenz-Homologie mit der translatierten Aminosäuresequenz aus Fig. 2.

2. PF4AR-Polypeptid nach Anspruch 1, welches das Polypeptid aus Fig. 2 umfasst.

3. Isoliertes Polypeptid, welches das PF4AR-Polypeptid nach einem der vorangegangenen Ansprüche an ein Polypeptid fusioniert umfasst, das zum PF4AR-Polypeptid heterolog ist.

4. PF4AR-Polypeptid nach einem der vorangegangenen Ansprüche, worin das Polypeptid IL-8 bindet, ohne sich an f-Met-Leu-Phe zu binden oder darauf anzusprechen.

5. PF4AR-Polypeptid nach einem der vorangegangenen Ansprüche, das ein humanes ist.

6. Isoliertes Nucleinsäuremolekül, das eine Nucleinsäuresequenz umfasst, die für das PF4AR-Polypeptid nach einem der Ansprüche 1 bis 5 kodiert.

7. Nucleinsäuremolekül nach Anspruch 6, das DNA ist und die in Fig. 2 gezeigte translatierte DNA-Sequenz aufweist.

8. Nucleinsäuremolekül nach Anspruch 6 oder Anspruch 7, das weiters einen Promotor umfasst, der an die für das PF4AR-Polypeptid kodierende Nucleinsäuresequenz operabel gebunden ist.

9. Expressionsvektor, der die Nucleinsäuresequenz nach einem der Ansprüche 6 bis 8 operabel an Kontrollsequenzen gebunden umfasst, die von einer mit dem Vektor transformierten Wirtszelle erkannt werden.

10. Mit dem Vektor nach Anspruch 9 transformierte Wirtszelle.

11. Verfahren zur Verwendung eines Nucleinsäuremoleküls, das eine für das PF4AR-Polypeptid nach einem der Ansprüche 1 bis 5 kodierende Nucleinsäuresequenz enthält, umfassend das Exprimieren des Nucleinsäuremoleküls in einer kultivierten Wirtszelle, die mit einem Vektor transformiert ist, der die Nucleinsäuresequenz umfasst, die für das PF4AR-Polypeptid kodiert und operabel an Kontrollsequenzen gebunden ist, die von der mit dem Vektor transformierten Wirtszelle erkannt werden, wodurch das PF4AR-Polypeptid produziert wird, sowie das Gewinnen des PF4AR-Polypeptids aus der Wirtszelle.

12. Verfahren nach Anspruch 11, worin das PF4AR-Polypeptid aus den Wirtszellmembranen gewonnen wird.

13. Monoklonaler Antikörper, der fähig ist, das PF4AR-Polypeptid nach Anspruch 1 spezifisch zu binden.

14. Monoklonaler Antikörper, der fähig ist, das PF4AR-Polypeptid aus Fig. 2 spezifisch zu binden.

15. Monoklonaler Antikörper nach Anspruch 14, der fähig ist, eine N-terminale extrazelluläre Region des PF4AR-Polypeptids spezifisch zu binden.

16. Zusammensetzung, die den monoklonalen Antikörper nach einem der Ansprüche 13 bis 15 und einen pharmazeutisch annehmbaren Träger umfasst.

17. Monoklonaler Antikörper nach einem der Ansprüche 13 bis 15 zur Verwendung bei der Therapie oder Diagnose.

18. Verfahren zur Verwendung eines PF4AR-Polypeptids nach einem der Ansprüche 1 bis 9, wobei das Verfahren umfasst:
die Verwendung einer das PF4AR-Polypeptid exprimierenden rekombinanten Zelle oder des PF4AR-Polypeptids und eines Adjuvans oder des an ein immunogenes Peptid konjugierten PF4AR-Polypeptids zum Immunisieren eines Tiers, um Antikörper gegen ein PF4AR-Epitop hervorzubringen; und
die Gewinnung monoklonaler Antikörper aus Zellen des immunisierten Tieres, worin die monoklonalen Antikörper zur spezifischen Bindung eines PF4AR-Polypeptids nach Anspruch 1 fähig sind.

19. Verfahren zur Verwendung eines PF4AR-Polypeptids nach einem der Ansprüche 1 bis 5, wobei das Verfahren umfasst:
die Verwendung einer das PF4AR-Polypeptid exprimierenden rekombinanten Zelle oder des PF4AR-Polypeptids und eines Adjuvans oder des an ein immunogenes Peptid konjugierten PF4AR-Polypeptids zum Immunisieren eines Tiers, um Antikörper gegen ein PF4AR-Epitop hervorzubringen; und
die Gewinnung monoklonaler Antikörper aus Zellen des immunisierten Tieres, worin die monoklonalen Antikörper zur spezifischen Bindung eines PF4AR-Polypeptids aus Fig. 2 fähig sind.

20. Verfahren nach Anspruch 19, worin die Antikörper fähig sind, eine N-terminale extrazelluläre Region des PF4AR-Polypeptids spezifisch zu binden.

21. Verfahren nach einem der Ansprüche 18 bis 20, das weiters das Vermischen der Antikörper mit einem pharmazeutisch annehmbaren Träger umfasst.

22. Verfahren zur Herstellung einer therapeutischen Formulierung eines PF4AR-Polypeptids nach einem der Ansprüche 1 bis 5 oder eines monoklonalen Antikörpers nach einem der Ansprüche 13 bis 15, wobei das Verfahren das Vermischen des Polypeptids oder Antikörpers mit physiologisch annehmbaren Trägern, Exzipienten oder Stabilisatoren umfasst.

23. Verfahren nach Anspruch 22, worin der Antikörper ein Antikörper nach Anspruch 15 ist.

24. Isoliertes Blutplättchen-Faktor-4-Überfamilien-Rezeptor- (PF4AR-) Polypeptid mit zumindest 85%iger Aminosäuresequenz-Homologie mit der translatierten Aminosäuresequenz aus Fig. 4 oder Fig. 5.

25. Isoliertes PF4AR-Polypeptid, worin die Nucleinsäure, die für das PF4AR-Polypeptid kodiert, unter hochstringenten Bedingungen mit dem Komplement der für das Polypeptid aus Fig. 4 oder 5 kodierenden Nucleinsäure hybridisiert.

26. Isoliertes PF4AR-Polypeptid, das eine Aminosäuresequenz mit einer Länge von zumindest 10 Resten umfasst, die in einer extrazellulären Region des Polypeptids aus Fig. 4 oder 5 enthalten ist und fähig ist, einen Antikörper hervorzubringen, der mit dem Polypeptid aus Fig. 4 oder 5 kreuzreagiert.

27. PF4AR-Polypeptid nach Anspruch 26, worin die extrazelluläre Region die N-terminale extrazelluläre Region aus Fig. 4 oder 5 ist.

28. Isoliertes Polypeptid, welches das PF4AR-Polypeptid nach einem der Ansprüche 24 bis 27 an ein Polypeptid fusioniert umfasst, das zum PF4AR-Polypeptid heterolog ist.

29. Isoliertes Nucleinsäuremolekül, das eine Nucleinsäuresequenz umfasst, die für das PF4AR-Polypeptid nach einem der Ansprüche 24 bis 28 kodiert.

30. Nucleinsäuremolekül nach Anspruch 29, das DNA ist und die in Fig. 4 oder Fig. 5 gezeigte translatierte DNA-Sequenz aufweist.

31. Nucleinsäuremolekül nach Anspruch 29 oder Anspruch 30, das weiters einen Promotor umfasst, der an die für das PF4AR-Polypeptid kodierende Nucleinsäuresequenz operabel gebunden ist.

32. Expressionsvektor, der die Nucleinsäuresequenz nach einem der Ansprüche 29 bis 31 operabel an Kontrollsequenzen gebunden umfasst, die von einer mit dem Vektor transformierten Wirtszelle erkannt werden.

33. Mit dem Vektor nach Anspruch 32 transformierte Wirtszelle.

34. Verfahren zur Verwendung eines Nucleinsäuremoleküls, das eine für das PF4AR-Polypeptid nach einem der Ansprüche 24 bis 28 kodierende Nucleinsäuresequenz enthält, umfassend das Exprimieren des Nucleinsäuremoleküls in einer kultivierten Wirtszelle, die mit einem Vektor transformiert ist, der die Nucleinsäuresequenz umfasst, die für das PF4AR-Polypeptid kodiert und an Kontrollsequenzen operabel gebunden ist, die von der mit dem Vektor transformierten Wirtszelle erkannt werden, wodurch das PF4AR-Polypeptid produziert wird, sowie das Gewinnen des PF4AR-Polypeptids aus der Wirtszelle.

35. Verfahren nach Anspruch 34, worin das PF4AR-Polypeptid aus den Wirtszellmembranen gewonnen wird.

36. Monoklonaler Antikörper, der fähig ist, das PF4AR-Polypeptid nach einem der Ansprüche 24 bis 27 spezifisch zu binden.

37. Monoklonaler Antikörper nach Anspruch 36, der fähig ist, eine N-terminale extrazelluläre Region des PF4AR-Polypeptids spezifisch zu binden.

38. Monoklonaler Antikörper nach Anspruch 36 oder 37, der fähig ist, das PF4AR-Polypeptid aus Fig. 4 spezifisch zu binden.

39. Verfahren zur Verwendung eines PF4AR-Polypeptids nach einem der Ansprüche 24 bis 28, wobei das Verfahren umfasst:
die Verwendung einer das PF4AR-Polypeptid exprimierenden rekombinanten Zelle oder des PF4AR-Polypeptids und eines Adjuvans oder des an ein immunogenes Peptid konjugierten PF4AR-Polypeptids zum Immunisieren eines Tiers, um Antikörper gegen ein PF4AR-Epitop hervorzubringen; und
die Gewinnung monoklonaler Antikörper aus Zellen des immunisierten Tieres, worin die monoklonalen Antikörper zur spezifischen Bindung eines PF4AR-Polypeptids nach einem der Ansprüche 24 bis 27 fähig sind.

40. Verfahren zur Verwendung eines PF4AR-Polypeptids nach einem der Ansprüche 24 bis 28, wobei das Verfahren umfasst:
die Verwendung einer das PF4AR-Polypeptid exprimierenden rekombinanten Zelle oder des PF4AR-Polypeptids und eines Adjuvans oder des an ein immunogenes Peptid konjugierten PF4AR-Polypeptids zum Immunisieren eines Tiers, um Antikörper gegen ein PF4AR-Epitop hervorzubringen; und
die Gewinnung monoklonaler Antikörper aus Zellen des immunisierten Tieres, worin die monoklonalen Antikörper zur spezifischen Bindung eines PF4AR-Polypeptids aus Fig. 4 oder Fig. 5 fähig sind.

41. Verfahren nach Anspruch 40, worin die Antikörper fähig sind, eine N-terminale extrazelluläre Region des PF4AR-Polypeptids spezifisch zu binden.

42. Verfahren nach Anspruch 40 oder 41, worin die Antikörper fähig sind, das PF4AR-Polypeptid aus Fig. 4 spezifisch zu binden.

## Revendications (Revendications pour l'(les) Etat(s) contractant(s) suivant(s): AT, BE, CH, DE, DR, FR, GB, IT, LI, LU, MC, NL, SE)

1. Polypeptide récepteur de superfamille du facteur plaquettaire 4 isolé (PF4AR) présentant une homologie de séquence d'aminoacides d'au moins 85 % avec la séquence d'aminoacides traduite de la figure 2.

2. Polypeptide PF4AR suivant la revendication 1, qui comprend le polypeptide de la figure 2.

3. Polypeptide isolé comprenant le polypeptide PF4AR suivant l'une quelconque des revendications précédentes, fusionné à un polypeptide hétérologue au polypeptide PF4AR.

4. Polypeptide PF4AR suivant l'une quelconque des revendications précédentes, qui se lie à la IL-8 sans aucune liaison ou réponse à f-Met-Leu-Phe.

5. Polypeptide PF4AR suivant l'une quelconque des revendications précédentes, qui est d'origine humaine.

6. Molécule d'acide nucléique isolée comprenant une séquence d'acide nucléique codant pour le polypeptide PF4AR suivant l'une quelconque des revendications 1 à 5.

7. Molécule d'acide nucléique suivant la revendication 6, qui consiste en ADN et qui a la séquence d'ADN traduite représentée sur la figure 2.

8. Molécule d'acide nucléique suivant la revendication 6 ou la revendication 7, comprenant en outre un promoteur lié de manière fonctionnelle à la séquence d'acide nucléique codant pour le polypeptide PF4AR.

9. Vecteur d'expression comprenant la séquence d'acide nucléique suivant l'une quelconque des revendications 6 et 7 liée de manière fonctionnelle à des séquences de régulation reconnues par une cellule hôte transformée avec le vecteur.

10. Cellule hôte transformée avec le vecteur suivant la revendication 9.

11. Procédé d'utilisation d'une molécule d'acide nucléique contenant une séquence d'acide nucléique codant pour le polypeptide PF4AR suivant l'une quelconque des revendications 1 à 5, comprenant les étapes consistant à exprimer la molécule d'acide nucléique dans une cellule hôte en culture transformée avec un vecteur comprenant la séquence d'acide nucléique codant pour le polypeptide PF4AR liée de manière fonctionnelle à des séquences de régulation reconnues par la cellule hôte transformée avec le vecteur, ce qui produit le polypeptide PF4AR, et à recueillir le polypeptide PF4AR à partir de la cellule hôte.

12. Procédé suivant la revendication 11, dans lequel le polypeptide PF4AR est recueilli à partir des membranes de la cellule hôte.

13. Anticorps monoclonal qui est capable de se lier spécifiquement au polypeptide PF4AR suivant la revendication 1.

14. Anticorps monoclonal qui est capable de se lier spécifiquement au polypeptide PF4AR de la figure 2.

15. Anticorps monoclonal suivant la revendication 14, qui est capable de se lier spécifiquement à une région extracellulaire N-terminale du polypeptide PF4AR.

16. Composition comprenant l'anticorps monoclonal suivant l'une quelconque des revendications 13 à 15 et un support pharmaceutiquement acceptable.

17. Anticorps monoclonal suivant l'une quelconque des revendications 13 à 15, destiné à être utilisé en thérapeutique ou en diagnostic.

18. Polypeptide récepteur de superfamille du facteur plaquettaire 4 isolé (PF4AR) présentant une homologie de séquence d'aminoacides d'au moins 85 % avec la séquence d'aminoacides traduite de la figure 4 ou de la figure 5.

19. Polypeptide PF4AR isolé, où l'acide nucléique codant pour le polypeptide PF4AR s'hybride avec le complément de l'acide nucléique codant pour le polypeptide de la figure 4 ou 5 dans des conditions hautement drastiques.

20. Polypeptide PF4AR isolé comprenant une séquence d'aminoacides qui a une longueur d'au moins 10 résidus et qui est présente dans une région extracellulaire du polypeptide de la figure 4 ou 5 et qui est capable d'engendrer un anticorps qui présentera une réaction croisée avec le polypeptide de la figure 4 ou 5.

21. Polypeptide PF4AR suivant la revendication 20, dans lequel la région extracellulaire est la région extracellulaire N-terminale de la figure 4 ou 5.

22. Polypeptide isolé comprenant le polypeptide PF4AR de l'une quelconque des revendications 18 à 21 fusionné à un polypeptide hétérologue vis-à-vis du polypeptide PF4AR.

23. Molécule d'acide nucléique isolée comprenant une séquence d'acide nucléique codant pour le polypeptide PF4AR de l'une quelconque des revendications 18 à 22.

24. Molécule d'acide nucléique suivant la revendication 23, qui consiste en ADN et qui a la séquence d'ADN traduite représentée sur la figure 4 ou la figure 5.

25. Molécule d'acide nucléique suivant la revendication 23 ou la revendication 24, comprenant en outre un promoteur lié de manière fonctionnelle à la séquence d'acide nucléique codant pour le polypeptide PF4AR.

26. Vecteur d'expression comprenant la séquence d'acide nucléique de l'une quelconque des revendications 23 à 25 liée de manière fonctionnelle à des séquences de régulation reconnues par une cellule hôte transformée avec le vecteur.

27. Cellule hôte transformée avec le vecteur de la revendication 26.

28. Procédé d'utilisation d'une molécule d'acide nucléique contenant une séquence d'acide nucléique codant pour le polypeptide PF4AR de l'une quelconque des revendications 18 à 22, comprenant les étapes consistant à exprimer la molécule d'acide nucléique dans une cellule hôte en culture transformée avec un vecteur comprenant la séquence d'acide nucléique codant pour le polypeptide PF4AR liée de manière fonctionnelle à des séquences de régulation reconnues par la cellule hôte transformée avec le vecteur, ce qui produit le polypeptide PF4AR, et à recueillir le polypeptide PF4AR à partir de la cellule hôte.

29. Procédé suivant la revendication 28, dans lequel le polypeptide PF4AR est recueilli à partir des membranes des cellules hôtes.

30. Anticorps monoclonal qui est capable de se lier spécifiquement au polypeptide PF4AR suivant l'une quelconque des revendications 18 à 21.

31. Anticorps monoclonal suivant la revendication 30, qui est capable de se lier spécifiquement à une région extracellulaire N-terminale du polypeptide PF4AR.

32. Anticorps monoclonal suivant la revendication 30 ou 31, qui est capable de se lier spécifiquement au polypeptide PF4AR de la figure 4.

## Revendications (Revendications pour l'(les) Etat(s) contractant(s) suivant(s): ES, GR)

1. Polypeptide récepteur de la superfamille du facteur plaquettaire 4 isolé (PF4AR) isolé présentant une homologie de séquence d'aminoacides d'au moins 85 % avec la séquence d'aminoacides traduite de la figure 2.

2. Polypeptide PF4AR suivant la revendication 1, qui comprend le polypeptide de la figure 2.

3. Polypeptide isolé comprenant le polypeptide PF4AR suivant l'une quelconque des revendications précédentes fusionné à un polypeptide hétérologue au polypeptide PF4AR.

4. Polypeptide PF4AR suivant l'une quelconque des revendications précédentes, qui se lie à la IL-8 sans aucune liaison ou réponse à f-Met-Leu-Phe.

5. Polypeptide PF4AR suivant l'une quelconque des revendications précédentes, qui est d'origine humaine.

6. Molécule d'acide nucléique isolée comprenant une séquence d'acide nucléique codant pour le polypeptide PF4AR suivant l'une quelconque des revendications 1 à 5.

7. Molécule d'acide nucléique suivant la revendication 6, qui consiste en ADN et qui a la séquence d'ADN traduite représentée sur la figure 2.

8. Molécule d'acide nucléique suivant la revendication 6 ou la revendication 7, comprenant en outre un promoteur lié de manière fonctionnelle à la séquence d'acide nucléique codant pour le polypeptide PF4AR.

9. Vecteur d'expression comprenant la séquence d'acide nucléique suivant l'une quelconque des revendications 6 à 8 liée de manière fonctionnelle à des séquences de régulation reconnues par une cellule hôte transformée avec le vecteur.

10. Cellule hôte transformée avec le vecteur suivant la revendication 9.

11. Procédé d'utilisation d'une molécule d'acide nucléique contenant une séquence d'acide nucléique codant pour le polypeptide PF4AR suivant l'une quelconque des revendications 1 à 5, comprenant les étapes consistant à exprimer la molécule d'acide nucléique dans une cellule hôte en culture transformée avec un vecteur comprenant la séquence d'acide nucléique codant pour le polypeptide PF4AR liée de manière fonctionnelle à des séquences de régulation reconnues par la cellule hôte transformée avec le vecteur, ce qui produit le polypeptide PF4AR, et à recueillir le polypeptide PF4AR à partir de la cellule hôte.

12. Procédé suivant la revendication 11, dans lequel le polypeptide PF4AR est recueilli à partir des membranes de la cellule hôte.

13. Anticorps monoclonal qui est capable de se lier spécifiquement au polypeptide PF4AR suivant la revendication 1.

14. Anticorps monoclonal qui est capable de se lier spécifiquement au polypeptide PF4AR de la figure 2.

15. Anticorps monoclonal suivant la revendication 14, qui est capable de se lier spécifiquement à une région extracellulaire N-terminale du polypeptide PF4AR.

16. Composition comprenant l'anticorps monoclonal suivant l'une quelconque des revendications 13 à 15 et un support pharmaceutiquement acceptable.

17. Anticorps monoclonal suivant l'une quelconque des revendications 13 à 15, destiné à être utilisé en thérapeutique ou en diagnostic.

18. Procédé d'utilisation d'un polypeptide PF4AR répondant à la définition suivant l'une quelconque des revendications 1 à 9, procédé qui comprend les étapes consistant :
à utiliser une cellule recombinante exprimant le polypeptide PF4AR, ou le polypeptide PF4AR et un adjuvant, ou le polypeptide PF4AR conjugué à un peptide immunogène, pour immuniser un animal afin d'engendrer des anticorps contre un épitope de PF4AR ; et
à préparer des anticorps monoclonaux à partir de cellules de l'animal immunisé, les anticorps monoclonaux étant capables de se lier spécifiquement à un polypeptide PF4AR répondant à la définition suivant la revendication 1.

19. Procédé d'utilisation d'un polypeptide PF4AR répondant à la définition suivant l'une quelconque des revendications 1 à 5, procédé qui comprend les étapes consistant :
à utiliser une cellule recombinante exprimant le polypeptide PF4AR, ou le polypeptide PF4AR et un adjuvant, ou le polypeptide PF4AR conjugué à un peptide immunogène, pour immuniser un animal afin d'engendrer des anticorps contre un épitope de PF4AR ; et
à préparer des anticorps monoclonaux à partir de cellules de l'animal immunisé, les anticorps monoclonaux étant capables de se lier spécifiquement au polypeptide PF4AR de la figure 2.

20. Procédé suivant la revendication 19, dans lequel les anticorps sont capables de se lier spécifiquement à une région extracellulaire N-terminale du polypeptide PF4AR.

21. Procédé suivant l'une quelconque des revendications 18 à 20, comprenant en outre le mélange des anticorps à un support pharmaceutiquement acceptable.

22. Procédé de préparation d'une formulation thérapeutique d'un polypeptide PF4AR répondant à la définition suivant l'une quelconque des revendications 1 à 5 ou d'un anticorps monoclonal répondant à la définition suivant l'une quelconque des revendications 13 à 15, procédé qui comprend le mélange du polypeptide ou de l'anticorps à des supports, excipients ou stabilisants physiologiquement acceptables.

23. Procédé suivant la revendication 22, dans lequel l'anticorps est un anticorps suivant la revendication 15.

24. Polypeptide récepteur de superfamille du facteur plaquettaire 4 isolé (PF4AR) ayant une homologie de séquence d'aminoacides d'au moins 85 % avec la séquence d'aminoacides traduite de la figure 4 ou de la figure 5.

25. Polypeptide PF4AR isolé, où l'acide nucléique codant pour le polypeptide PF4AR s'hybride avec le complément de l'acide nucléique codant pour le polypeptide de la figure 4 ou 5 dans des conditions hautement drastiques.

26. Polypeptide PF4AR isolé comprenant une séquence d'aminoacides qui a une longueur d'au moins 10 résidus et qui est présente dans une région extracellulaire du polypeptide de la figure 4 ou 5 et qui est capable d'engendrer un anticorps qui présentera une réaction croisée avec le polypeptide de la figure 4 ou 5.

27. Polypeptide PF4AR suivant la revendication 26, dans lequel la région extracellulaire est la région extracellulaire N-terminale de la figure 4 ou 5.

28. Polypeptide isolé comprenant le polypeptide PF4AR de l'une quelconque des revendications 24 à 27 fusionné à un polypeptide hétérologue vis-à-vis du polypeptide PF4AR.

29. Molécule d'acide nucléique isolée comprenant une séquence d'acide nucléique codant pour le polypeptide PF4AR de l'une quelconque des revendications 24 à 28.

30. Molécule d'acide nucléique suivant la revendication 29, qui consiste en ADN et qui a la séquence d'ADN traduite représentée sur la figure 4 ou la figure 5.

31. Molécule d'acide nucléique suivant la revendication 29 ou la revendication 30, comprenant en outre un promoteur lié de manière fonctionnelle à la séquence d'acide nucléique codant pour le polypeptide PF4AR.

32. Vecteur d'expression comprenant la séquence d'acide nucléique suivant l'une quelconque des revendications 29 à 31 liée de manière fonctionnelle à des séquences de régulation reconnues par une cellule hôte transformée avec le vecteur.

33. Cellule hôte transformée avec le vecteur de la revendication 32.

34. Procédé d'utilisation d'une molécule d'acide nucléique contenant une séquence d'acide nucléique codant pour le polypeptide PF4AR de l'une quelconque des revendications 24 à 28, comprenant les étapes consistant à exprimer la molécule d'acide nucléique dans une cellule hôte en culture transformée avec un vecteur comprenant la séquence d'acide nucléique codant pour le polypeptide PF4AR liée de manière fonctionnelle à des séquences de régulation reconnues par la cellule hôte transformée avec le vecteur, ce qui produit le polypeptide PF4AR, et à recueillir le polypeptide PF4AR à partir de la cellule hôte.

35. Procédé suivant la revendication 34, dans laquelle le polypeptide PF4AR est recueilli à partir des membranes de la cellule hôte.

36. Anticorps monoclonal qui est capable de se lier spécifiquement au polypeptide PF4AR suivant l'une quelconque des revendications 24 à 27.

37. Anticorps monoclonal suivant la revendication 36, qui est capable de se lier spécifiquement à une région extracellulaire N-terminale du polypeptide PF4AR.

38. Anticorps monoclonal suivant la revendication 36 ou 37, qui est capable de se lier spécifiquement au polypeptide PF4AR de la figure 4.

39. Procédé d'utilisation d'un polypeptide PF4AR tel que défini dans l'une quelconque des revendications 24 à 28, procédé qui comprend :
l'utilisation d'une cellule recombinante exprimant le polypeptide PF4AR, ou le polypeptide PF4AR et un adjuvant, ou bien le polypeptide PF4AR conjugué à un peptide immunogène, pour immuniser un animal afin d'engendrer des anticorps contre un épitope de PF4AR ; et
la préparation d'anticorps monoclonaux à partir de cellules de l'animal immunisé,
les anticorps monoclonaux étant capables de se lier spécifiquement à un polypeptide PF4AR tel que défini dans l'une quelconque des revendications 24 à 27.

40. Procédé d'utilisation d'un polypeptide PF4AR tel que défini dans l'une quelconque des revendications 24 à 28, procédé qui comprend:
l'utilisation d'une cellule recombinante exprimant le polypeptide PF4AR, ou le polypeptide PF4AR et un adjuvant, ou bien le polypeptide PF4AR conjugué à un peptide immunogène, pour immuniser un animal afin d'engendrer des anticorps contre un épitope de PF4AR ; et
la préparation d'anticorps monoclonaux à partir de cellules de l'animal immunisé,
les anticorps monoclonaux étant capables de se lier spécifiquement au polypeptide PF4AR de la figure 4 ou 5.

41. Procédé suivant la revendication 40, dans lequel les anticorps sont capables de se lier spécifiquement à une région extracellulaire N-terminale du polypeptide PF4AR.

42. Procédé suivant la revendication 40 ou 41, dans lequel les anticorps sont capables de se lier spécifiquement au polypeptide PF4AR de la figure 4.
